# EUROPEAN PATENT SPECIFICATION

(11) **EP 3 528 640 B1**
(45) Date of publication and mention of the grant of the patent: **15.01.2025**
(21) Application number: 17862466.4
(22) Date of filing: 20.10.2017
(51) Int. Cl.: C07H 15/256, C07H 13/08, A23L 2/60, A23L 27/30

(54) **DITERPENE GLYCOSIDES ISOLATED FROM STEVIA, COMPOSITIONS AND METHODS**
AUS STEVIA ISOLIERTE DITERPENGLYKOSIDE, ZUSAMMENSETZUNGEN UND VERFAHREN
GLYCOSIDES DITERPÉNIQUES ISOLÉS À PARTIR DE STEVIA, COMPOSITIONS ET PROCÉDÉS

(30) Priority: 20.10.2016 US 201662410562 P
(43) Date of publication of application: 28.08.2019
(73) Proprietor: The Coca-Cola Company, Atlanta, GA 30313 (US)
(72) Inventor: PRAKASH, Indra, Alpharetta GA 30022 (US); MA, Gil, Atlanta GA 30308 (US)
(74) Representative: Dehns
(86) International application number: PCT/US2017/057561
(87) International publication number: WO 2018/075874

(56) References cited:
- EP-A1- 3 564 251
- WO-A1-2016/120486
- WO-A1-2018/071744
- WO-A2-2014/146135
- US-A1- 2003 060 428
- US-A1- 2014 296 499
- US-A1- 2016 039 856
- US-B2- 9 115 166
- OHTANI ET AL.: "Minor diterpene glycosides from sweet leaves of Rubus suavissimus", PHYTOCHEMISTRY, vol. 31, no. 5, 20 March 2001 (2001-03-20), pages 1553 - 1559, XP026659910

## Description

### FIELD OF THE INVENTION

The present invention relates generally to beverages of beverages products comprising a novel diterpene glycoside.

### BACKGROUND OF THE INVENTION

Natural caloric sugars, such as sucrose, fructose and glucose, are utilized to provide a pleasant taste to beverages, foods, pharmaceuticals, and oral hygienic/cosmetic products. Sucrose, in particular, imparts a taste preferred by consumers. Although sucrose provides superior sweetness characteristics, it is disadvantageously caloric.

Non-caloric or low caloric sweeteners have been introduced to satisfy consumer demand. However, non- and low caloric sweeteners taste different from natural caloric sugars in ways that frustrate consumers. On a taste basis, non-caloric or low caloric sweeteners exhibit a temporal profile, maximal response, flavor profile, mouth feel, and/or adaptation behavior that differ from sugar. Specifically, non-caloric or low caloric sweeteners exhibit delayed sweetness onset, lingering sweet aftertaste, bitter taste, metallic taste, astringent taste, cooling taste and/or licorice-like taste. On a source basis, many non-caloric or low caloric sweeteners are synthetic sweeteners. Consumer desire for natural non-caloric or low caloric sweeteners that tastes like sucrose remains high.

*Stevia rebaudiana* Bertoni is a perennial shrub of the *Asteraceae (Compositae)* family native to certain regions of South America. Its leaves have been traditionally used for hundreds of years in Paraguay and Brazil to sweeten local teas and medicines. The plant is commercially cultivated in Japan, Singapore, Taiwan, Malaysia, South Korea, China, Israel, India, Brazil, Australia and Paraguay.

The leaves of the plant contain a mixture containing diterpene glycosides in an amount ranging from about 10% to 15% of the total dry weight. Structurally, the diterpene glycosides are characterized by a single base, steviol, and differ by the presence of carbohydrate residues at positions C13 and C19. Typically, on a dry weight basis, the four major steviol glycosides found in the leaves of Stevia are dulcoside A (0.3%), rebaudioside C (0.6-1.0%), rebaudioside A (3.8%) and stevioside (9.1%). Other glycosides identified in Stevia extract include rebaudioside B, D, E, and F, steviolbioside and rubusoside. Among these, only stevioside and rebaudioside A are available on a commercial scale.

The use of steviol glycosides has been limited to date by certain undesirable taste properties, including licorice taste, bitterness, astringency, sweet aftertaste, bitter aftertaste, licorice aftertaste, and become more prominent with increase of concentration. These undesirable taste attributes are particularly prominent in carbonated beverages, where full replacement of sugar requires concentrations of steviol glycosides that exceed 600 mg/L. Use of steviol glycosides in such high concentrations results in significant deterioration in the final product taste.

Accordingly, there remains a need to develop natural reduced or non-caloric sweeteners that provide a temporal and flavor profile similar to the temporal and flavor profile of sucrose.

There remains a further need for methods for purifying glycosides from stevia.

WO 2014/146135 discloses steviol glycosides, their compositions and their purification. EP 3564251 discloses a novel steviol glycoside, a method for producing same, and a sweetener composition containing same. WO 2016/120486 discloses recombinant microorganisms and methods for producing steviol glycosides, glycosylated ent-kaurenol, and glycosylated ent-kaurenoic acid. US 9,115,166 discloses a method for degrading rebaudioside A and the rebaudioside A derivative products derived therefrom. US 2014/296499 discloses a process for the preparation of rebaudioside D and other related naturally occurring sweetners. US 2003/060428 discloses a medicament for the treatment of non-insulin dependent diabetes mellitus, hypertension, metabolic syndromes and other conditions in mammals. Ohtani et al (Phytochemistry, Vol 31, No. 5, 1992, p1553-1559) discloses minor diterpene glycosides from sweet leaves of Rubus suavissimus. WO 2018/071744 discloses the biosynthetic production of steviol glycosides.

### SUMMARY OF THE INVENTION

The invention is defined in the appended claims. Any disclosure going beyond the scope of said claims is only intended for illustrative purposes and should not be construed as being part of the invention.

In one aspect, the present invention provides a beverage or beverage product comprising an isolated and purified diterpene glycoside of the following formula: 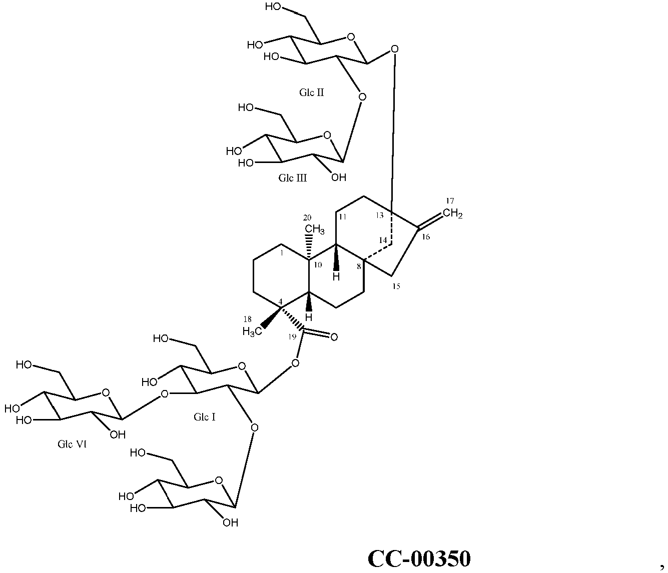 wherein the diterpene glycoside is present in the beverage in a concentration from about 25 ppm to about 600 ppm.

In a particular embodiment, the present invention is a beverage comprising at least the diterpene glycoside CC-00350. In a particular embodiment, the diterpene glycoside is present in the beverage at a concentration that is above, at or below the threshold sweetness recognition concentration of the diterpene glycoside.

In another particular embodiment, the present invention is a beverage product comprising the diterpene glycoside CC-00350. In a particular embodiment, the diterpene glycoside is present in the beverage product at a concentration that is above, at or below the threshold flavor recognition concentration of the diterpene glycoside.

In another embodiment, the present invention is a beverage comprising at least the diterpene glycoside CC-00350and one or more sweeteners, additives and/or functional ingredients.

A method for purifying a diterpene glycoside of the present invention comprises (i) passing a solution comprising a source material comprising a diterpene glycoside of the formulae described herein through a HPLC column and (ii) eluting fractions comprising the diterpene glycoside of the formulae described herein to provide a purified diterpene glycoside of the formulae described herein. The method provides a purified diterpene glycoside of the formulae described herein in a purity greater than about 50% by weight on a dry basis.

The HPLC column can be preparative or semi-preparative. The fractions comprising the diterpene glycoside of interest may be eluted by adding an appropriate eluent. The method may optionally comprise additional steps, such as partial or substantially full removal of solvents and/or further purification steps, e.g. extraction, crystallization, chromatography and distillation.

In still other embodiments, the source material can be one fraction, or multiple fractions, containing unpurified diterpene glycoside of interest collected from a previous method or HPLC protocol. The material isolated can be subjected to further methods 2, 3, 4 or more times, each time providing a higher level of purity of the diterpene glycoside. The second and subsequent methods may have different HPLC protocols and different steps following elution.

### BRIEF DESCRIPTION OF THE DRAWINGS

**Figure 1****:** Shows the aglycone region of diterpene glycosides **CC-00336, CC-00338, CC-00339, CC-00343** and **CC-00350.** HMBC and COSY correlations used to for assignment are described in the Examples.
**Figure 2** Shows a summary of key HMBC and COSY correlations used to assign the aglycone region of diterpene glycoside **CC-00327.**
**Figure 3****:** Shows a summary of key HMBC and COSY correlations used to assign the aglycone region of diterpene glycoside **CC-00340.**
**Figure 4****:** Shows a summary of key HMBC and COSY correlations used to assign the aglycone region of diterpene glycoside **CC-00344.**
**Figure** 5: Shows a summary of key HMBC and COSY correlations used to assign the aglycone region of diterpene glycoside CC-00349.

### DETAILED DESCRIPTION OF THE INVENTION

### I. Compounds

Described herein are diterpene glycosides of Formula I: 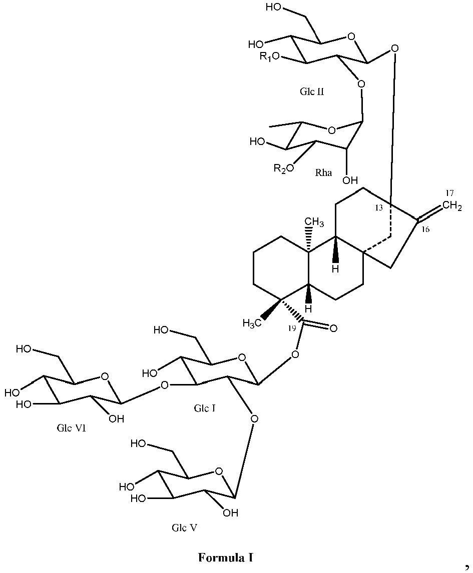 wherein R₁ and R₂ are are each independently selected from hydrogen, monosaccharide, disaccharide and oligosaccharide.

R₁ may be selected from hydrogen, monosaccharide and disaccharide and R₂ is selected from hydrogen, monosaccharide and disaccharide.

Each saccharide may be selected from the group consisting of glucose, xylose, rhamnose, fructose and 6-deoxy-glucose. For example, each saccharide may selected from glucose and rhamnose.

The linkage between the saccharides in the disaccharide or oligosaccharide can be α-, β or a mixture thereof (if applicable).

The linkage between the saccharides (which may be part of a disaccharide or oligosaccharide) at R₁ and Glc II can be α- or β. The linkage between the saccharides (which may be part of a disaccharide or oligosaccharide) at R₂ and Rha can be α- or β.

A diterpene glycoside of Formula I may comprise at least three saccharides pendant to C13 (including Glc II, Rha and Glc V), such as, for example, three saccharides, four saccharides, five saccharides or six saccharides. For example, a diterpene glycoside of Formula I may comprise from three to six saccharides pendant to C13.

Diterpene glycosides of Formula I include: 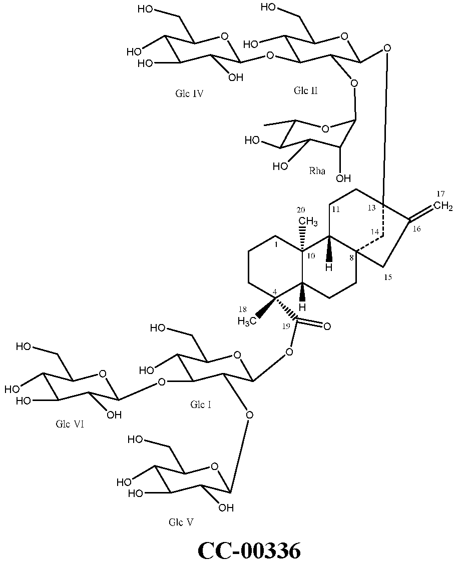 and

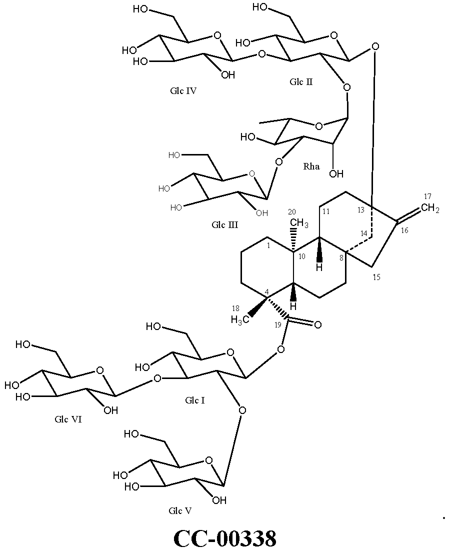

Also described herein are diterpene glycosides of Formula II: 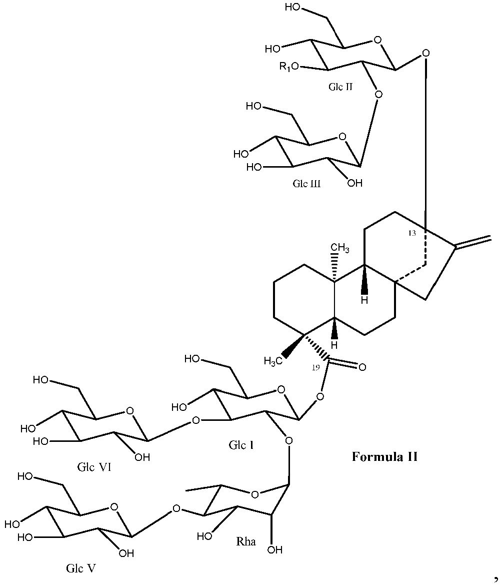 wherein R₁ is selected from hydrogen, monosaccharide, disaccharide and oligosaccharide.

R₁ may be selected from hydrogen, monosaccharide and disaccharide.

Each saccharide may be selected from the group consisting of glucose, xylose, rhamnose, fructose and 6-deoxy-glucose. For example, each saccharide may be selected from glucose and rhamnose.

The linkage between the saccharides in the disaccharide or oligosaccharide can be α-, β or a mixture thereof (if applicable).

The linkage between the saccharides (which may be part of a disaccharide or oligosaccharide) at R₁ and Glc II can be α- or β.

A diterpene glycoside of Formula II may comprise at least two saccharides pendant to C13 (including Glc II and Glc III), such as, for example, three saccharides, four saccharides, five saccharides or six saccharides. For example, a diterpene glycoside of Formula I may comprise from three to six saccharides pendant to C13.

Example diterpene glycosides include the following compound:

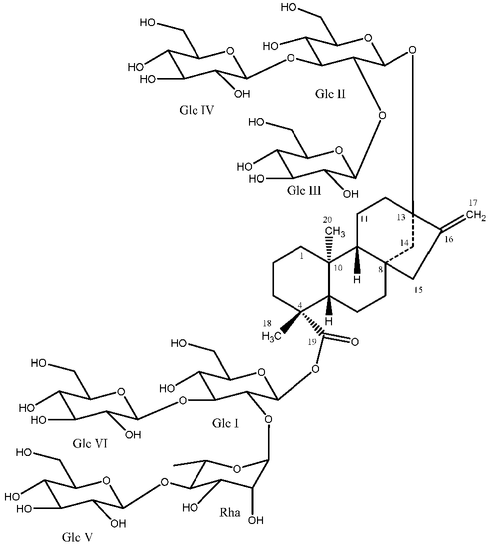

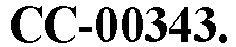

Other diterpene glycosides include:

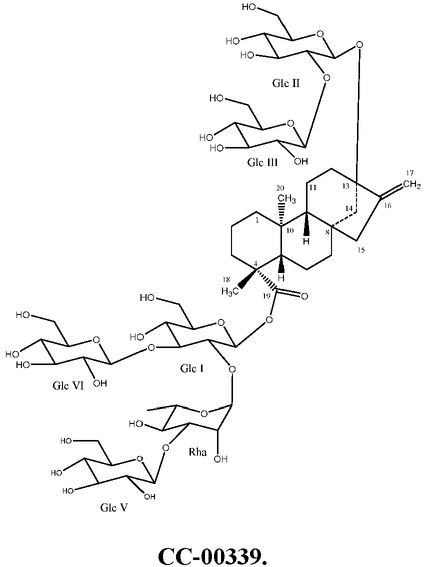

In the present invention, the beverage or beverage product comprises the following diterpene glycoside:

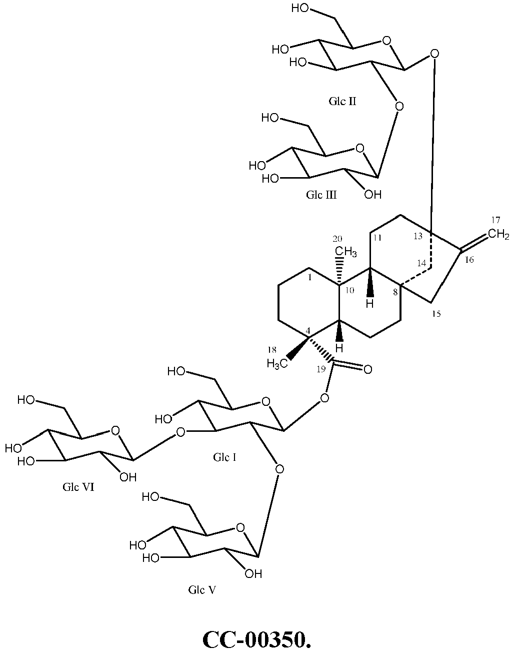

Other diterpene glycosides include those of Formula III: 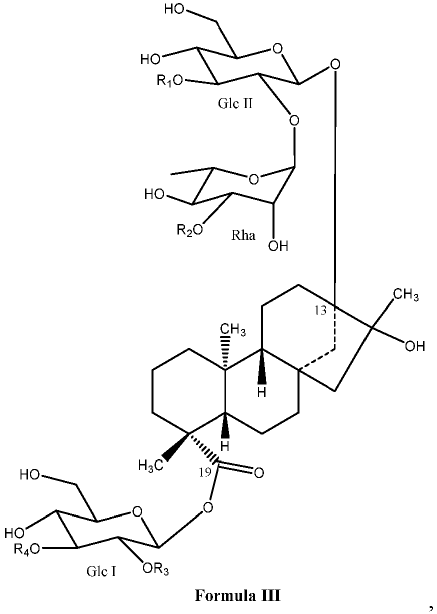 wherein R₁, R₂, R₃ and R₄ are each independently selected from hydrogen, monosaccharide, disaccharide and oligosaccharide.

R₁ may be selected from hydrogen, monosaccharide and disaccharide and R₂ is selected from hydrogen, monosaccharide and disaccharide.

Each saccharide may be selected from the group consisting of glucose, xylose, rhamnose, fructose and 6-deoxy-glucose. For example, each saccharide may be selected from glucose and rhamnose.

The linkage between the saccharides in the disaccharide or oligosaccharide can be α-, β or a mixture thereof (if applicable).

The linkage between the saccharides (which may be part of a disaccharide or oligosaccharide) at R₁ and Glc II can be α- or β. The linkage between the saccharides (which may be part of a disaccharide or oligosaccharide) at R₂ and Rha can be α- or β. The linkage between the saccharides (which may be part of a disaccharide or oligosaccharide) at R₃ and Glc I can be α- or β. The linkage between the saccharides (which may be part of a disaccharide or oligosaccharide) at R₄ and Glc I can be α- or β.

A diterpene glycoside of Formula III may comprise at least two saccharides pendant to C13 (including Glc II and Rha), such as, for example, three saccharides, four saccharides, five saccharides or six saccharides. For example, a diterpene glycoside of Formula III may comprise from three to six saccharides pendant to C13.

A diterpene glycoside of Formula III may comprise at least one saccharide pendant to C19 (including Glc I), such as, for example, two saccharides, three saccharides, four saccharides, five saccharides or six saccharides. For example, a diterpene glycoside of Formula III may comprise from one to six saccharides pendant to C19.

An example diterpene glycoside is the following compound:

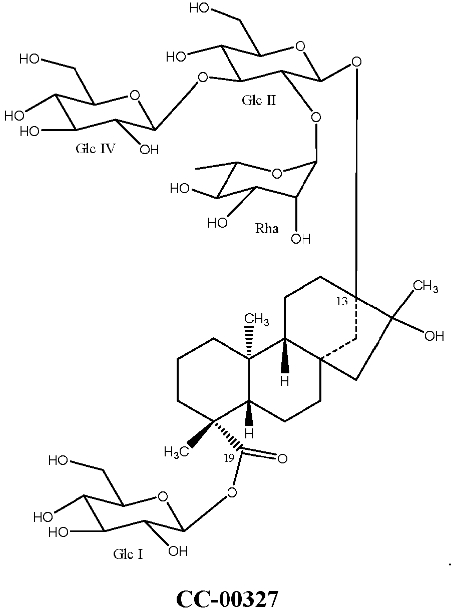

Other diterpene glycosides include those of Formula IV: 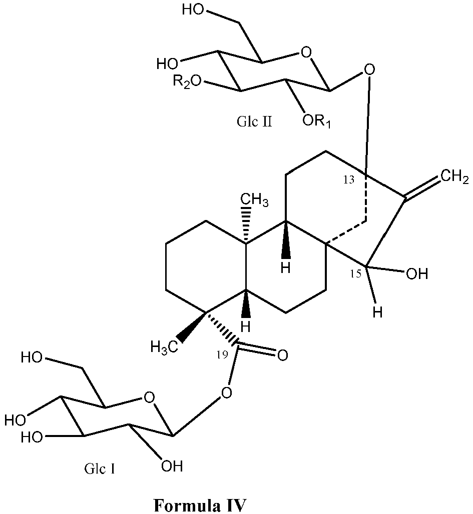 wherein R₁ and R₂ are each independently selected from hydrogen, monosaccharide and disaccharide.

Each saccharide may be selected from the group consisting of glucose, xylose, rhamnose, fructose and 6-deoxy-glucose. For example, each saccharide may be selected from glucose and rhamnose.

The linkage between the saccharides in the disaccharide or oligosaccharide can be α-, β-, or a mixture thereof (if applicable). The linkage between the saccharides (which may be part of a disaccharide) at R₁ and Glc II can be α- or β. The linkage between the saccharides (which may be part of a disaccharide) at R₂ and Glc II can be α- or β.

A diterpene glycoside of Formula IV may comprise at least two saccharides pendant to C13 (including Glc II), such as, for example, three saccharides, four saccharides, five saccharides or six saccharides. For example, a diterpene glycoside of Formula IV may comprise from three to six saccharides pendant to C13.

An example diterpene glycoside is the following compound:

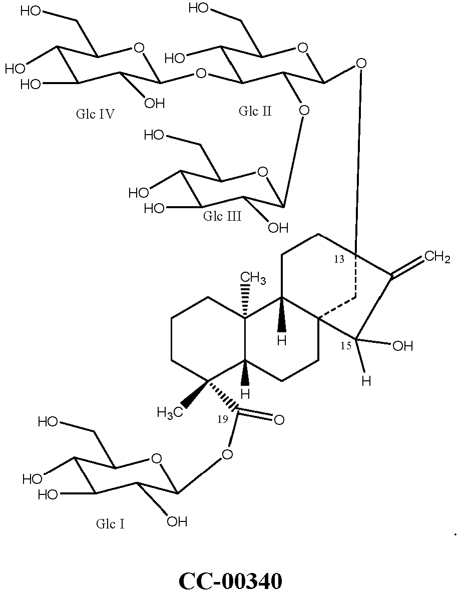

Other diterpene glycosides include those of Formula V: 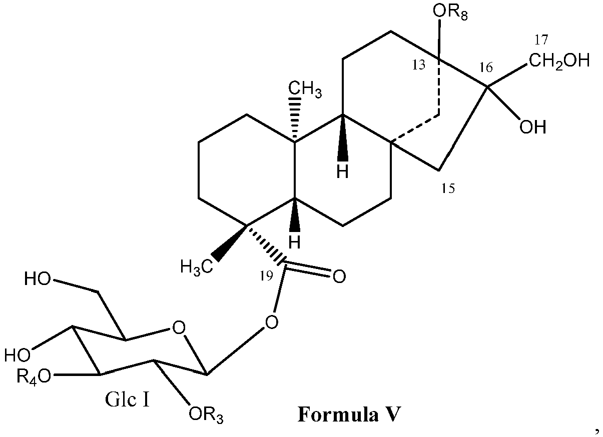 wherein R₈ is selected from hydrogen, monosaccharide, disaccharide and oligosaccharide; and R₃ and R₄ are each independently selected from hydrogen and monosaccharide.

R₈ may be hydrogen and R₃ and R₄ are monosaccharides.

R₈ may be trisaccharide and R₃ and R₄ are hydrogen.

Each saccharide may be selected from the group consisting of glucose, xylose, rhamnose, fructose and 6-deoxy-glucose. For example, each saccharide may be selected from glucose and rhamnose.

The linkage between the saccharides in the disaccharide or oligosaccharide can be α-, β-, or a mixture thereof (if applicable). The linkage between the monosaccharide at R₃ or R₄ and and Glc I can be α- or β. In one embodiment, R₃ is glucose and the linkage to Glc I is β. In another particular embodiment, R₃ is rhamnose and the linkage to Glc I is α.

A diterpene glycoside of Formula V may comprise no saccharides pendant to C13. A diterpene glycoside of Formula V may comprise at least one saccharide pendant to C13, such as, for example, three saccharides, four saccharides, five saccharides or six saccharides. For example, a diterpene glycoside of Formula V may comprise from three to six saccharides pendant to C13.

A diterpene glycoside of Formula V may comprise at least one saccharide pendant to C19 (including Glc I), such as, for example, three saccharides, four saccharides, five saccharides or six saccharides. For example, a diterpene glycoside of Formula V may comprise from three to six saccharides pendant to C19.

Examples diterpene glycoside include the group consisting of: 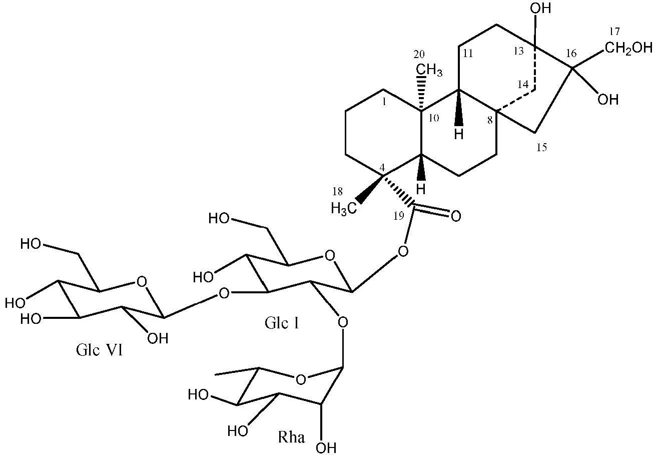 and

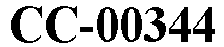

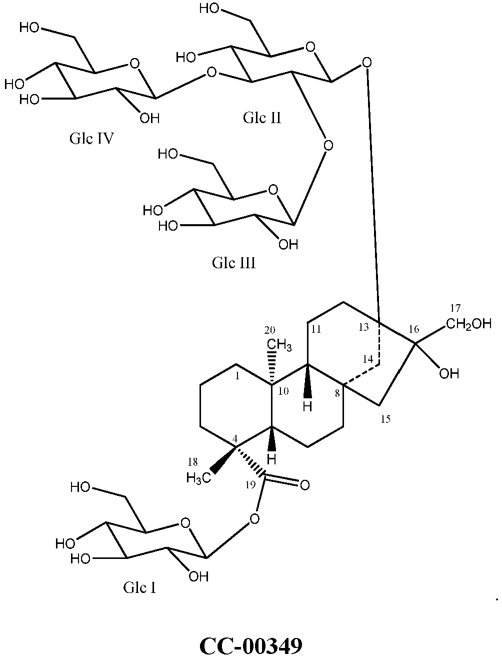

The compounds described herein have a plurality of stereocenters (R,S). Unless stereochemistry is specifically provided for, all stereochemical configurations are contemplated herein.

The diterpene glycoside used in the present invention is isolated and purified. The term "isolated and purified", as used herein, means that the compound is about 95% by weight or greater on a dry basis, i.e. is greater than 95% pure. The remainder of the mixture is typically other steviol glycoside and/or Stevia extract. In more specific embodiments, the diterpene glycoside has a purity of about 96% or greater, about 97% or greater, about 98% or greater or about 99% or greater. In some embodiments, the compound is enzymatically produced and is in a purity of at least about 95% by weight or greater in a mixture.

In some embodiments, the diterpene glycoside used in the present invention is sweet. The sweetness of a given composition is typically measured with reference to a solution of sucrose. See generally "A Systematic Study of Concentration-Response Relationships of Sweeteners," G.E. DuBois, D.E. Walters, S.S. Schiffman, Z.S. Warwick, B.J. Booth, S.D. Pecore, K. Gibes, B.T. Carr, and L.M. Brands, in Sweeteners: Discovery, Molecular Design and Chemoreception, D.E. Walters, F.T. Orthoefer, and G.E. DuBois, Eds., American Chemical Society, Washington, DC (1991), pp 261-276.

The sweetness of a non-sucrose sweetener can be measured against a sucrose reference by determining the non-sucrose sweetener's sucrose equivalence (SE). Typically, taste panelists are trained to detect sweetness of reference sucrose solutions containing between 1-15% sucrose (w/v). Other non-sucrose sweeteners are then tasted at a series of dilutions to determine the concentration of the non-sucrose sweetener that is as sweet as a given percent sucrose reference. For example, if a 1% solution of a sweetener is as sweet as a 10% sucrose solution, then the sweetener is said to be 10 times as potent as sucrose, and has 10% sucrose equivalence.

In one embodiment, the diterpene glycoside is present in an amount that, when added to a beverage, provides a sucrose equivalence of greater than about 2% (w/v), such as, for example, greater than about 3%, about 4%, about 5%, about 6%, about 7%, about 8%, about 9%, about 10%, about 11%, about 12%, about 13% or about 14%.

The amount of sucrose, and thus another measure of sweetness, in a reference solution may be described in degrees Brix (°Bx). One degree Brix is 1 gram of sucrose in 100 grams of solution and represents the strength of the solution as percentage by weight (% w/w) (strictly speaking, by mass). In one embodiment, the diterpene glycoside of the present invention is present in an amount that, when added to a beverage, provides a sweetness equivalent from about 0.50 to 14 degrees Brix, such as, for example, from about 5 to about 12 degrees Brix, about 7 to 10 degrees Brix, or above 10 degrees Brix.

In exemplary embodiments, an isolated and purified diterpene glycoside used in the present invention has about 30% or more sweetness compared to the partially purified diterpene glycoside or Stevia leaf, such as, for example, about 40% or more, about 50% or more, about 60% or more, about 70% or more, about 80% or more or about 90% or more.

In other exemplary embodiments, an isolated and purified diterpene glycoside used in the present invention has at least about 30% less bitterness (the taste stimulated by certain substances such as quinine, caffeine and sucrose octa-acetate) compared the partially purified diterpene glycoside or Stevia leaf, such as, for example, at least about 40%, at least about 50%, at least about 60%, at least about 70%, at least about 80% or at least about 90%. In a particular embodiment, the isolated and purified diterpene glycoside used in the present invention has substantially no bitterness. Methods of measuring bitterness of a compound are known in the art

In still other exemplary embodiments, an isolated and purified diterpene glycoside used in the present invention has at least about 30% less sweet lingering aftertaste (the intensity of the sweet taste after expectoration) compared to the partially purified diterpene glycoside or Stevia leaf, such as, for example, at least about 50%, at least about 60%, at least about 70%, at least about 80% or at least about 90%. In a particular embodiment, the isolated and purified diterpene glycoside used in the present invention has substantially no sweet lingering aftertaste. Methods of measuring sweet lingering aftertaste are known in the art.

In yet other exemplary embodiments, an isolated and purified diterpene glycoside used in the present invention has at least about 30% less metallic taste (taste associated with metals, tinny or iron) compared to the partially purified diterpene glycoside or Stevia leaf, such as, for example, at least about 50%, at least about 60%, at least about 70%, at least about 80% or at least about 90%. In a particular embodiment, the isolated and purified diterpene glycoside used in the present invention has substantially no metallic taste.

In exemplary embodiments, an isolated and purified diterpene glycoside used in the present invention exhibits a maximal response (maximum sweetness (%SE) achieved with increasing concentration of compound) that is at least about 30% greater compared to the partially purified diterpene glycoside or Stevia leaf, such as, for example, at least about 40% greater, at least about 50% greater, at least about 60% greater, at least about 70% greater, at least about 80% greater or at least about 90% greater. Methods of measuring the maximal response of a compound are known in the art. In one embodiment, the method is an in vitro cell assay. In some embodiments, the cell is expressing a sweet taste receptor or a dimer of sweet taste receptor.

In other exemplary embodiments, an isolated and purified diterpene glycoside used in the present invention exhibits a sweetness onset (the time until maximum sweetness is experienced) that is at least about 30% shorter than the partially purified diterpene glycoside or Stevia leaf, such as, for example, at least about 40% short, at least about 50% shorter, at least about 60% shorter, at least about 70% shorter, at least about 80% shorter or at least about 90% shorter. Methods of measuring sweetness onset are known in the art. In one embodiment, the method is an in vitro cell assay. In some embodiments, the cell is expressing a sweet taste receptor or a dimer of sweet taste receptor.

### II. Compositions

Described herein are compositions comprising at least one diterpene glycoside of the present invention. "Composition," as the term is used herein, refers to a mixture of at least one diterpene glycoside of the present invention and at least one other substance, wherein the diterpene glycoside is admixed with the at least one other substance. As used herein, "admix" means to mingle or add to something else, but in any case, requires an active step.

In a particular embodiment, the at least one other substance does not occur and/or is not admixed with the diterpene glycoside in nature, i.e. the Stevia leaf. As such, the compositions contemplated by the present invention do not occur in nature.

Also described herein is a composition comprising at least one diterpene glycoside of the present invention, provided as part of a mixture. In a particular embodiment, the mixture is selected from the group consisting of diterpene glycosides, stevia extract, by-products of other diterpene glycosides' isolation and purification processes, commercially available diterpene extracts or stevia extracts, by-products of biotransformation reactions of other diterpene glycosides, or any combination thereof.

In one embodiment, the mixture contains at least one diterpene glycoside of the present invention in an amount that ranges from about 1% to about 99% by weight on a dry basis, such as, for example, about 5% to about 99% by weight on a dry basis, from about 10% to about 99%, from about 20% to about 99%, from about 30% to about 99%, from about 40% to about 99%, from about 50% to about 99%, from about 60% to about 99%, from about 70% to about 99%, from about 80% to about 99% and from about 90% to about 99%. In a particular embodiment, the mixture contains at least one diterpene glycoside of the present invention in an amount greater than about 90% by weight on a dry basis, for example, greater than about 91%, greater than about 92%, greater than about 93%, greater than about 94%, greater than about 95%, greater than about 96%, greater than about 97%, greater than about 98% and greater than about 99%.

The term "purified diterpene glycoside", as used herein, refers to a diterpene glycoside present in at least about 50% by weight in a mixture, e.g. stevia extract, such as, for example, at least about 60%, at least about 70%, at least about 80%, at least about 90%, at least about 95% or at least about 97%.

In a particular embodiment, the mixture is an extract of a stevia plant variety. Suitable Stevia varieties include, but are not limited to *S. rebaudiana* Bertoni and *S. rebaudiana* Morita.

The stevia extract may contain one or more additional diterpene glycosides, i.e., diterpene glycosides that are not the diterpene glycosides of the present invention, including, but not limited to, stevioside, rebaudioside A, rebaudioside C, dulcoside A, rubusoside, steviolbioside, rebaudioside B, rebaudioside D, rebaudioside E, rebaudioside F, rebaudioside M, rebaudioside N, rebaudioside O and combinations thereof.

In one embodiment, the present invention is a composition comprising at least one diterpene glycoside described herein provided as a pure compound, i.e. > 99% purity on a dry basis.

The diterpene glycosides of the present invention may be present in the composition in an amount effective to provide a concentration of diterpene glycoside of the present invention from about 1 ppm to about 10,000 ppm when the composition is added to a consumable, such as, for example, from about 1 ppm to about 4,000 ppm, from about 1 ppm to about 3,000 ppm, from about 1 ppm to about 2,000 ppm, from about 1 ppm to about 1,000 ppm.

In another embodiment, the diterpene glycoside of the present invention is present in the composition in an amount effective to provide a concentration of diterpene glycoside of the present invention from about 10 ppm to about 1,000 ppm when the composition is added to a consumable, such as, for example, from about 10 ppm to about 800 ppm, from about 50 ppm to about 800 ppm, or from about 50 ppm to about 600 ppm.

### Sweetener Compositions

As noted above, in some embodiments, the diterpene glycoside of the present invention is sweet. Accordingly, also described herein is a sweetener composition comprising at least one diterpene glycoside of the present invention. "Sweetener composition," as the term is used herein, refers to a mixture of at least one diterpene of the present invention and at least one other substance, wherein the at least one diterpene glycoside is admixed with the at least one other substance.

In a particular embodiment, the at least one other substance does not occur and/or is not admixed with the diterpene glycoside in nature, i.e. the Stevia leaf. As such, the sweetener compositions contemplated by the present invention do not occur in nature. In one embodiment, the at least one other substance modulates the taste profile of the at least one diterpene glycoside to provide a composition with a more sucrose-like taste profile compared to the diterpene glycoside in nature and (if applicable) the at least one other substance in nature. For example, in certain embodiments the composition exhibits one or more of the following characteristics: improved sweetness potency, improved mouthfeel, decreased sweetness linger, decreased bitterness and/or decreased metallic taste.

In certain exemplary embodiments, the sweetener composition comprises at least one purified diterpene glycoside of this invention.

In one embodiment, the diterpene glycoside of the present invention is the sole sweetener in the sweetener composition, i.e. the diterpene glycoside is the only compound present in the sweetener composition that provides a detectable sweetness.

In further embodiments, the sweetener composition comprising at least one diterpene glycoside of the present invention in combination with at least one additional sweetener. In a particular embodiment, the at least one additional sweetener does not occur with the diterpene glycoside in nature, i.e. Stevia leaf. In a more particular embodiment, a sweetener composition comprises at least one purified diterpene glycoside at least one additional sweetener that does not occur with the diterpene glycoside in nature.

The amount of diterpene glycoside of the present invention in the sweetener composition may vary. In one embodiment, the diterpene glycoside of the present invention is present in a sweetener composition in any amount to impart the desired sweetness when the sweetener composition is added to a sweetenable composition or sweetenable consumable. In a particular embodiment, the diterpene glycoside of the present invention is present in a concentration above its threshold sweetness recognition concentration.

In one embodiment, the diterpene glycoside of the present invention is present in the sweetener composition in an amount effective to provide a sucrose equivalence of greater than about 2% (w/v) when the sweetener composition is added to a sweetenable composition or sweetenable consumable, such as, for example, greater than about 3%, about 4%, about 5%, about 6%, about 7%, about 8%, about 9%, about 10%, about 11%, about 12%, about 13% or about 14%.

In some embodiments, a diterpene glycoside of the present invention is present in the sweetener composition in an amount that, when added to a consumable, will provide a concentration of the diterpene glycoside of the present invention from about 1 ppm to about 100 ppm, such as, for example, from about 1 ppm to about 90 ppm, from about 5 ppm to about 80 ppm, from about 5 ppm to about 70 ppm, from about 5 ppm to about 60 ppm, from about 5 ppm to about 50 ppm, from about 5 ppm to about 40 ppm, from about 5 ppm to about 30 ppm, from about 5 ppm to about 20 ppm, or 5 ppm to about 15 ppm.

In other embodiments, a diterpene glycoside of the present invention is present in the sweetener composition in an amount that, when added to a consumable, will provide a concentration of the diterpene glycoside of the present invention greater than about 10 ppm, such as, for example, greater than about 20 ppm, about 30 ppm, about 40 ppm, about 50 ppm, about 60 ppm, about 70 ppm, about 80 ppm, about 90 ppm, about 100 ppm, about 200 ppm, about 300 ppm, about 400 ppm, about 500 ppm, about 600 ppm, about 700 ppm, about 800 ppm or about 900 ppm.

In still other embodiments, a diterpene glycoside of the present invention is present in the sweetener composition in an amount that, when added to a consumable, will provide a concentration of the diterpene glycoside of the present invention from about 1 ppm to about 1,000 ppm, such as, for example, from about 10 ppm to about 1,000 ppm, from about 20 ppm to about 1,000 ppm, from about 30 ppm to about 1,000 ppm, from about 30 ppm to about 1,000 ppm, from about 40 ppm to about 1,000 ppm, from about 50 ppm to about 1,000 ppm, from about 60 ppm to about 1,000 ppm, from about 70 ppm to about 1,000 ppm, from about 80 ppm to about 1,000 ppm, from about 90 ppm to about 1,000 ppm, from about 100 ppm to about 1,000 ppm, from about 200 ppm to about 1,000 ppm, from about 300 ppm to about 1,000 ppm, from about 400 ppm to about 1,000 ppm, from about 500 ppm to about 1,000 ppm, from about 600 ppm to about 1,000 ppm, from about 700 ppm to about 1,000 ppm, from about 800 ppm to about 1,000 ppm, from about 900 ppm to about 1,000 ppm or from about 50 ppm to about 600 ppm.

In one embodiment, the sweetener is at least one natural high-potency sweetener. As used herein, the phrase "natural high potency sweetener" refers to any sweetener found naturally in nature and characteristically has a sweetness potency greater than sucrose, fructose, or glucose, yet has less calories. The natural high potency sweetener can be provided as a pure compound or, alternatively, as part of an extract.

In another embodiment, the sweetener is at least one synthetic sweetener. As used herein, the phrase "synthetic sweetener" refers to any composition which is not found naturally in nature and characteristically has a sweetness potency greater than sucrose, fructose, or glucose, yet has less calories.

In still other embodiments, combinations of natural high potency sweeteners and synthetic sweeteners are contemplated.

In other embodiments, the sweetener is at least one carbohydrate sweetener. Suitable carbohydrate sweeteners are selected from, but not limited to, the group consisting of sucrose, glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, fucose, rhamnose, arabinose, turanose, sialose and combinations thereof.

Other suitable sweeteners include rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside M, rebaudioside N, rebaudioside O, dulcoside A, dulcoside B, rubusoside, stevia, stevioside, mogroside IV, mogroside V, mogroside VI, *Luo han guo,* siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, steviolbioside, hesperitin and cyclocarioside I, sugar alcohols such as erythritol, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, glucosylated steviol glycosides (GSGs) and combinations thereof.

In a particular embodiment, the sweetener is at least one calorie-providing carbohydrate sweetener. Accordingly, incorporation of the sweetness enhancer reduces the quantity of the calorie-providing carbohydrate sweetener that must be used in a given consumable to achieve a particular SE, thereby allowing the preparation of reduced-calorie consumables.

In one embodiment, the sweetener is a caloric sweetener or mixture of caloric sweeteners. In another embodiment, the caloric sweetener is selected from sucrose, fructose, glucose, high fructose corn/starch syrup, a beet sugar, a cane sugar, and combinations thereof.

In another embodiment, the sweetener is a rare sugar selected from allulose, sorbose, lyxose, ribulose, xylose, xylulose, D-allose, L-ribose, D-tagatose, L-glucose, L-fucose, L-arabinose, turanose, kojibiose and combinations thereof.

In still another embodiment, the sweetener is a mixture of at least one natural high potency sweeteners and at least one carbohydrate sweetener. In yet another embodiment, the sweetener is a mixture of at least one synthetic sweetener and at least one carbohydrate sweetener. In a further embodiment, the sweetener is at least one natural high potency sweetener, at least one synthetic sweetener and at least one carbohydrate sweetener.

In exemplary embodiments, a sweetener composition comprising at least one purified diterpene glycoside of the present invention has about 30% or more sweetness compared to a corresponding sweetener composition comprising partially purified diterpene glycoside or Stevia, such as, for example, about 40% or more, about 50% or more, about 60% or more, about 70% or more, about 80% or more or about 90% or more.

In other exemplary embodiments, a sweetener composition comprising at least one purified diterpene glycoside of the present invention has at least about 30% less bitterness (the taste stimulated by certain substances such as quinine, caffeine and sucrose octa-acetate) compared to a corresponding composition comprising partially purified diterpene glycoside or Stevia leaf, such as, for example, at least about 40%, at least about 50%, at least about 60%, at least about 70%, at least about 80% or at least about 90%. In a particular embodiment, a sweetener composition comprising at least one purified diterpene glycoside of the present invention has substantially no bitterness. Methods of measuring bitterness of a compound are known in the art

In still other exemplary embodiments, a sweetener composition comprising at least one purified diterpene glycoside of the present invention has at least about 30% less sweet lingering aftertaste (the intensity of the sweet taste after expectoration) compared to a corresponding sweetener composition comprising partially purified diterpene glycoside or Stevia leaf, such as, for example, at least about 50%, at least about 60%, at least about 70%, at least about 80% or at least about 90%. In a particular embodiment, a sweetener composition comprising at least one purified diterpene glycoside of the present invention has substantially no sweet lingering aftertaste. Methods of measuring sweet lingering aftertaste are known in the art.

In yet other exemplary embodiments, a sweetener composition comprising at least one purified diterpene glycoside of the present invention has at least about 30% less metallic taste (taste associated with metals, tinny or iron) compared to a corresponding sweetener composition comprising partially purified diterpene glycoside or Stevia leaf, such as, for example, at least about 50%, at least about 60%, at least about 70%, at least about 80% or at least about 90%. In a particular embodiment, a sweetener composition comprising at least one purified diterpene glycoside of the present invention has substantially no metallic taste.

In exemplary embodiments, a sweetener composition comprising at least one purified diterpene glycoside of the present invention exhibits a maximal response (maximum sweetness (%SE) achieved with increasing concentration of compound) that is at least about 30% greater compared to a corresponding sweetener composition comprising partially purified diterpene glycoside or Stevia leaf, such as, for example, at least about 40% greater, at least about 50% greater, at least about 60% greater, at least about 70% greater, at least about 80% greater or at least about 90% greater. Methods of measuring the maximal response of a compound are known in the art.

In other exemplary embodiments, a sweetener composition comprising at least one purified diterpene glycoside of the present invention exhibits a sweetness onset (the time until maximum sweetness is experienced) that is at least about 30% shorter than a sweetener composition comprising partially purified diterpene glycoside or Stevia leaf, such as, for example, at least about 40% short, at least about 50% shorter, at least about 60% shorter, at least about 70% shorter, at least about 80% shorter or at least about 90% shorter. Methods of measuring sweetness onset are known in the art.

### Sweetness Enhancers

In a particular embodiment, the diterpene glycoside of the present invention is a sweetness enhancer or modifier. "Sweetness enhancer", as the term is used herein, refers to a compound that enhances, amplifies or potentiates the perception of sweetness of a consumable (e.g. a beverage) when said compound is present in the consumable in a concentration at or below the compound's sweetener recognition threshold, i.e. in a concentration at which compound does not contribute any noticeable sweet taste in the absence of additional sweetener(s).

"Sweetness modifier", as the term is used herein, refers to a compound that changes the taste properties (such as linger, off-notes, or the like) of sweetness of a consumable (e.g. a beverage) when said compound is present in the consumable in a concentration at or below the compound's sweetener recognition threshold.

The term "sweetness enhancer" is synonymous with the terms "sweet taste potentiator," "sweetness potentiator," "sweetness amplifier," and "sweetness intensifier."

In a particular embodiment, the additional sweetener(s) does not naturally occur and/or is not admixed with the at least one diterpene glycoside sweetness enhancer in nature, i.e. Stevia leaf. As such, the sweetness-enhanced consumables contemplated by the present invention do not occur in nature.

In one embodiment, a diterpene glycoside of the present invention may be added directly to the consumable, i.e., not provided in the form of a composition but rather as compound, to enhance sweetness. In this embodiment, a diterpene glycoside of the present invention is added to the consumable at a concentration at or below its sweetness recognition threshold concentration, i.e., a sweetness enhancer. In a particular embodiment, a diterpene glycoside of the present invention is added to the consumable at a concentration below its sweetness recognition threshold concentration, i.e., a sweetness enhancer.

In certain embodiments, a diterpene glycoside of the present invention is a sweetness enhancer or modifier and is added to the consumable in an amount that will provide a concentration of the diterpene glycoside that is at least about 1%, at least about 5%, at least about 10%, at least about 15%, at least about 20%, at least about 25%, at least about 30%, at least about 35%, at least about 40%, at least about 45% or at least about 50% or more below its sweetness recognition threshold.

In some embodiments, the diterpene glycosides of the present invention enhances the sucrose equivalence (SE) of the consumable by at least about 0.5%, about 0.6%, about 0.7%, about 0.8%, about 0.9%, about 1.0%, about 1.5%, about 2.0%, about 2.5%, about 3.0%, about 4.0% or about 5.0%, when compared to the SE of the consumable in the absence of the diterpene glycoside of the present invention.

In other embodiments, at least one diterpene glycoside of the present invention may be added to the consumable in the form of a sweetness enhancing composition. "Sweetness enhancing composition," as the term is used herein, refers to a composition of the present invention - as described above - wherein the composition enhances, amplifies or potentiates the perception of sweetness of a consumable (e.g. a beverage) when a diterpene glycoside of the present invention is present in the sweetness enhancer composition in an amount that will provide a concentration of the diterpene glycoside that is at or below its sweetness recognition threshold when added to the consumable. In a particular embodiment, the diterpene glycoside of the present invention in an amount that will provide a concentration of the diterpene glycoside of that is below its sweetness recognition threshold.

In certain embodiments, a diterpene glycoside of the present invention is present in the sweetness enhancing composition in an amount effective to provide a concentration of the diterpene glycoside that is at least about 1%, at least about 5%, at least about 10%, at least about 15%, at least about 20%, at least about 25%, at least about 30%, at least about 35%, at least about 40%, at least about 45% or at least about 50% or more below its sweetness recognition threshold when the sweetness enhancing composition is added to a consumable.

It is contemplated that the sweetness enhancing composition can include one or more sweetness enhancers or modifiers in addition to at least one diterpene glycoside of the present invention. In one embodiment, the sweetness enhancing composition can include one additional sweetness enhancer. In other embodiments, the composition can include two or more additional sweetness enhancers. In embodiments where two or more sweetness enhancers or modifiers are utilized, each one should be present at or below its respective sweetness recognition threshold concentration.

The one or more other sweetness enhancers or modifiers are selected from, but not limited to, the group consisting of 2-hydroxybenzoic acid, 3-hydroxybenzoic acid, 4-hydroxybenzoic acid, 2,4-dihydroxybenzoic acid, 3,4-dihydroxybenzoic acid, 2,5-dihydroxybenzoic acid, 2,6-dihydroxybenzoic acid, 2,3,4-trihydroxybenzoic acid, 2,4,6-trihydroxybenzoic acid, 3-aminobenzoic acid, 4-aminobenzoic acid, 4-O-β-D-glucosyl-hesperetin dihydrochalcone, MG isomogrosaide V, 4-hydroxycinnamic acid, 4-methoxycinnamic acid, 1-(2-hydroxyphenyl)-3-(4-pyridyl)-1-propanone, 4-ethoxybenzonitrile, 2-methoxy-5-(phenoxymethyl)-phenol, 1-(2, 4-dihydroxyphenyl)-2-(3-methoxy-4-hydroxyphenyl)-ethanone, hesperetin, 2,3',6-trihydroxy-4'-methoxydihydrochalcone, N-(3'-methoxy-4'-hydroxybenzyl)-2,4,6-trihydroxybenzamide, 3'-7-dihydroxy-4'-methoxyflavan, FEMA GRAS flavor 4669, FEMA GRAS flavor 4701, FEMA GRAS flavor 4720, FEMA GRAS flavor 4774, FEMA GRAS flavor 4708, FEMA GRAS flavor 4728, FEMA GRAS flavor 4601, FEMA GRAS flavor 4802, 4-amino-5-(cyclohexyloxy)-2-methylquinoline-3-carboxylic acid, rebaudioside M, rebaudioside N, rebaudioside O, rebaudioside C and combinations thereof.

In one embodiment, addition of the sweetness enhancer or modifier increases the detected sucrose equivalence of the at least one sweetener in a consumable compared to the sucrose equivalence of the same consumable in the absence of the sweetness enhancer.

In a particular embodiment, the consumable is a beverage. According to this embodiment, a diterpene glycoside of the present invention and at least one sweetener is added to a beverage, wherein the diterpene glycoside is present in a concentration at or below its sweetness recognition threshold. In a particular embodiment, the detected sucrose equivalence is increased from about 0.2% to about 5.0%, such as, for example, about 1%, about 2%, about 3%, about 4% or about 5%.

### Flavor Enhancers

In another particular embodiment, the diterpene glycoside of the present invention is a flavor enhancer. "Flavor enhancer", as the term is used herein, refers to a compound that enhances, amplifies or potentiates the perceptions of a flavor ingredient (i.e. any substance that provides sweetness, sourness, saltiness, savoriness, bitterness, metallic taste, etc.) when said compound is present in a consumable (e.g. a beverage) in a concentration at or below the compound's flavor recognition threshold, i.e. in a concentration at which compound does not contribute any noticeable flavor in the absence of any flavor ingredient(s). The term "flavor recognition threshold", as generally used herein, is the lowest known concentration of a compound that is perceivable by the human sense of taste as the particular flavor. The flavor recognition threshold concentration is specific for a particular compound, and can vary based on temperature, matrix, ingredients and/or flavor system.

The term "flavor enhancer" is synonymous with the terms "flavor potentiator," "flavor amplifier," and "flavor intensifier."

In a particular embodiment, the flavor ingredient(s) does not naturally occur and/or is not admixed with the at least one diterpene glycoside sweetness enhancer in nature, i.e. Stevia leaf. As such, the flavor-enhanced consumables contemplated by the present invention do not occur in nature.

In one embodiment, at least one diterpene glycoside of the present invention is added directly to the consumable, i.e., not provided in the form of a composition but rather as a compound, to enhance a flavor. In this embodiment, the diterpene glycoside of the present invention is added to the consumable at a concentration at or below its flavor recognition threshold concentration, i.e., a flavor enhancer. In a particular embodiment, the diterpene glycoside of the present invention is added to the consumable at a concentration below its flavor recognition threshold concentration, i.e., a flavor enhancer.

In certain embodiments, a diterpene glycoside of the present invention is a flavor enhancer and is added to the consumable in an amount that will provide a concentration of the diterpene glycoside that is at least about 1%, at least about 5%, at least about 10%, at least about 15%, at least about 20%, at least about 25%, at least about 30%, at least about 35%, at least about 40%, at least about 45% or at least about 50% or more below its sweetness recognition threshold.

The diterpene glycosides of the present invention enhances the flavor of the consumable by at least about 0.5%, about 0.6%, about 0.7%, about 0.8%, about 0.9%, about 1.0%, about 1.5%, about 2.0%, about 2.5%, about 3.0%, about 4.0% or about 5.0%, when compared to the flavor of the consumable in the absence of the diterpene glycosides of the present invention.

In other embodiments, at least one diterpene glycoside of the present invention may be added to the consumable in the form of a flavor enhancing composition. "Flavor enhancing composition," as the term is used herein, refers to a mixture of at least one diterpene glycoside of the present invention and at least one flavor ingredient, wherein the at least one diterpene is admixed with the at least one flavor ingredient - wherein the composition enhances, amplifies or potentiates the perception of the flavor ingredient in a consumable (e.g. a beverage) when the at least one diterpene glycoside of the present invention is present in the flavor enhancer composition in an amount that will provide a concentration of the diterpene glycoside that is at or below its flavor recognition threshold when added to the consumable. Thus, the flavor enhancing compositions contemplated by the present invention do not occur in nature.

Addition of the flavor enhancing composition increases the detected flavor of the at least one flavor ingredient in the consumable compared to the detected flavor of the same ingredient in the consumable in the absence of the flavor enhancer. Without being bound by theory, the flavor enhancing composition likely does not contribute any noticeable taste to the consumable to which it is added because the flavor enhancer is present in the consumable in a concentration at or below the its flavor recognition threshold.

In one embodiment, the flavor enhancing composition comprises at least one diterpene glycoside of the present invention in an amount effective to provide a concentration of the at least one diterpene glycoside that is at or below its flavor recognition threshold when the flavor enhancing composition is added to a consumable.

In a particular embodiment, a diterpene glycoside of the present invention is present in the flavor enhancing composition in an amount effective to provide a concentration of the diterpene glycoside below its flavor recognition threshold when the flavor enhancing composition is added to a consumable.

In certain embodiment, a diterpene glycoside of the present invention is present in the flavor enhancing composition in an amount that, when added to a consumable, is effective to provide a concentration of the compound that is at least about 1%, at least about 5%, at least about 10%, at least about 15%, at least about 20%, at least about 25%, at least about 30%, at least about 35%, at least about 40%, at least about 45% or at least about 50% or more below its flavor recognition threshold.

A person of skill in the art will be able to select the concentration of the diterpene glycoside of the present invention in the flavor enhancing composition so that it may impart an enhanced flavor to a consumable comprising at least one flavor ingredient.

Suitable flavor ingredients include, but are not limited to, vanillin, vanilla extract, mango extract, cinnamon, citrus, coconut, ginger, viridiflorol, almond, menthol (including menthol without mint), grape skin extract, and grape seed extract. "Flavorant" and "flavoring ingredient" are synonymous and can include natural or synthetic substances or combinations thereof. Flavorants also include any other substance which imparts flavor and may include natural or non-natural (synthetic) substances which are safe for human or animals when used in a generally accepted range. Non-limiting examples of proprietary flavorants include Döhler^{™} Natural Flavoring Sweetness Enhancer K14323 (Döhler^{™}, Darmstadt, Germany), Symrise^{™} Natural Flavor Mask for Sweeteners 161453 and 164126 (Symrise^{™}, Holzminden, Germany), Natural Advantage^{™} Bitterness Blockers 1, 2, 9 and 10 (Natural Advantage^{™}, Freehold, New Jersey, U.S.A.), and Sucramask^{™} (Creative Research Management, Stockton, California, U.S.A.).

In another embodiment, the flavor enhancing composition comprising at least one diterpene glycoside of the present invention enhances flavors (either individual flavors or the overall flavor) when added to the consumable. These flavors include, but are not limited to, fruit flavors, including tropical fruit flavors, and vanilla-caramel type flavors.

The compositions described herein can be customized to provide the desired calorie content. For example, compositions can be "full-calorie", such that they impart the desired sweetness when added to a consumable (such as, for example, a beverage) and have about 120 calories per 8 oz serving. Alternatively, compositions can be "mid-calorie", such that they impart the desired sweetness when added to a consumable (such as, for example, as beverage) and have less than about 60 calories per 8 oz serving. In other embodiments, compositions can be "low-calorie", such that they impart the desired sweetness when added to a consumable (such as, for example, as beverage) and have less than 40 calories per 8 oz serving. In still other embodiments, the compositions can be "zero-calorie", such that they impart the desired sweetness when added to a consumable (such as, for example, a beverage) and have less than 5 calories per 8 oz. serving.

### Additives

The compositions may comprise, in addition to at least one diterpene glycoside of the present invention, one or more additives and/or functional ingredients, detailed herein below.

Exemplary additives include, but not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, caffeine, flavorants and flavoring ingredients, astringent compounds, proteins or protein hydrolysates, surfactants, emulsifiers, plant extracts, flavonoids, alcohols, polymers and combinations thereof.

In one embodiment, the composition further comprises one or more polyols. The term "polyol", as used herein, refers to a molecule that contains more than one hydroxyl group. A polyol may be a diol, triol, or a tetraol which contains 2, 3, and 4 hydroxyl groups respectively. A polyol also may contain more than 4 hydroxyl groups, such as a pentaol, hexaol, heptaol, or the like, which contain 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group.

Non-limiting examples of polyols in some embodiments include maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect taste.

Suitable amino acid additives include, but are not limited to, aspartic acid, arginine, glycine, glutamic acid, proline, threonine, theanine, cysteine, cystine, alanine, valine, tyrosine, leucine, arabinose, trans-4-hydroxyproline, isoleucine, asparagine, serine, lysine, histidine, ornithine, methionine, carnitine, aminobutyric acid (α-, β-, and/or δ-isomers), glutamine, hydroxyproline, taurine, norvaline, sarcosine, and their salt forms such as sodium or potassium salts or acid salts. The amino acid additives also may be in the D- or L-configuration and in the mono-, di-, or tri-form of the same or different amino acids. Additionally, the amino acids may be α-, β-, γ- and/or δ-isomers if appropriate. Combinations of the foregoing amino acids and their corresponding salts (*e.g*., sodium, potassium, calcium, magnesium salts or other alkali or alkaline earth metal salts thereof, or acid salts) also are suitable additives in some embodiments. The amino acids may be natural or synthetic. The amino acids also may be modified. Modified amino acids refers to any amino acid wherein at least one atom has been added, removed, substituted, or combinations thereof (*e.g*., N-alkyl amino acid, N-acyl amino acid, or N-methyl amino acid). Non-limiting examples of modified amino acids include amino acid derivatives such as trimethyl glycine, N-methyl-glycine, and N-methyl-alanine. As used herein, modified amino acids encompass both modified and unmodified amino acids. As used herein, amino acids also encompass both peptides and polypeptides (*e.g*., dipeptides, tripeptides, tetrapeptides, and pentapeptides) such as glutathione and L-alanyl-L-glutamine. Suitable polyamino acid additives include poly-L-aspartic acid, poly-L-lysine (e.g., poly-L-α-lysine or poly-L-ε-lysine), poly-L-ornithine *(e.g.,* poly-L-α-ornithine or poly-L-ε-ornithine), poly-L-arginine, other polymeric forms of amino acids, and salt forms thereof *(e.g.,* calcium, potassium, sodium, or magnesium salts such as L-glutamic acid mono sodium salt). The poly-amino acid additives also may be in the D- or L-configuration. Additionally, the poly-amino acids may be α-,β-, γ-, δ-, and ε-isomers if appropriate. Combinations of the foregoing poly-amino acids and their corresponding salts *(e.g.,* sodium, potassium, calcium, magnesium salts or other alkali or alkaline earth metal salts thereof or acid salts) also are suitable additives in some embodiments. The poly-amino acids described herein also may comprise co-polymers of different amino acids. The poly-amino acids may be natural or synthetic. The poly-amino acids also may be modified, such that at least one atom has been added, removed, substituted, or combinations thereof (e.g., N-alkyl poly-amino acid or N-acyl poly-amino acid). As used herein, poly-amino acids encompass both modified and unmodified poly-amino acids. For example, modified poly-amino acids include, but are not limited to, poly-amino acids of various molecular weights (MW), such as poly-L-α-lysine with a MW of 1,500, MW of 6,000, MW of 25,200, MW of 63,000, MW of 83,000, or MW of 300,000.

Suitable sugar acid additives include, but are not limited to, aldonic, uronic, aldaric, alginic, gluconic, glucuronic, glucaric, galactaric, galacturonic, and salts thereof (e.g., sodium, potassium, calcium, magnesium salts or other physiologically acceptable salts), and combinations thereof.

Suitable nucleotide additives include, but are not limited to, inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, alkali or alkaline earth metal salts thereof, and combinations thereof. The nucleotides described herein also may comprise nucleotide-related additives, such as nucleosides or nucleic acid bases (e.g., guanine, cytosine, adenine, thymine, uracil).

Suitable organic acid additives include any compound which comprises a -COOH moiety, such as, for example, C2-C30 carboxylic acids, substituted hydroxyl C2-C30 carboxylic acids, butyric acid (ethyl esters), substituted butyric acid (ethyl esters), benzoic acid, substituted benzoic acids *(e.g.,* 2,4-dihydroxybenzoic acid), substituted cinnamic acids, hydroxyacids, substituted hydroxybenzoic acids, anisic acid substituted cyclohexyl carboxylic acids, tannic acid, aconitic acid, lactic acid, tartaric acid, citric acid, isocitric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D- or L-configuration.

Suitable organic acid additive salts include, but are not limited to, sodium, calcium, potassium, and magnesium salts of all organic acids, such as salts of citric acid, malic acid, tartaric acid, fumaric acid, lactic acid *(e.g.,* sodium lactate), alginic acid *(e.g.,* sodium alginate), ascorbic acid *(e.g.,* sodium ascorbate), benzoic acid *(e.g.,* sodium benzoate or potassium benzoate), sorbic acid and adipic acid. The examples of the organic acid additives described optionally may be substituted with at least one group chosen from hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phosphor or phosphonato.

Suitable inorganic acid additives include, but are not limited to, phosphoric acid, phosphorous acid, polyphosphoric acid, hydrochloric acid, sulfuric acid, carbonic acid, sodium dihydrogen phosphate, and alkali or alkaline earth metal salts thereof *(e.g.,* inositol hexaphosphate Mg/Ca).

Suitable bitter compound additives include, but are not limited to, caffeine, quinine, urea, bitter orange oil, naringin, quassia, and salts thereof.

Suitable flavorants and flavoring ingredient additives include, but are not limited to, vanillin, vanilla extract, mango extract, cinnamon, citrus, coconut, ginger, viridiflorol, almond, menthol (including menthol without mint), grape skin extract, and grape seed extract. "Flavorant" and "flavoring ingredient" are synonymous and can include natural or synthetic substances or combinations thereof. Flavorants also include any other substance which imparts flavor and may include natural or non-natural (synthetic) substances which are safe for human or animals when used in a generally accepted range. Non-limiting examples of proprietary flavorants include Döhler^{™} Natural Flavoring Sweetness Enhancer K14323 (Döhler^{™}, Darmstadt, Germany), Symrise^{™} Natural Flavor Mask for Sweeteners 161453 and 164126 (Symrise^{™}, Holzminden, Germany), Natural Advantage^{™} Bitterness Blockers 1, 2, 9 and 10 (Natural Advantage^{™}, Freehold, New Jersey, U.S.A.), and Sucramask^{™} (Creative Research Management, Stockton, California, U.S.A.).

Suitable polymer additives include, but are not limited to, chitosan, pectin, pectic, pectinic, polyuronic, polygalacturonic acid, starch, food hydrocolloid or crude extracts thereof (e.g., gum acacia senegal (Fibergum^{™}), gum acacia seyal, carageenan), poly-L-lysine (e.g., poly-L-α-lysine or poly-L-ε-lysine), poly-L-ornithine (e.g., poly-L-α-ornithine or poly-L-ε-ornithine), polypropylene glycol, polyethylene glycol, poly(ethylene glycol methyl ether), polyarginine, polyaspartic acid, polyglutamic acid, polyethylene imine, alginic acid, sodium alginate, propylene glycol alginate, and sodium polyethyleneglycolalginate, sodium hexametaphosphate and its salts, and other cationic polymers and anionic polymers.

Suitable protein or protein hydrolysate additives include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate).

Suitable surfactant additives include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

Suitable flavonoid additives are classified as flavonols, flavones, flavanones, flavan-3-ols, isoflavones, or anthocyanidins. Non-limiting examples of flavonoid additives include, but are not limited to, catechins (e.g., green tea extracts such as Polyphenon^{™} 60, Polyphenon^{™} 30, and Polyphenon^{™} 25 (Mitsui Norin Co., Ltd., Japan), polyphenols, rutins (e.g., enzyme modified rutin Sanmelin^{™} AO (San-fi Gen F.F.I., Inc., Osaka, Japan)), neohesperidin, naringin, neohesperidin dihydrochalcone, and the like.

Suitable alcohol additives include, but are not limited to, ethanol.

Suitable astringent compound additives include, but are not limited to, tannic acid, europium chloride (EuCl₃), gadolinium chloride (GdCl₃), terbium chloride (TbCl₃), alum, tannic acid, and polyphenols (e.g., tea polyphenols).

Exemplary functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

### Saponin

In certain embodiments, the functional ingredient is at least one saponin. As used herein, the at least one saponin may comprise a single saponin or a plurality of saponins as a functional ingredient for the composition provided herein. Generally, according to particular embodiments of this invention, the at least one saponin is present in the composition in an amount sufficient to promote health and wellness.

Saponins are glycosidic natural plant products comprising an aglycone ring structure and one or more sugar moieties. The combination of the nonpolar aglycone and the water soluble sugar moiety gives saponins surfactant properties, which allow them to form a foam when shaken in an aqueous solution.

Non-limiting examples of specific saponins for use in particular embodiments of the invention include group A acetyl saponin, group B acetyl saponin, and group E acetyl saponin.

Saponins can be found in a large variety of plants and plant products, and are especially prevalent in plant skins and barks where they form a waxy protective coating. Several common sources of saponins include soybeans, which have approximately 5% saponin content by dry weight, soapwort plants (*Saponaria*), the root of which was used historically as soap, as well as alfalfa, aloe, asparagus, grapes, chickpeas, yucca, and various other beans and weeds. Saponins may be obtained from these sources by using extraction techniques well known to those of ordinary skill in the art. A description of conventional extraction techniques can be found in U.S. Pat. Appl. No. 2005/0123662.

### Antioxidant

In certain embodiments, the functional ingredient is at least one antioxidant. As used herein, the at least one antioxidant may comprise a single antioxidant or a plurality of antioxidants. Generally, according to particular embodiments of this invention, the at least one antioxidant is present in the composition in an amount sufficient to promote health and wellness.

As used herein "antioxidant" refers to any substance which inhibits, suppresses, or reduces oxidative damage to cells and biomolecules. Without being bound by theory, it is believed that antioxidants inhibit, suppress, or reduce oxidative damage to cells or biomolecules by stabilizing free radicals before they can cause harmful reactions. As such, antioxidants may prevent or postpone the onset of some degenerative diseases.

Examples of suitable antioxidants for embodiments of this invention include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, α-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (e.g., anthocyanidins), gallocatechins, epicatechin and its gallate forms, epigallocatechin and its gallate forms (ECGC) theaflavin and its gallate forms, thearubigins, isoflavone, phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-α-lipoic acid, N-acetylcysteine, emblicanin, apple extract, apple skin extract (applephenon), rooibos extract red, rooibos extract, green, hawthorn berry extract, red raspberry extract, green coffee antioxidant (GCA), aronia extract 20%, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolfberry (gogi) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants for embodiments of this invention include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. A variety of health benefits may be derived from polyphenols, including prevention of cancer, heart disease, and chronic inflammatory disease and improved mental strength and physical strength, for example. Suitable polyphenols for embodiments of this invention include catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

In particular embodiments, the antioxidant is a catechin such as, for example, epigallocatechin gallate (EGCG). Suitable sources of catechins for embodiments of this invention include, but are not limited to, green tea, white tea, black tea, oolong tea, chocolate, cocoa, red wine, grape seed, red grape skin, purple grape skin, red grape juice, purple grape juice, berries, pycnogenol, and red apple peel.

In some embodiments, the antioxidant is chosen from proanthocyanidins, procyanidins or combinations thereof. Suitable sources of proanthocyanidins and procyanidins for embodiments of this invention include, but are not limited to, red grapes, purple grapes, cocoa, chocolate, grape seeds, red wine, cacao beans, cranberry, apple peel, plum, blueberry, black currants, choke berry, green tea, sorghum, cinnamon, barley, red kidney bean, pinto bean, hops, almonds, hazelnuts, pecans, pistachio, pycnogenol, and colorful berries.

In particular embodiments, the antioxidant is an anthocyanin. Suitable sources of anthocyanins for embodiments of this invention include, but are not limited to, red berries, blueberries, bilberry, cranberry, raspberry, cherry, pomegranate, strawberry, elderberry, choke berry, red grape skin, purple grape skin, grape seed, red wine, black currant, red currant, cocoa, plum, apple peel, peach, red pear, red cabbage, red onion, red orange, and blackberries.

In some embodiments, the antioxidant is chosen from quercetin, rutin or combinations thereof. Suitable sources of quercetin and rutin for embodiments of this invention include, but are not limited to, red apples, onions, kale, bog whortleberry, lingonberrys, chokeberry, cranberry, blackberry, blueberry, strawberry, raspberry, black currant, green tea, black tea, plum, apricot, parsley, leek, broccoli, chili pepper, berry wine, and ginkgo.

In some embodiments, the antioxidant is reservatrol. Suitable sources of reservatrol for embodiments of this invention include, but are not limited to, red grapes, peanuts, cranberry, blueberry, bilberry, mulberry, Japanese Itadori tea, and red wine.

In particular embodiments, the antioxidant is an isoflavone. Suitable sources of isoflavones for embodiments of this invention include, but are not limited to, soy beans, soy products, legumes, alfalfa sprouts, chickpeas, peanuts, and red clover.

In some embodiments, the antioxidant is curcumin. Suitable sources of curcumin for embodiments of this invention include, but are not limited to, turmeric and mustard.

In particular embodiments, the antioxidant is chosen from punicalagin, ellagitannin or combinations thereof. Suitable sources of punicalagin and ellagitannin for embodiments of this invention include, but are not limited to, pomegranate, raspberry, strawberry, walnut, and oak-aged red wine.

In particular embodiments, the antioxidant is chlorogenic acid. Suitable sources of chlorogenic acid for embodiments of this invention include, but are not limited to, green coffee, yerba mate, red wine, grape seed, red grape skin, purple grape skin, red grape juice, purple grape juice, apple juice, cranberry, pomegranate, blueberry, strawberry, sunflower, Echinacea, pycnogenol, and apple peel.

### Dietary Fiber

In certain embodiments, the functional ingredient is at least one dietary fiber source. As used herein, the at least one dietary fiber source may comprise a single dietary fiber source or a plurality of dietary fiber sources as a functional ingredient for the compositions provided herein. Generally, according to particular embodiments of this invention, the at least one dietary fiber source is present in the composition in an amount sufficient to promote health and wellness.

Numerous polymeric carbohydrates having significantly different structures in both composition and linkages fall within the definition of dietary fiber. Such compounds are well known to those skilled in the art, non-limiting examples of which include non-starch polysaccharides, lignin, cellulose, methylcellulose, the hemicelluloses, β-glucans, pectins, gums, mucilage, waxes, inulins, oligosaccharides, fructooligosaccharides, cyclodextrins, chitins, and combinations thereof.

Food sources of dietary fiber include, but are not limited to, grains, legumes, fruits, and vegetables. Grains providing dietary fiber include, but are not limited to, oats, rye, barley, wheat,. Legumes providing fiber include, but are not limited to, peas and beans such as soybeans. Fruits and vegetables providing a source of fiber include, but are not limited to, apples, oranges, pears, bananas, berries, tomatoes, green beans, broccoli, cauliflower, carrots, potatoes, celery. Plant foods such as bran, nuts, and seeds (such as flax seeds) are also sources of dietary fiber. Parts of plants providing dietary fiber include, but are not limited to, the stems, roots, leaves, seeds, pulp, and skin.

Although dietary fiber generally is derived from plant sources, indigestible animal products such as chitins are also classified as dietary fiber. Chitin is a polysaccharide composed of units of acetylglucosamine joined by β(1-4) linkages, similar to the linkages of cellulose.

### Fatty Acid

In certain embodiments, the functional ingredient is at least one fatty acid. As used herein, the at least one fatty acid may be single fatty acid or a plurality of fatty acids. Generally, according to particular embodiments of this invention, the at least one fatty acid is present in the composition in an amount sufficient to promote health and wellness.

As used herein, "fatty acid" refers to any straight chain monocarboxylic acid and includes saturated fatty acids, unsaturated fatty acids, long chain fatty acids, medium chain fatty acids, short chain fatty acids, fatty acid precursors (including omega-9 fatty acid precursors), and esterified fatty acids. As used herein, "long chain polyunsaturated fatty acid" refers to any polyunsaturated carboxylic acid or organic acid with a long aliphatic tail. As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" any polyunsaturated fatty acid having a first double bond as the sixth carbon-carbon bond from the terminal methyl end of its carbon chain.

Suitable omega-3 fatty acids for use in embodiments of the present invention can be derived from algae, fish, animals, plants, or combinations thereof, for example. Examples of suitable omega-3 fatty acids include, but are not limited to, linolenic acid, alpha-linolenic acid, eicosapentaenoic acid, docosahexaenoic acid, stearidonic acid, eicosatetraenoic acid and combinations thereof. In some embodiments, suitable omega-3 fatty acids can be provided in fish oils, (e.g., menhaden oil, tuna oil, salmon oil, bonito oil, and cod oil), microalgae omega-3 oils or combinations thereof. In particular embodiments, suitable omega-3 fatty acids may be derived from commercially available omega-3 fatty acid oils such as Microalgae DHA oil (from Martek, Columbia, MD), OmegaPure (from Omega Protein, Houston, TX), Marinol C-38 (from Lipid Nutrition, Channahon, IL), Bonito oil and MEG-3 (from Ocean Nutrition, Dartmouth, NS), Evogel (from Symrise, Holzminden, Germany), Marine Oil, from tuna or salmon (from Arista Wilton, CT), OmegaSource 2000, Marine Oil, from menhaden and Marine Oil, from cod (from OmegaSource, RTP, NC).

Suitable omega-6 fatty acids include, but are not limited to, linoleic acid, gamma-linolenic acid, dihommo-gamma-linolenic acid, arachidonic acid, eicosadienoic acid, docosadienoic acid, adrenic acid, docosapentaenoic acid and combinations thereof.

Suitable esterified fatty acids for embodiments of the present invention may include, but are not limited to, monoacylgycerols containing omega-3 and/or omega-6 fatty acids, diacylgycerols containing omega-3 and/or omega-6 fatty acids, or triacylgycerols containing omega-3 and/or omega-6 fatty acids and combinations thereof.

### Vitamin

In certain embodiments, the functional ingredient is at least one vitamin.

As used herein, the at least one vitamin may be single vitamin or a plurality of vitamins. Generally, according to particular embodiments of this invention, the at least one vitamin is present in the composition in an amount sufficient to promote health and wellness.

Vitamins are organic compounds that the human body needs in small quantities for normal functioning. The body uses vitamins without breaking them down, unlike other nutrients such as carbohydrates and proteins. To date, thirteen vitamins have been recognized, and one or more can be used in the compositions herein. Suitable vitamins include, vitamin A, vitamin D, vitamin E, vitamin K, vitamin B1, vitamin B2, vitamin B3, vitamin B5, vitamin B6, vitamin B7, vitamin B9, vitamin B12, and vitamin C.

Various other compounds have been classified as vitamins by some authorities. These compounds may be termed pseudo-vitamins and include, but are not limited to, compounds such as ubiquinone (coenzyme Q10), pangamic acid, dimethylglycine, taestrile, amygdaline, flavanoids, para-aminobenzoic acid, adenine, adenylic acid, and s-methylmethionine. As used herein, the term vitamin includes pseudo-vitamins.

In some embodiments, the vitamin is a fat-soluble vitamin chosen from vitamin A, D, E, K and combinations thereof.

In other embodiments, the vitamin is a water-soluble vitamin chosen from vitamin B1, vitamin B2, vitamin B3, vitamin B6, vitamin B12, folic acid, biotin, pantothenic acid, vitamin C and combinations thereof.

### Glucosamine

In certain embodiments, the functional ingredient is glucosamine. Generally, according to particular embodiments of this invention, glucosamine is present in the compositions in an amount sufficient to promote health and wellness. The compositions can further comprise chondroitin sulfate.

### Mineral

In certain embodiments, the functional ingredient is at least one mineral. As used herein, the at least one mineral may be single mineral or a plurality of minerals. Generally, according to particular embodiments of this invention, the at least one mineral is present in an amount sufficient to promote health and wellness.

Minerals, in accordance with the teachings of this invention, comprise inorganic chemical elements required by living organisms. Minerals are comprised of a broad range of compositions (e.g., elements, simple salts, and complex silicates) and also vary broadly in crystalline structure. They may naturally occur in foods and beverages, may be added as a supplement, or may be consumed or administered separately from foods or beverages.

Minerals may be categorized as either bulk minerals, which are required in relatively large amounts, or trace minerals, which are required in relatively small amounts. Bulk minerals generally are required in amounts greater than or equal to about 100 mg per day and trace minerals are those that are required in amounts less than about 100 mg per day.

In particular embodiments of this invention, the mineral is chosen from bulk minerals, trace minerals or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral.

In other particular embodiments of this invention, the mineral is a trace mineral, believed to be necessary for human nutrition, non-limiting examples of which include bismuth, boron, lithium, nickel, rubidium, silicon, strontium, tellurium, tin, titanium, tungsten, and vanadium.

The minerals embodied herein may be in any form known to those of ordinary skill in the art. For example, in a particular embodiment the minerals may be in their ionic form, having either a positive or negative charge. In another particular embodiment the minerals may be in their molecular form. For example, sulfur and phosphorous often are found naturally as sulfates, sulfides, and phosphates.

### Preservative

In certain embodiments, the functional ingredient is at least one preservative. As used herein, the at least one preservative may be single preservative or a plurality of preservatives. Generally, according to particular embodiments of this invention, the at least one preservative is present in an amount sufficient to promote health and wellness.

In particular embodiments of this invention, the preservative is chosen from antimicrobials, antioxidants, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

According to a particular embodiment, the preservative is a sulfite. Sulfites include, but are not limited to, sulfur dioxide, sodium bisulfite, and potassium hydrogen sulfite.

According to another particular embodiment, the preservative is a propionate. Propionates include, but are not limited to, propionic acid, calcium propionate, and sodium propionate.

According to yet another particular embodiment, the preservative is a benzoate. Benzoates include, but are not limited to, sodium benzoate and benzoic acid.

In another particular embodiment, the preservative is a sorbate. Sorbates include, but are not limited to, potassium sorbate, sodium sorbate, calcium sorbate, and sorbic acid.

In still another particular embodiment, the preservative is a nitrate and/or a nitrite. Nitrates and nitrites include, but are not limited to, sodium nitrate and sodium nitrite.

In yet another particular embodiment, the at least one preservative is a bacteriocin, such as, for example, nisin.

In another particular embodiment, the preservative is ethanol.

In still another particular embodiment, the preservative is ozone.

Non-limiting examples of antienzymatics suitable for use as preservatives in particular embodiments of the invention include ascorbic acid, citric acid, and metal chelating agents such as ethylenediaminetetraacetic acid (EDTA).

### Hydration Agent

In certain embodiments, the functional ingredient is at least one hydration agent. As used herein, the at least one hydration agent may be single hydration agent or a plurality of hydration agents. Generally, according to particular embodiments of this invention, the at least one hydration agent is present in an amount sufficient to promote health and wellness.

Hydration agents help the body to replace fluids that are lost through excretion. For example, fluid is lost as sweat in order to regulate body temperature, as urine in order to excrete waste substances, and as water vapor in order to exchange gases in the lungs. Fluid loss can also occur due to a wide range of external causes, non-limiting examples of which include physical activity, exposure to dry air, diarrhea, vomiting, hyperthermia, shock, blood loss, and hypotension. Diseases causing fluid loss include diabetes, cholera, gastroenteritis, shigellosis, and yellow fever. Forms of malnutrition that cause fluid loss include the excessive consumption of alcohol, electrolyte imbalance, fasting, and rapid weight loss.

In a particular embodiment, the hydration agent is an electrolyte, non-limiting examples of which include sodium, potassium, calcium, magnesium, chloride, phosphate, bicarbonate, and combinations thereof. Suitable electrolytes for use in particular embodiments of this invention are also described in U.S. Patent No. 5,681,569. In particular embodiments, the electrolytes are obtained from their corresponding water-soluble salts. Non-limiting examples of salts for use in particular embodiments include chlorides, carbonates, sulfates, acetates, bicarbonates, citrates, phosphates, hydrogen phosphates, tartrates, sorbates, citrates, benzoates, or combinations thereof. In other embodiments, the electrolytes are provided by juice, fruit extracts, vegetable extracts, tea, or teas extracts.

In particular embodiments of this invention, the hydration agent is a carbohydrate to supplement energy stores burned by muscles. Suitable carbohydrates for use in particular embodiments of this invention are described in U.S. Patent Numbers 4,312,856, 4,853,237, 5,681,569, and 6,989,171. Non-limiting examples of suitable carbohydrates include monosaccharides, disaccharides, oligosaccharides, complex polysaccharides or combinations thereof. Non-limiting examples of suitable types of monosaccharides for use in particular embodiments include trioses, tetroses, pentoses, hexoses, heptoses, octoses, and nonoses. Non-limiting examples of specific types of suitable monosaccharides include glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, and sialose. Non-limiting examples of suitable disaccharides include sucrose, lactose, and maltose. Non-limiting examples of suitable oligosaccharides include saccharose, maltotriose, and maltodextrin. In other particular embodiments, the carbohydrates are provided by a corn syrup, a beet sugar, a cane sugar, a juice, or a tea.

In another particular embodiment, the hydration agent is a flavanol that provides cellular rehydration. Flavanols are a class of natural substances present in plants, and generally comprise a 2-phenylbenzopyrone molecular skeleton attached to one or more chemical moieties. Non-limiting examples of suitable flavanols for use in particular embodiments of this invention include catechin, epicatechin, gallocatechin, epigallocatechin, epicatechin gallate, epigallocatechin 3-gallate, theaflavin, theaflavin 3-gallate, theaflavin 3'-gallate, theaflavin 3,3' gallate, thearubigin or combinations thereof. Several common sources of flavanols include tea plants, fruits, vegetables, and flowers. In preferred embodiments, the flavanol is extracted from green tea.

In a particular embodiment, the hydration agent is a glycerol solution to enhance exercise endurance. The ingestion of a glycerol containing solution has been shown to provide beneficial physiological effects, such as expanded blood volume, lower heart rate, and lower rectal temperature.

### Probiotics/Prebiotics

In certain embodiments, the functional ingredient is chosen from at least one probiotic, prebiotic and combination thereof. As used herein, the at least one probiotic or prebiotic may be single probiotic or prebiotic or a plurality of probiotics or prebiotics. Generally, according to particular embodiments of this invention, the at least one probiotic, prebiotic or combination thereof is present in an amount sufficient to promote health and wellness.

Probiotics, in accordance with the teachings of this invention, comprise microorganisms that benefit health when consumed in an effective amount. Desirably, probiotics beneficially affect the human body's naturally-occurring gastrointestinal microflora and impart health benefits apart from nutrition. Probiotics may include, without limitation, bacteria, yeasts, and fungi.

Prebiotics, in accordance with the teachings of this invention, are compositions that promote the growth of beneficial bacteria in the intestines. Prebiotic substances can be consumed by a relevant probiotic, or otherwise assist in keeping the relevant probiotic alive or stimulate its growth. When consumed in an effective amount, prebiotics also beneficially affect the human body's naturally-occurring gastrointestinal microflora and thereby impart health benefits apart from just nutrition. Prebiotic foods enter the colon and serve as substrate for the endogenous bacteria, thereby indirectly providing the host with energy, metabolic substrates, and essential micronutrients. The body's digestion and absorption of prebiotic foods is dependent upon bacterial metabolic activity, which salvages energy for the host from nutrients that escaped digestion and absorption in the small intestine.

According to particular embodiments, the probiotic is a beneficial microorganisms that beneficially affects the human body's naturally-occurring gastrointestinal microflora and imparts health benefits apart from nutrition. Examples of probiotics include, but are not limited to, bacteria of the genus *Lactobacilli, Bifidobacteria, Streptococci,* or combinations thereof, that confer beneficial effects to humans.

In particular embodiments of the invention, the at least one probiotic is chosen from the genus *Lactobacilli. Lactobacilli* (i.e., bacteria of the genus *Lactobacillus,* hereinafter "*L*.") have been used for several hundred years as a food preservative and for promoting human health. Non-limiting examples of species of *Lactobacilli* found in the human intestinal tract include *L. acidophilus, L. casei, L. fermentum, L. saliva roes, L. brevis, L. leichmannii, L. plantarum, L. cellobiosus, L. reuteri, L. rhamnosus, L. GG, L. bulgaricus,* and *L. thermophilus, .*

According to other particular embodiments of this invention, the probiotic is chosen from the genus *Bifidobacteria. Bifidobacteria* also are known to exert a beneficial influence on human health by producing short chain fatty acids (e.g., acetic, propionic, and butyric acids), lactic, and formic acids as a result of carbohydrate metabolism. Non-limiting species of *Bifidobacteria* found in the human gastrointestinal tract include *B. angulatum, B. animalis, B. asteroides, B. bifidum, B. boum, B. breve, B. catenulatum, B. choerinum, B. coryneforme, B. cuniculi, B. dentium, B. gallicum, B. gallinarum, B indicum, B. longum, B. magnum, B. merycicum, B. minimum, B. pseudocatenulatum, B. pseudolongum, B. psychraerophilum, B. pullorum, B. ruminantium, B. saeculare, B. scardovii, B. simiae, B. subtile, B. thermacidophilum, B. thermophilum, B. urinalis, and B. sp.*

According to other particular embodiments of this invention, the probiotic is chosen from the genus *Streptococcus. Streptococcus thermophilus* is a gram-positive facultative anaerobe. It is classified as a lactic acid bacteria and commonly is found in milk and milk products, and is used in the production of yogurt. Other non-limiting probiotic species of this bacteria include *Streptococcus salivarus* and *Streptococcus cremoris.*

Probiotics that may be used in accordance with this invention are well-known to those of skill in the art. Non-limiting examples of foodstuffs comprising probiotics include yogurt, sauerkraut, kefir, kimchi, fermented vegetables, and other foodstuffs containing a microbial element that beneficially affects the host animal by improving the intestinal microbalance.

Prebiotics, in accordance with the embodiments of this invention, include, without limitation, mucopolysaccharides, oligosaccharides, polysaccharides, amino acids, vitamins, nutrient precursors, proteins and combinations thereof.

According to a particular embodiment of this invention, the prebiotic is chosen from dietary fibers, including, without limitation, polysaccharides and oligosaccharides. These compounds have the ability to increase the number of probiotics, which leads to the benefits conferred by the probiotics. Non-limiting examples of oligosaccharides that are categorized as prebiotics in accordance with particular embodiments of this invention include fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

According to other particular embodiments of the invention, the prebiotic is an amino acid. Although a number of known prebiotics break down to provide carbohydrates for probiotics, some probiotics also require amino acids for nourishment.

Prebiotics are found naturally in a variety of foods including, without limitation, bananas, berries, asparagus, garlic, wheat, oats, barley (and other whole grains), flaxseed, tomatoes, Jerusalem artichoke, onions and chicory, greens (e.g., dandelion greens, spinach, collard greens, chard, kale, mustard greens, turnip greens), and legumes (e.g., lentils, kidney beans, chickpeas, navy beans, white beans, black beans).

### Weight Management Agent

In certain embodiments, the functional ingredient is at least one weight management agent. As used herein, the at least one weight management agent may be single weight management agent or a plurality of weight management agents. Generally, according to particular embodiments of this invention, the at least one weight management agent is present in an amount sufficient to promote health and wellness.

As used herein, "a weight management agent" includes an appetite suppressant and/or a thermogenesis agent. As used herein, the phrases "appetite suppressant", "appetite satiation compositions", "satiety agents", and "satiety ingredients" are synonymous. The phrase "appetite suppressant" describes macronutrients, herbal extracts, exogenous hormones, anorectics, anorexigenics, pharmaceutical drugs, and combinations thereof, that when delivered in an effective amount, suppress, inhibit, reduce, or otherwise curtail a person's appetite. The phrase "thermogenesis agent" describes macronutrients, herbal extracts, exogenous hormones, anorectics, anorexigenics, pharmaceutical drugs, and combinations thereof, that when delivered in an effective amount, activate or otherwise enhance a person's thermogenesis or metabolism.

Suitable weight management agents include macronutrient selected from the group consisting of proteins, carbohydrates, dietary fats, and combinations thereof. Consumption of proteins, carbohydrates, and dietary fats stimulates the release of peptides with appetite-suppressing effects. For example, consumption of proteins and dietary fats stimulates the release of the gut hormone cholecytokinin (CCK), while consumption of carbohydrates and dietary fats stimulates release of Glucagon-like peptide 1 (GLP-1).

Suitable macronutrient weight management agents also include carbohydrates. Carbohydrates generally comprise sugars, starches, cellulose and gums that the body converts into glucose for energy. Carbohydrates often are classified into two categories, digestible carbohydrates (e.g., monosaccharides, disaccharides, and starch) and non-digestible carbohydrates (e.g., dietary fiber). Studies have shown that non-digestible carbohydrates and complex polymeric carbohydrates having reduced absorption and digestibility in the small intestine stimulate physiologic responses that inhibit food intake. Accordingly, the carbohydrates embodied herein desirably comprise non-digestible carbohydrates or carbohydrates with reduced digestibility. Non-limiting examples of such carbohydrates include polydextrose; inulin; monosaccharide-derived polyols such as erythritol, mannitol, xylitol, and sorbitol; disaccharide-derived alcohols such as isomalt, lactitol, and maltitol; and hydrogenated starch hydrolysates. Carbohydrates are described in more detail herein below.

In another particular embodiment weight management agent is a dietary fat. Dietary fats are lipids comprising combinations of saturated and unsaturated fatty acids. Polyunsaturated fatty acids have been shown to have a greater satiating power than mono-unsaturated fatty acids. Accordingly, the dietary fats embodied herein desirably comprise poly-unsaturated fatty acids, non-limiting examples of which include triacylglycerols.

In a particular embodiment, the weight management agent is an herbal extract. Extracts from numerous types of plants have been identified as possessing appetite suppressant properties. Non-limiting examples of plants whose extracts have appetite suppressant properties include plants of the genus *Hoodia, Trichocaulon, Caralluma, Stapelia, Orbea, Asclepias,* and *Camelia.* Other embodiments include extracts derived from Gymnema Sylvestre, Kola Nut, Citrus Auran tium, Yerba Mate, Griffonia Simplicifolia, Guarana, myrrh, guggul Lipid, and black current seed oil.

The herbal extracts may be prepared from any type of plant material or plant biomass. Non-limiting examples of plant material and biomass include the stems, roots, leaves, dried powder obtained from the plant material, and sap or dried sap. The herbal extracts generally are prepared by extracting sap from the plant and then spray-drying the sap. Alternatively, solvent extraction procedures may be employed. Following the initial extraction, it may be desirable to further fractionate the initial extract (e.g., by column chromatography) in order to obtain an herbal extract with enhanced activity. Such techniques are well known to those of ordinary skill in the art.

In a particular embodiment, the herbal extract is derived from a plant of the genus *Hoodia,* species of which include *H. alstonii, H. currorii, H. dregei, H. flava, H. gordonii, H. jutatae, H. mossamedensis, H. officinalis, H. parviflorai, H. pedicellata, H. pilifera, H. ruschii,* and *H. triebneri. Hoodia* plants are stem succulents native to southern Africa. A sterol glycoside of *Hoodia,* known as P57, is believed to be responsible for the appetite-suppressant effect of the *Hoodia* species.

In another particular embodiment, the herbal extract is derived from a plant of the genus *Caralluma,* species of which include *C*. *indica, C. fimbriata, C. attenuate, C. tuberculata, C. edulis, C. adscendens, C. stalagmifera, C. umbellate, C. penicillata, C. russeliana, C. retrospicens, C. Arabica,* and *C. lasiantha. Carralluma* plants belong to the same Subfamily as *Hoodia,* Asclepiadaceae. *Caralluma* are small, erect and fleshy plants native to India having medicinal properties, such as appetite suppression, that generally are attributed to glycosides belonging to the pregnane group of glycosides, non-limiting examples of which include caratuberside A, caratuberside B, bouceroside I, bouceroside II, bouceroside III, bouceroside IV, bouceroside V, bouceroside VI, bouceroside VII, bouceroside VIII, bouceroside IX, and bouceroside X.

In another particular embodiment, the at least one herbal extract is derived from a plant of the genus *Trichocaulon. Trichocaulon* plants are succulents that generally are native to southern Africa, similar to *Hoodia,* and include the species *T. piliferum* and *T. officinale.*

In another particular embodiment, the herbal extract is derived from a plant of the genus *Stapelia* or *Orbea,* species of which include *S. gigantean* and *O*. *variegate,* respectively. Both *Stapelia* and *Orbea* plants belong to the same Subfamily as *Hoodia,* Asclepiadaceae. Not wishing to be bound by any theory, it is believed that the compounds exhibiting appetite suppressant activity are saponins, such as pregnane glycosides, which include stavarosides A, B, C, D, E, F, G, H, I, J, and K.

In another particular embodiment, the herbal extract is derived from a plant of the genus *Asclepias. Asclepias* plants also belong to the Asclepiadaceae family of plants. Non-limiting examples of *Asclepias* plants include *A. incarnate, A. curassayica, A. syriaca,* and *A. tuberose.* Not wishing to be bound by any theory, it is believed that the extracts comprise steroidal compounds, such as pregnane glycosides and pregnane aglycone, having appetite suppressant effects.

In a particular embodiment, the weight management agent is an exogenous hormone having a weight management effect. Non-limiting examples of such hormones include CCK, peptide YY, ghrelin, bombesin and gastrin-releasing peptide (GRP), enterostatin, apolipoprotein A-IV, GLP-1, amylin, somastatin, and leptin.

In another embodiment, the weight management agent is a pharmaceutical drug. Non-limiting examples include phentenime, diethylpropion, phendimetrazine, sibutramine, rimonabant, oxyntomodulin, floxetine hydrochloride, ephedrine, phenethylamine, or other stimulants.

### Osteoporosis Management Agent

In certain embodiments, the functional ingredient is at least one osteoporosis management agent. As used herein, the at least one osteoporosis management agent may be single osteoporosis management agent or a plurality of osteoporosis management agents. Generally, according to particular embodiments of this invention, the at least one osteoporosis management agent is present in an amount sufficient to promote health and wellness.

Osteoporosis is a skeletal disorder of compromised bone strength, resulting in an increased risk of bone fracture. Generally, osteoporosis is characterized by reduction of the bone mineral density (BMD), disruption of bone micro-architecture, and changes to the amount and variety of non-collagenous proteins in the bone.

In certain embodiments, the osteoporosis management agent is at least one calcium source. According to a particular embodiment, the calcium source is any compound containing calcium, including salt complexes, solubilized species, and other forms of calcium. Non-limiting examples of calcium sources include amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium citrate malate, calcium gluconate, calcium tartrate, calcium lactate, solubilized species thereof, and combinations thereof.

According to a particular embodiment, the osteoporosis management agent is a magnesium soucrce. The magnesium source is any compound containing magnesium, including salt complexes, solubilized species, and other forms of magnesium. Non-limiting examples of magnesium sources include magnesium chloride, magnesium citrate, magnesium gluceptate, magnesium gluconate, magnesium lactate, magnesium hydroxide, magnesium picolate, magnesium sulfate, solubilized species thereof, and mixtures thereof. In another particular embodiment, the magnesium source comprises an amino acid chelated or creatine chelated magnesium.

In other embodiments, the osteoporosis agent is chosen from vitamins D, C, K, their precursors and/or beta-carotene and combinations thereof.

Numerous plants and plant extracts also have been identified as being effective in the prevention and treatment of osteoporosis. Non-limiting examples of suitable plants and plant extracts as osteoporosis management agents include species of the genus *Taraxacum* and *Amelanchier,* as disclosed in U.S. Patent Publication No. 2005/0106215, and species of the genus *Lindera, Artemisia, Acorus, Carthamus, Carum, Cnidium, Curcuma, Cyperus, Juniperus, Prunus, Iris, Cichorium, Dodonaea, Epimedium, Erigonoum, Soya, Mentha, Ocimum, thymus, Tanacetum, Plantago, Spearmint, Bixa, Vitis, Rosemarinus, Rhus,* and *Anethum,* as disclosed in U.S. Patent Publication No. 2005/0079232.

### Phytoestrogen

In certain embodiments, the functional ingredient is at least one phytoestrogen. As used herein, the at least one phytoestrogen may be single phytoestrogen or a plurality of phytoestrogens. Generally, according to particular embodiments of this invention, the at least one phytoestrogen is present in an amount sufficient to promote health and wellness.

Phytoestrogens are compounds found in plants which can typically be delivered into human bodies by ingestion of the plants or the plant parts having the phytoestrogens. As used herein, "phytoestrogen" refers to any substance which, when introduced into a body causes an estrogen-like effect of any degree. For example, a phytoestrogen may bind to estrogen receptors within the body and have a small estrogen-like effect.

Examples of suitable phytoestrogens for embodiments of this invention include, but are not limited to, isoflavones, stilbenes, lignans, resorcyclic acid lactones, coumestans, coumestrol, equol, and combinations thereof. Sources of suitable phytoestrogens include, but are not limited to, whole grains, cereals, fibers, fruits, vegetables, black cohosh, agave root, black currant, black haw, chasteberries, cramp bark, dong quai root, devil's club root, false unicorn root, ginseng root, groundsel herb, licorice, liferoot herb, motherwort herb, peony root, raspberry leaves, rose family plants, sage leaves, sarsaparilla root, saw palmetto berried, wild yam root, yarrow blossoms, legumes, soybeans, soy products (e.g., miso, soy flour, soymilk, soy nuts, soy protein isolate, tempen, or tofu) chick peas, nuts, lentils, seeds, clover, red clover, dandelion leaves, dandelion roots, fenugreek seeds, green tea, hops, red wine, flaxseed, garlic, onions, linseed, borage, butterfly weed, caraway, chaste tree, vitex, dates, dill, fennel seed, gotu kola, milk thistle, pennyroyal, pomegranates, southernwood, soya flour, tansy, and root of the kudzu vine (pueraria root) and the like, and combinations thereof.

Isoflavones belong to the group of phytonutrients called polyphenols. In general, polyphenols (also known as "polyphenolics"), are a group of chemical substances found in plants, characterized by the presence of more than one phenol group per molecule.

Suitable phytoestrogen isoflavones in accordance with embodiments of this invention include genistein, daidzein, glycitein, biochanin A, formononetin, their respective naturally occurring glycosides and glycoside conjugates, matairesinol, secoisolariciresinol, enterolactone, enterodiol, textured vegetable protein, and combinations thereof.

Suitable sources of isoflavones for embodiments of this invention include, but are not limited to, soy beans, soy products, legumes, alfalfa sprouts, chickpeas, peanuts, and red clover.

### Long-Chain Primary Aliphatic Saturated Alcohol

In certain embodiments, the functional ingredient is at least one long chain primary aliphatic saturated alcohol. As used herein, the at least one long chain primary aliphatic saturated alcohol may be single long chain primary aliphatic saturated alcohol or a plurality of long chain primary aliphatic saturated alcohols. Generally, according to particular embodiments of this invention, the at least one long chain primary aliphatic saturated alcohol is present in an amount sufficient to promote health and wellness.

Long-chain primary aliphatic saturated alcohols are a diverse group of organic compounds. The term alcohol refers to the fact these compounds feature a hydroxyl group (-OH) bound to a carbon atom. The term primary refers to the fact that in these compounds the carbon atom which is bound to the hydroxyl group is bound to only one other carbon atom. The term saturated refers to the fact that these compounds feature no carbon to carbon pi bonds. The term aliphatic refers to the fact that the carbon atoms in these compounds are joined together in straight or branched chains rather than in rings. The term long-chain refers to the fact that the number of carbon atoms in these compounds is at least 8 carbons).

Non-limiting examples of particular long-chain primary aliphatic saturated alcohols for use in particular embodiments of the invention include the 8 carbon atom 1-octanol, the 9 carbon 1-nonanol, the 10 carbon atom 1-decanol, the 12 carbon atom 1-dodecanol, the 14 carbon atom 1-tetradecanol, the 16 carbon atom 1-hexadecanol, the 18 carbon atom 1-octadecanol, the 20 carbon atom l-eicosanol, the 22 carbon 1-docosanol, the 24 carbon 1-tetracosanol, the 26 carbon 1-hexacosanol, the 27 carbon 1-heptacosanol, the 28 carbon 1-octanosol, the 29 carbon 1-nonacosanol, the 30 carbon 1-triacontanol, the 32 carbon 1-dotriacontanol, and the 34 carbon 1-tetracontanol.

In a particularly desirable embodiment of the invention, the long-chain primary aliphatic saturated alcohols are policosanol. Policosanol is the term for a mixture of long-chain primary aliphatic saturated alcohols composed primarily of 28 carbon 1-octanosol and 30 carbon 1-triacontanol, as well as other alcohols in lower concentrations such as 22 carbon 1-docosanol, 24 carbon 1-tetracosanol, 26 carbon 1-hexacosanol, 27 carbon 1-heptacosanol, 29 carbon 1-nonacosanol, 32 carbon 1-dotriacontanol, and 34 carbon 1-tetracontanol.

### Phytosterols

In certain embodiments, the functional ingredient is at least one phytosterol, phytostanol or combination thereof. Generally, according to particular embodiments of this invention, the at least one phytosterol, phytostanol or combination thereof is present in an amount sufficient to promote health and wellness.

As used herein, the phrases "stanol", "plant stanol" and "phytostanol" are synonymous.

Plant sterols and stanols are present naturally in small quantities in many fruits, vegetables, nuts, seeds, cereals, legumes, vegetable oils, bark of the trees and other plant sources. Although people normally consume plant sterols and stanols every day, the amounts consumed are insufficient to have significant cholesterol-lowering effects or other health benefits. Accordingly, it would be desirable to supplement food and beverages with plant sterols and stanols.

Sterols are a subgroup of steroids with a hydroxyl group at C-3. Generally, phytosterols have a double bond within the steroid nucleus, like cholesterol; however, phytosterols also may comprise a substituted side chain (R) at C-24, such as an ethyl or methyl group, or an additional double bond. The structures of phytosterols are well known to those of skill in the art.

At least 44 naturally-occurring phytosterols have been discovered, and generally are derived from plants, such as corn, soy, wheat, and wood oils; however, they also may be produced synthetically to form compositions identical to those in nature or having properties similar to those of naturally-occurring phytosterols. According to particular embodiments of this invention, non-limiting examples of phytosterols well known to those or ordinary skill in the art include 4-desmethylsterols (e.g., β-sitosterol, campesterol, stigmasterol, brassicasterol, 22-dehydrobrassicasterol, and ΔS-avenasterol), 4-monomethyl sterols, and 4,4-dimethyl sterols (triterpene alcohols) (e.g., cycloartenol, 24-methylenecycloartanol, and cyclobranol).

As used herein, the phrases "stanol", "plant stanol" and "phytostanol" are synonymous. Phytostanols are saturated sterol alcohols present in only trace amounts in nature and also may be synthetically produced, such as by hydrogenation of phytosterols. According to particular embodiments of this invention, non-limiting examples of phytostanols include β-sitostanol, campestanol, cycloartanol, and saturated forms of other triterpene alcohols.

Both phytosterols and phytostanols, as used herein, include the various isomers such as the α and β isomers (e.g., α-sitosterol and β-sitostanol, which comprise one of the most effective phytosterols and phytostanols, respectively, for lowering serum cholesterol in mammals).

The phytosterols and phytostanols of the present invention also may be in their ester form. Suitable methods for deriving the esters of phytosterols and phytostanols are well known to those of ordinary skill in the art, and are disclosed in U.S. Patent Numbers 6,589,588, 6,635,774, 6,800,317, and U.S. Patent Publication Number 2003/0045473. Non-limiting examples of suitable phytosterol and phytostanol esters include sitosterol acetate, sitosterol oleate, stigmasterol oleate, and their corresponding phytostanol esters. The phytosterols and phytostanols of the present invention also may include their derivatives.

Generally, the amount of functional ingredient in the composition varies widely depending on the particular composition and the desired functional ingredient. Those of ordinary skill in the art will readily ascertain the appropriate amount of functional ingredient for each composition.

In one embodiment, a method for preparing a composition comprises combining at least one diterpene glycoside of the present invention and at least one sweetener and/or additive and/or functional ingredient.

In a particular embodiment, a method for preparing a composition comprises combining at least one diterpene glycoside of the present invention and at least one sweetener and/or additive and/or functional ingredient, wherein the at least one sweetener and/or additive and/or functional ingredient does not exist with (is not admixed with) the at least one diterpene glycoside in nature, i.e. Stevia leaf, and the composition provides a more sucrose-like taste profile compared to the diterpene glycoside in nature and (if applicable) the at least one sweetener and/or additive and/or functional ingredient in nature. For example, in certain embodiments the composition exhibits one or more of the following characteristics: improved sweetness potency, improved mouthfeel, decreased sweetness linger, decreased bitterness and/or decreased metallic taste.

### Consumables

The diterpene glycoside(s) described herein, or a composition comprising the same, can be admixed with any known edible or oral composition, referred to herein as a "consumable". Consumables, as used herein, mean substances which are contacted with the mouth of man or animal, including substances which are taken into and subsequently ejected from the mouth and substances which are drunk, eaten, swallowed or otherwise ingested, and are safe for human or animal consumption when used in a generally acceptable range.

Exemplary consumables include pharmaceutical compositions, edible gel mixes and compositions, dental compositions, foodstuffs (confections, condiments, chewing gum, cereal compositions baked goods dairy products, and tabletop sweetener compositions) beverages and beverage products. The consumables of the present invention require admixing and, as such, do not occur in nature.

For example, a beverage is a consumable. The beverage may be sweetened or unsweetened. The diterpene glycoside(s) described herein, or a composition comprising the same, may be added to a beverage or beverage matrix to sweeten the beverage or enhance its existing sweetness or flavor.

In an embodiment, the present invention is a beverage or beverage product comprising a composition that comprises the isolated and purified diterpene glycoside CC-00350.

As used herein a "beverage product" is a ready-to-drink beverage, a beverage concentrate, a beverage syrup, or a powdered beverage. Suitable ready-to-drink beverages include carbonated and non-carbonated beverages. Carbonated beverages include, but are not limited to, enhanced sparkling beverages, cola, lemon-lime flavored sparkling beverage, orange flavored sparkling beverage, grape flavored sparkling beverage, strawberry flavored sparkling beverage, pineapple flavored sparkling beverage, ginger-ale, soft drinks and root beer. Non-carbonated beverages include, but are not limited to fruit juice, fruit-flavored juice, juice drinks, nectars, vegetable juice, vegetable-flavored juice, sports drinks, energy drinks, enhanced water drinks, enhanced water with vitamins, near water drinks (e.g., water with natural or synthetic flavorants), coconut water, tea type drinks (e.g. black tea, green tea, red tea, oolong tea), coffee, cocoa drink, beverage containing milk components (e.g. milk beverages, coffee containing milk

components, café au lait, milk tea, fruit milk beverages), beverages containing cereal extracts, smoothies and combinations thereof.

Beverage concentrates and beverage syrups are prepared with an initial volume of liquid matrix (e.g. water) and the desired beverage ingredients. Full strength beverages are then prepared by adding further volumes of water. Powdered beverages are prepared by dry-mixing all of the beverage ingredients in the absence of a liquid matrix. Full strength beverages are then prepared by adding the full volume of water.

Beverages comprise a matrix, i.e. the basic ingredient in which the ingredients - including the compositions of the present invention - are dissolved. In one embodiment, a beverage comprises water of beverage quality as the matrix, such as, for example deionized water, distilled water, reverse osmosis water, carbon-treated water, purified water, demineralized water and combinations thereof, can be used. Additional suitable matrices include, but are not limited to phosphoric acid, phosphate buffer, citric acid, citrate buffer and carbon-treated water.

In one embodiment, the present invention is a beverage comprising at least the diterpene glycoside CC-00350.

In a further embodiment, the present invention is a beverage product comprising at least the diterpene glycoside CC-00350.

The diterpene glycoside CC-00350 can be provided as a single compound or as part of any composition described above.

In a particular embodiment, a beverage or beverage product comprises at least the diterpene glycoside CC-00350in purified form and at least one other substance that does not occur with the diterpene glycoside in nature, i.e. Stevia leaf. In one embodiment, the at least other additional substance modulates the taste profile of the diterpene glycoside to provide a beverage with a more sucrose-like taste profile compared to the diterpene glycoside in nature and (if applicable) the at least one other substance in nature. For example, in certain embodiments the beverage exhibits one or more of the following characteristics: improved sweetness potency, improved mouthfeel, decreased sweetness linger, decreased bitterness and/or decreased metallic taste.

The concentration of the diterpene glycoside in the beverage may be above, at or below the threshold sweetness or flavor recognition concentration of the diterpene glycoside.

The diterpene glycoside CC-00350is present in the beverage in a concentration from about 25 ppm to about 600 ppm, such as, for example, from about 25 ppm to about 500 ppm, from about 25 ppm to about 400 ppm, from about 25 ppm to about 300 ppm, from about 25 ppm to about 200 ppm, from about 25 ppm to about 100 ppm, from about 50 ppm to about 600 ppm, from about 50 ppm to about 500 ppm, from about 50 ppm to about 400 ppm, from about 50 ppm to about 300 ppm, from about 50 ppm to about 200 ppm, from about 50 ppm to about 100 ppm, from about 100 ppm to about 600 ppm, from about 100 ppm to about 500 ppm, from about 100 ppm to about 400 ppm, from about 100 ppm to about 300 ppm, from about 100 ppm to about 200 ppm, from about 200 ppm to about 600 ppm, from about 200 ppm to about 500 ppm, from about 200 ppm to about 400 ppm, from about 200 ppm to about 300 ppm, from about 300 ppm to about 600 ppm, from about 300 ppm to about 500 ppm, from about 300 ppm to about 400 ppm, from about 400 ppm to about 600 ppm, from about 400 ppm to about 500 ppm or from about 500 ppm to about 600 ppm.

In other particular embodiments, the diterpene glycoside is present in the beverage in a purity of at least about 5% with respect to a mixture of diterpene glycosides or stevia extract, such as, for example, at least about 10%, at least about 20%, at least about 30%, at least about 40%, at least about 50%, at least about 60%, at least about 70%, at least about 80%, at least about 90%, at least about 95% or at least about 97%. In still other embodiments, a diterpene glycoside of the present invention is present in the beverage in >99% purity.

The beverage can include one or more sweeteners. Any of the sweeteners detailed herein can be used, including natural, non-natural, or synthetic sweeteners. These may be added to the beverage either before, contemporaneously with or after the diterpene glycoside(s) of the present invention. In a particular embodiment, the sweetener does not occur with the at least one diterpene glycoside in nature, i.e. Stevia leaf.

The beverage or beverage productcan optionally include additives, functional ingredients and combinations thereof, as described herein. Any of the additives and functional ingredients described above can be present in the consumable. In certain embodiments, the additive and/or functional ingredient modulates the taste profile of the at least one diterpene glycoside to provide a composition with a more sucrose-like taste profile compared to the diterpene glycoside in nature and (if applicable) the additive and/or functional ingredient in nature. For example, in certain embodiments the composition exhibits one or more of the following characteristics: improved sweetness potency, improved mouthfeel, decreased sweetness linger, decreased bitterness and/or decreased metallic taste.

It is contemplated that the pH of the beverage or beverage product, such as, for example, a beverage, does not materially or adversely affect the taste of the sweetener. A non-limiting example of the pH range of the beverage may be from about 1.8 to about 10. A further example includes a pH range from about 2 to about 5. In a particular embodiment, the pH of beverage can be from about 2.5 to about 4.2. On of skill in the art will understand that the pH of the beverage can vary based on the type of beverage. Dairy beverages, for example, can have pHs greater than 4.2.

The titratable acidity of a beverage may, for example, range from about 0.01 to about 1.0% by weight of beverage.

In one embodiment, the sparkling beverage product has an acidity from about 0.01 to about 1.0% by weight of the beverage, such as, for example, from about 0.05% to about 0.25% by weight of beverage.

The carbonation of a sparkling beverage product has 0 to about 2% (w/w) of carbon dioxide or its equivalent, for example, from about 0.1 to about 1.0% (w/w).

The temperature of a beverage may, for example, range from about 4°C to about 100 °C, such as, for example, from about 4°C to about 25°C.

The beverage can be a full-calorie beverage that has up to about 120 calories per 8 oz serving.

The beverage can be a mid-calorie beverage that has up to about 60 calories per 8 oz serving.

The beverage can be a low-calorie beverage that has up to about 40 calories per 8 oz serving.

The beverage can be a zero-calorie that has less than about 5 calories per 8 oz. serving.

In one embodiment, the beverage comprises natural sweetener(s) only, i.e. the only type of sweetener(s) are naturally-occurring.

In exemplary embodiments, a beverage of the present invention has about 30% or more sweetness compared to a corresponding beverage comprising partially purified diterpene glycoside or Stevia, such as, for example, about 40% or more, about 50% or more, about 60% or more, about 70% or more, about 80% or more or about 90% or more.

In other exemplary embodiments, a beverage of the present invention has at least about 30% less bitterness (the taste stimulated by certain substances such as quinine, caffeine and sucrose octa-acetate) compared to a corresponding beverage comprising partially purified diterpene glycoside or Stevia leaf, such as, for example, at least about 40%, at least about 50%, at least about 60%, at least about 70%, at least about 80% or at least about 90%. In a particular embodiment, a beverage of the present invention has substantially no bitterness.

In still other exemplary embodiments, a beverage of the present invention has at least about 30% less sweet lingering aftertaste (the intensity of the sweet taste after expectoration) compared to a corresponding beverage comprising partially purified diterpene glycoside or Stevia leaf, such as, for example, at least about 50%, at least about 60%, at least about 70%, at least about 80% or at least about 90%. In a particular embodiment, a beverage of the present invention has substantially no sweet lingering aftertaste.

In yet other exemplary embodiments, a beverage of the present invention has at least about 30% less metallic taste (taste associated with metals, tinny or iron) compared to a corresponding beverage comprising partially purified diterpene glycoside or Stevia leaf, such as, for example, at least about 50%, at least about 60%, at least about 70%, at least about 80% or at least about 90%. In a particular embodiment, a beverage of the present invention has substantially no metallic taste.

In exemplary embodiments, a beverage of the present invention exhibits a maximal response (maximum sweetness (%SE) achieved with increasing concentration of compound) that is at least about 30% greater compared to a corresponding beverage comprising partially purified diterpene glycoside or Stevia leaf, such as, for example, at least about 40% greater, at least about 50% greater, at least about 60% greater, at least about 70% greater, at least about 80% greater or at least about 90% greater.

In other exemplary embodiments, a beverage of the present invention exhibits a sweetness onset (the time until maximum sweetness is experienced) that is at least about 30% shorter than a beverage comprising partially purified diterpene glycoside or Stevia leaf, such as, for example, at least about 40% short, at least about 50% shorter, at least about 60% shorter, at least about 70% shorter, at least about 80% shorter or at least about 90% shorter.

### III. Methods of Use

The compounds and compositions described herein can be used to impart sweetness or to enhance the flavor or sweetness of consumables or other compositions.

A method of preparing a sweetened consumable comprises (i) providing a consumable and (ii) adding at least one diterpene glycoside of the present invention to the consumable to provide a sweetened consumable.

A method of preparing a sweetened consumable comprises (i) providing an unsweetened consumable and (ii) adding at least one diterpene glycoside of the present invention to the unsweetened consumable to provide a sweetened consumable.

A method of preparing a sweetened beverage comprises (i) providing a beverage and (ii) adding at least one diterpene glycoside of the present invention to the beverage to provide a sweetened beverage.

A method of preparing a sweetened beverage comprises (i) providing an unsweetened beverage and (ii) adding at least one diterpene glycoside of the present invention to the unsweetened beverage to provide a sweetened beverage.

In the above methods, the diterpene glycoside(s) of the present invention may be provided as such, i.e., in the form of a compound, or in form of a composition. When provided as a composition, the amount of diterpene glycoside in the composition is effective to provide a concentration of the diterpene glycoside that is above, at or below its flavor or sweetness recognition threshold when the composition is added to the consumable (e.g., the beverage). When the diterpene glycoside(s) of the present invention is not provided as a composition, it may be added to the consumable at a concentration that is above, at or below its flavor or sweetness recognition threshold.

A method for enhancing the sweetness of a consumable comprises (i) providing a consumable comprising at least one sweet ingredient and (ii) adding at least one diterpene glycoside of the present invention, or a composition comprising the same, to the consumable to provide a consumable with enhanced sweetness, wherein the diterpene glycoside of the present invention is added to the consumable at a concentration at or below its sweetness recognition threshold. In a particular embodiment, a diterpene glycoside of the present invention is added to the consumable at a concentration below its sweetness recognition threshold.

A method for enhancing the sweetness of a beverage comprises (i) providing a beverage comprising at least one sweet ingredient and (ii) adding at least one diterpene glycoside of the present invention, or a composition comprising the same, to the beverage to provide a beverage with enhanced sweetness, wherein the diterpene glycoside is added to the beverage at a concentration at or below its sweetness recognition threshold. In a particular embodiment, the diterpene glycoside of the present invention is added to the consumable at a concentration below its sweetness recognition concentration threshold.

A method for enhancing the flavor of a consumable comprises (i) providing a consumable comprising at least one flavor ingredient and (ii) adding at least one diterpene glycoside of the present invention, or a composition comprising the same, to the consumable to provide a consumable with enhanced flavor, wherein the diterpene glycoside of the present invention is added to the consumable at a concentration at or below its flavor recognition threshold. In a particular embodiment, the diterpene glycoside of the present invention is added to the consumable at a concentration below its flavor recognition threshold.

A method for enhancing the flavor of a beverage comprises (i) providing a beverage comprising at least one flavor ingredient and (ii) adding at least one diterpene glycoside of the present invention, or a composition comprising the same, to the beverage to provide a beverage with enhanced flavor, wherein the diterpene glycoside is added to the beverage at a concentration at or below the flavor recognition threshold of the diterpene glycoside. In a particular embodiment, the diterpene glycoside of the present invention is added to the consumable at a concentration below its flavor recognition threshold.

### IV. Methods of Purification

Aa method for purifying a diterpene glycoside of the present invention compries (i) passing a solution comprising a source material comprising a diterpene glycoside of the present invention through a HPLC column and (ii) eluting fractions comprising a diterpene glycoside of the present invention to provide purified diterpene glycoside of the present invention. The HPLC column can be any suitable HPLC preparative or semi-preparative scale column.

As used herein, the term "preparative HPLC" refers to an HPLC system capable of producing high (500 or more) microgram, milligram, or gram sized product fractions. The term "preparative" includes both preparative and semi-preparative columns, but is not intended to include analytical columns, which provide fractions in the nanogram to low microgram range.

As used herein, an "HPLC compatible detector" is a detector suitable for use in an HPLC system which is capable of providing a detectable signal upon elution of a compound peak. For example, a detector capable of generating a signal when a compound elutes from the compound is an HPLC compatible detector. Where component absorbance varies widely, it may be necessary to utilize more than one detector. A detector capable of detecting a desired component is not an "incompatible" detector due to its inability to detect a non-desired peak.

An HPLC device typically includes at least the following components: a column, packed with a suitable stationary phase, a mobile phase, a pump for forcing the mobile phase through the column under pressure, and a detector for detecting the presence of compounds eluting off of the column. The devices can optionally include a means for providing for gradient elution, although such is not necessary using the methods described herein. Routine methods and apparatus for carrying out HPLC separations are well known in the art.

Suitable stationary phases are those in which the compound of interest elutes. Preferred columns can be, and are not limited to, normal phase columns (neutral, acidic or basic), reverse phase columns (of any length alkyl chain), a synthetic crosslinked polymer columns (e.g., styrene and divinylbenzene), size exclusion columns, ion exchange columns, bioaffinity columns, and any combination thereof. The particle size of the stationary phase is within the range from a few µm to several 100 µm.

Suitable detection devices include, but are not limited to, mass spectrometers, UV detectors, IR detectors and light scattering detectors. The methods described herein use any combination of these detectors. The most preferable embodiment uses mass spectrometers and UV detectors.

"Source material", as used herein, refers to the material being purified by the present method. The source material contains a diterpene glycoside of the present invention in a purity less than the purity provided by the present purification method. The source material can be liquid or solid. Exemplary source materials include, but are not limited to, mixtures of diterpene glycosides, stevia extract, Stevia plant leaves, by-products of other diterpene glycosides' isolation and purification processes, commercially available diterpene extracts or stevia extracts, by-products of biotransformation reactions of other diterpene glycosides, or any combination thereof.

As understood by persons skilled in the art, any solid source materials must be brought into solution prior to carrying out the HPLC method.

In one embodiment, a representative analytical HPLC protocol is correlated to a preparative or semi-preparative HPLC protocol used to purify a compound.

In another embodiment, appropriate conditions for purifying a diterpene glycoside of the present invention can be worked out by route scouting a representative sample for a given analytical HPLC column, solvent system and flow rate. In yet another embodiment, a correlated preparative or semipreparative HPLC method can be applied to purify a diterpene glycoside of the present invention with modifications to the purification parameters or without having to change the purification parameters.

In some embodiments, the eluent (mobile phase) is selected from the group consisting of water, acetonitrile, methanol, 2-propanol, ethyl acetate, dimethylformamide, dimethylsulfide, pyridine, triethylamine, formic acid, trifluoroacetic acid, acetic acid, an aqueous solution containing ammonium acetate, heptafluorobutyric acid, and any combination thereof.

In one embodiment, the HPLC method is isocratic. In another embodiment, the HPLC method is a gradient. In still another embodiment, the HPLC method is step-wise.

In one embodiment, impurities are eluted off of the HPLC column after eluting one or more fractions containing a diterpene glycoside of the present invention. In another embodiment, impurities are eluted off of the HPLC column before eluting one or more fractions containing a diterpene glycoside of the present invention.

The method can further include removal of solvent from the eluted solution, i.e. drying. In one embodiment, the method further comprises partial removal of solvents from the eluted solution to provide a concentrate comprising a diterpene glycoside of the present invention. In another embodiment, the method further comprises removing substantially all the solvent from the eluted solutions to provide substantially dry material comprising a diterpene glycoside of the present invention.

Removal of solvent can be performed by any known means to one of skill in the art including, but not limited to, evaporation, distillation, vacuum drying and spray drying.

The resulting purified fractions comprising a diterpene glycoside of the present invention can be further purified by other methods to increase purity. Suitable methods include, but are not limited to, crystallization, chromatography, extraction and distillation. Such methods are well-known to persons skilled in the art.

The source material can be one fraction, or multiple fractions, containing a diterpene glycoside of the present invention collected from at least one previous method or HPLC protocol. In one embodiment, multiple fractions from the same, previous methods or HPLC protocols are pooled and optionally, solvents are removed, prior to re-subjecting the source material to another method. In other embodiments, fractions from different, previous methods or HPLC protocol are pooled, and optionally, solvents are removed, prior to re-subjecting the source material to another method.

In one embodiment, the source material re-subjected to additional method(s) comprises liquid fractions obtained from one or more previous (and optionally, different) methods mixed with substantially dry material obtained via drying of fractions obtained from one or more previous (and optionally, different) methods. In another embodiment, the source material re-subjected to additional method(s) comprises substantially dry material obtained via drying of fractions obtained from one or more previous (and optionally, different) methods, where said source material is brought into solution prior to passing the solution through the next HPLC column.

The second and subsequent methods may have different HPLC protocols (e.g. solvent systems, columns, methods) and different steps following elution (e.g. partial removal of solvent, complete removal of solvent, elution of impurities, use of crystallization or extraction).

The material isolated can be subjected to further methods 2, 3, 4 or more times, each time providing a higher level of purity of purified diterpene glycoside of the present invention.

The method may provide a purified diterpene glycoside of the present invention in a purity of about 50% by weight or greater on a dry basis, such as, for example, about 60% or greater, about 65% or greater, about 70% or greater, about 75% or greater, about 80% or greater, about 85% or greater, about 90% or greater, about 95% or greater and about 97% or greater. For example, the method may provide a diterpene glycoside of the present invention in a purity greater of about 99% or greater by weight on a dry basis.

### EXAMPLES

### EXAMPLE 1: Isolation and Characterization of CC-00336 (Reference)

The sample used for the isolation of **CC-00336** was Stevia extract.

HPLC analyses were performed on a Waters 2695 Alliance System coupled to a Waters 996 Photo Diode Array (PDA) detector. In addition, sample purities were assessed using an ESA Corona Charged Aerosol Detector (CAD). Sample analyses were performed using the method conditions described in Tables 1-4.

**Table 1: Analytical HPLC Conditions for Fraction Analysis in Primary Process**

| | | |
|---|---|---|
| **Column** | Phenomenex Synergi Hydro RP (4.6 × 250 mm, 4 µm) | |
| **Column Temperature (°C)** | 55 | |
| **Sample Temperature (°C)** | Ambient | |
| **Mobile Phases** | (A) 0.0284% ammonium acetate (NH₄OAc) and 0.0116% acetic acid (HOAc) in water | |

| | | |
|---|---|---|
| | (B) MeCN | |
| **Flow Rate (mL/min)** | 1.0 | |
| **Detection** | CAD and UV at 210 nm | |

| **Gradient** | | |
|---|---|---|
| **Time (min)** | **% A** | **%B** |
| 0.0-8.5 | 75 | 25 |
| 10.0 | 71 | 29 |
| 16.5 | 70 | 30 |
| 18.5 - 24.5 | 66 | 34 |
| 26.5 - 29.0 | 48 | 52 |
| 31.0-37.0 | 30 | 70 |
| 38.0 | 75 | 25 |

**Table 2: Analytical HPLC Conditions for Fraction Analysis in Secondary Process**

| | | |
|---|---|---|
| **Column (Dimensions, mm)** | XBridge Phenyl (4.6 × 150 mm, 3.5 µm) PN186003352, SN012634135129 | |
| **Column Temperature (°C)** | Ambient | |
| **Sample Temperature (°C)** | Ambient | |
| **Mobile Phases** | (A) Water | |
| | (B) MeCN | |
| **Detection** | CAD and UV at 210 nm | |
| **Flow Rate (mL/min)** | 1 mL/min | |

| **Gradient** | | |
|---|---|---|
| **Time (min)** | **% A** | **%B** |
| 0.0 - 20.0 | 80 | 20 |
| 20.1 - 25.0 | 50 | 50 |
| 25.1 | 80 | 20 |

**Table 3: Analytical HPLC Conditions for Fraction Analysis in Tertiary Process**

| | | | |
|---|---|---|---|
| **Column (Dimensions, mm)** | | Waters XBridge Amide (4.6 × 150 mm, 3.5 µm) PN186004869, SN 01253516613401 | |
| **Column Temperature (°C)** | | Ambient | |
| **Sample Temperature (°C)** | | Ambient | |
| **Mobile Phases** | | (A) Water | |
| | | (B) MeCN | |
| **Detection** | | CAD and PDA at 210 nm | |
| **Flow Rate (mL/min)** | | 1 mL/min | |

| **Gradient** | | | |
|---|---|---|---|
| **Time (min)** | **% A** | | **%B** |
| 0.0 | 15 | | 85 |
| 20.0 | 30 | | 70 |
| 20.01 - 25.0 | 50 | | 50 |
| 37.01 | 15 | | 85 |

**Table 4: Analytical HPLC Conditions for Final Purity Evaluation**

| | | |
|---|---|---|
| Column | Phenomenex Synergi Hydro RP (4.6 × 250 mm, 4 µm) | |
| Column Temperature (°C) | 55 | |
| Sample Temperature (°C) | Ambient | |
| Mobile Phases | (A) 0.0284% Ammonium Acetate (NH₄OAc) and 0.0116% Acetic Acid (HOAc) in Water | |
| | (B) Acetonitrile (MeCN) | |
| Flow Rate (mL/min) | 1.0 | |
| Detection | CAD and UV at 210 nm | |

| **Gradient** | | |
|---|---|---|
| **Time (min)** | **% A** | **%B** |
| 0.0 | 85 | 15 |
| 28.5 | 75 | 25 |
| 30.0 | 71 | 29 |
| 36.5 | 70 | 30 |
| 38.5 - 44.5 | 66 | 34 |
| 46.5 - 49.0 | 48 | 52 |
| 51.0-57.0 | 30 | 70 |
| 58.0 | 85 | 15 |

Primary Preparative HPLC Method. Primary processing was performed using either a prepacked Waters XBridge RP18 column (50 × 250 mm, 7 µm) or Phenomenex Luna C18 (2) column (50 × 250 mm). The purification process was performed with a Waters Delta Prep LC Model 2000/4000 system coupled to a UV-Vis detector. Details of the preparative method are summarized in Table 5.

**Table 5: Conditions for Primary Preparative HPLC Method.**

| | | | | |
|---|---|---|---|---|
| **Column** | Waters XBridge RP18 (50 × 250 mm, 7 µm) or Phenomenex Luna C18 (2) (50 × 250 mm) @ ambient | | | |
| **Flow Rate (mL/min)** | 100 | | | |
| **Detection** | UV at 220 nm | | | |
| **Mobile Phases** | | (A) 65:35 Water/Methanol (MeOH) with 0.05% HOAc | | |
| | | (B) 40:60 Water/Methanol (MeOH) with 0.05% HOAc | | |
| | | (C) MeOH | | |
| **Sample preparation** | 4-10 g dissolved in 80 mL of MP-A | | | |

| **Gradient** | | | | |
|---|---|---|---|---|
| **Time (min)** | **% A** | | **%B** | **% C** |
| 0-35 | 100 | | 0 | 0 |
| 50-63 | 0 | | 100 | 0 |
| 63.1-68 | 0 | | 0 | 100 |
| 68.1 | 100 | | 0 | 0 |

Secondary Preparative HPLC Method. Secondary processing was performed using a Waters XBridge Phenyl (19 × 250 mm, 5 µm, PN 186004024, SN 125I130651II02) column. The purification process was performed with a Waters Delta Prep LC Model 2000/4000 system coupled to a UV-Vis detector. Details of the preparative method are summarized in Table 6.

**Table 6: Conditions for Secondary Preparative HPLC Method.**

| | |
|---|---|
| **Column** | Waters XBridge Phenyl (19 × 250 mm, 5 µm) |
| **Flow Rate (mL/min)** | 20 |
| **Detection** | UV at 210 nm |
| **Mobile Phases** | (A) 20% MeCN in Water |
| | (B) MeCN |
| **Sample preparation** | 250 mg in 25 mg/mL in mostly water |
| **Gradient:** | Isocratic 100% MP-A for 55 min |

Tertiary Processing Method. The tertiary processing was performed using a Waters XBridge Amide (19 × 250 mm, 5 µm, PN 186006606, SN 0107341600112 02)) column. The purification process was performed with a Waters Delta Prep LC Model 2000/4000 system coupled to a UV-Vis detector. Details of the preparative method are summarized in Table 7.

**Table 7: Conditions for Tertiary HPLC Process.**

| | | |
|---|---|---|
| **Column** | Waters XBridge Amide (19 × 250 mm, 5 µm) | |
| **Flow Rate (mL/min)** | 20 | |
| **Detection** | UV at 210 nm | |
| **Mobile Phases** | | (A) 15% Water in MeCN |
| | | (B) 30% Water in MeCN |
| **Sample preparation** | dissolved in minimum volume of MP-A | |
| **Gradient:** | 30-min linear ramp from 100% MP-A to 100% MP-B | |

Isolation Procedure. Fractions collected during the final pre-concentration step were filtered through a stainless steel sieve and concentrated *in vacuo* using a Buchi^{®} Rotary Evaporator, Model R-114. The concentrated solution was dried for 48 - 72 h using the Kinetics Flexi-Dry Personal Freeze Dryer.

MS and MS/MS. MS and MS/MS data were generated with a Waters QTof Micro mass spectrometer equipped with an electrospray ionization source. The sample was analyzed by negative ESI. The sample (0.1 mg) was diluted with 50:50 ACN:H₂O to a concentration of 25 µg/mL for HRMS and MS/MS. Both samples were introduced *via* direct infusion.

NMR. An attempt was made to dissolve 2.6 mg of the sample in 150 µL CD₃OD, however the sample did not dissolve readily. To this solution, another 50 µL CD₃OD was added and the resulting solution was still cloudy. Addition of another 50 µL CD₃OD turned it to faint cloudy. The sample dissolved completely in ~10 min and was utilized for NMR analysis. All NMR data were acquired on Bruker Avance 500 MHz instruments with either a 2.5 mm inverse probe or a 5 mm broad band probe. The ¹H and ¹³C NMR spectra were referenced to the solvent resonance at δ_{H} 3.30 ppm and δ_{C} 49.0 ppm, respectively.

### Results and Discussion

Unless otherwise noted, all solvent ratios are listed as percent by volume (v/v).

Primary Purification of **CC-00336.** Approximately 300 g of *Stevia* extract was processed using the primary preparative HPLC method described above. Collected fractions were analyzed by LC-MS using the analytical method summarized above.

Secondary Purification of **CC-00336.** Material collected from primary purification was processed with conditions summarized above. Fractions were analyzed using the analytical method summarized above.

Tertiary Purification of **CC-00336.** Material collected from secondary purification was processed with conditions summarized above. Fractions were analyzed using the analytical method summarized above.

Final Batch Preparation of **CC-00336.** The purified material was concentrated by rotary evaporation. The concentrated solution was further dried *via* lyophilization for 48 hours. The final yield of the batch was 6.2 mg. The retention time of **CC-00338** was 28.644 min for CAD and 28.588 for UV according under the conditions described above.

Mass Spectrometry. The ESI-TOF mass spectrum acquired by infusing a sample of **CC-00336** showed a [M-H]⁻ ion at *m*/*z* 1273.5537. The mass of the [M-H]⁻ ion was in good agreement with the molecular formula C₅₆H₉₀O₃₂ (calcd for C₅₆H₈₉O₃₂: 1273.5337, error: -3.1 ppm) expected. The MS data confirmed that **CC-00336** has a nominal mass of 1274 Daltons with the molecular formula, C₅₀H₉₀O₃₂. The ion observed at *m*/*z* 1387.5516 was due to [M +TFA-H]⁻.

The MS/MS spectrum of **CC-00336,** selecting the [M-H]⁻ ion at *m*/*z* 1273.0 for fragmentation, indicated loss of three glucose units at *m*/*z* 1111.5330, 949.4388 and 787.3802 followed by the loss of rhamnose unit at *m*/*z* 641.3223 and sequential loss of two glucose units at *m*/*z* 479.2623 and 317.2216. Another fragmentation pathway is evident from the ion at *m*/*z* 625.3406 which would correspond to loss of one glucose unit from the ion at m/z 787.3802 and subsequent loss of one rhamnose unit and one glucose unit would also give the ions at *m*/*z* 479.2623 and 317.2216, respectively. The fragment ions at *m*/*z* 1111.5330 and 949.4388 are relatively weaker than the other fragment ions. The data thus indicated the presence of five glucose units and one rhamnose unit in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at 300K over a range of mixing times (40-140 msec)) were acquired to allow assignment of **CC-00336.**

The 1D and 2D NMR data indicated that the central core of the glycoside is a diterpene. An HMBC correlation from the methyl protons at δ_{H} 1.23 to the carbonyl at δ_{C} 177.7 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.5, 45.2, and 58.6 allowed assignment of C-3, C-4, and C-5. Analysis of the ¹H-¹³C HSQC-DEPT data indicated that the carbon at δ_{C} 38.5 was a methylene and the carbon at δ_{C} 58.6 was a methine which were assigned as C-3 and C-5, respectively. This left the carbon at δ_{C} 45.2, which did not show a correlation in the HSQC-DEPT spectrum, to be assigned as the quaternary carbon, C-4. The ¹H chemical shifts for C-3 (δ_{H} 1.01 and 2.38) and C-5 (δ_{H} 1.07) were assigned using the HSQC-DEPT data. COSY correlations between the H-3 protons (δ_{H} 1.01 and 2.38) and protons at δ_{H} 1.42 and 1.98 allowed assignment of H-2 protons which in turn showed correlations with the protons at δ_{H} 0.84 and 1.87 which were assigned to H-1. The ¹³C chemical shifts for C-1 and C-2 were then assigned on the basis of HSQC-DEPT correlations.

The other tertiary methyl singlet, observed at δ_{H} 0.94 (δ_{C} 17.1 *via* HSQC-DEPT), showed HMBC correlations to C-1 and C-5 and was assigned as H-20. The methyl protons showed additional HMBC correlations to a quaternary carbon (δ_{C} 40.6) and a methine carbon (δ_{C} 55.2) which were assigned as C-10 and C-9, respectively. H-9 (δ_{H} 0.98) was then determined based on HSQC-DEPT data. COSY correlations between H-5 (δ_{H} 1.07) and the protons at δ_{H} 1.86 and 1.90 then allowed assignment of the H-6 protons which in turn showed correlations to protons at δ_{H} 1.43 and 1.57 which were assigned to H-7. The ¹³C chemical shifts for C-6 (δ_{C} 23.0) and C-7 (δ_{C} 42.7) were then determined from the HSQC-DEPT data. COSY correlations between H-9 (δ_{H} 0.98) and proton at δ_{H} 1.65 allowed assignment of one of the H-11 protons. The ¹H chemical shift for remaining proton at C-11 (δ_{H} 1.79) was assigned using the HSQC-DEPT data. COSY correlations from H-11 protons (δ_{H} 1.65 and 1.79) to the protons at δ_{H} 1.58 and 1.94 allowed assignment of H-12 protons. The HSQC-DEPT data was then used to assign C-11 (δ_{C} 21.3) and C-12 (δ_{C} 39.0). The olefinic protons observed at δ_{H} -4.84 (obscured by water resonance) and 5.14 showed HMBC correlations to a carbon at δ_{C} 88.1 (C-13) and were assigned to H-17 (δ_{C} 105.6 *via* HSQC-DEPT). In HMBC spectrum, the protons at H-11 and one of the protons at H-12 (δ_{H} 1.94) also showed correlations to δ_{C} 88.1 (C-13). The methine proton H-9 and one of the methylene protons at H-11 (δ_{H} 1.79) showed HMBC correlations to the carbon at δ_{C} 43.2 which was assigned as C-8. HMBC correlation from δ_{H} 1.65 to C-13 (δ_{C} 88.1) allowed assignment of one of the methylene protons at C-14. The remaining proton at H-14 was deduced at δ_{H} 2.16 based on HSQC-DEPT data. The HSQC-DEPT data was also used to assign C-14 at δ_{C} 44.4. An HMBC correlation from one of the H-17 protons at δ_{H} -4.84 to a carbon at δ_{C} -49.2 (obscured by CD₃OD resonance) allowed assignment of C-15. The ¹H chemical shifts at C-15 (δ_{H} 2.03 and 2.17) were then assigned using the HSQC-DEPT data. HMBC correlations from H-12 (δ_{H} 1.94), H-14 (δ_{H} 2.16), and H-17 (δ_{H} ~4.84 and 5.14) to a quaternary carbon at δ_{C} 152.2 allowed assignment of C-16 to complete the assignment of the central core.

Correlations observed in the ROESY spectrum were used to assign the relative stereochemistry of the central diterpene core. In the ROESY spectrum, NOE correlations were observed between H-14 (δ_{H} 2.16) and H-20 indicating that H-14 and H-20 are on the same face of the rings. Similarly, NOE correlations were observed between H-18 and H-5 as well as H-5 and H-9 but NOE correlations were not observed between H-9 and H-20/H-14 (δ_{H} 2.16) indicating that H-5, H-9 and H-18 were on the opposite face of the rings compared to H-14 and H-20.

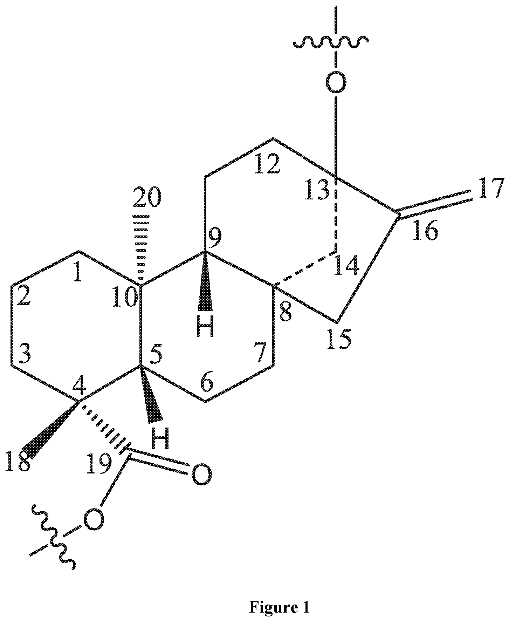

A summary of the ¹H and ¹³C chemical shifts for the aglycone are found in Table 8 and the structure of the aglycone core is provided in Figure 1.

**Table 8. ¹H and ¹³C NMR (500 and 125 MHz, CD₃OD) assignments of the aglycone.**

| | **CC-00336** | |
|---|---|---|
| **Position** | **¹³C** | **¹H** |
| 1 | 41.7 | 0.84 m |
| | | 1.87 m |
| 2 | 20.5 | 1.42 m |
| | | 1.98 m |
| 3 | 38.5 | 1.01 m |
| | | 2.38 brd (12.5) |
| 4 | 45.2 | --- |
| 5 | 58.6 | 1.07 m |
| 6 | 23.0 | 1.86 m |
| | | 1.90 m |
| 7 | 42.7 | 1.43 m |
| | | 1.57 m |
| 8 | 43.2 | --- |
| 9 | 55.2 | 0.98 m |
| 10 | 40.6 | --- |
| 11 | 21.3 | 1.65 m |
| | | 1.79 m |
| 12 | 39.0 | 1.58 m |
| | | 1.94 m |
| 13 | 88.1 | --- |
| 14 | 44.4 | 1.65 m |
| | | 2.16 m |
| 15 | ~49. 2^{†} | 2.03 brd (16.8) |
| | | 2.17 m |
| 16 | 154. 2 | --- |
| 17 | 105. 6 | ~4.84^{€} |
| | | 5.14 brs |
| 18 | 29.4 | 1.23 s |
| 19 | 177. 7 | --- |
| 20 | 17.1 | 0.94 s |

| | | |
|---|---|---|
| ^{†}Resonance obscured by CD₃OD, assignment based on HSQC-DEPT data. ^{€}Resonance obscured by H₂O, assignment based on HSQC-DEPT data. | | |

Analysis of the ¹H-¹³C HSQC-DEPT data for **CC-00336** confirmed the presence of six anomeric protons which were well resolved at δ_{H} 5.56 (δ_{C} 94.1), 5.42 (δ_{C} 102.1), 4.96 (δ_{C} 103.3), 4.70 (δ_{C} 104.2), 4.65 (δ_{C} 98.2), and 4.53 (δ_{C} 104.2). Five of the anomeric protons had large couplings (7.7 - 7.9 Hz) indicating that they had β-configurations. One of the anomeric protons at δ_{H} 5.42 observed as a broad singlet in ¹H NMR spectrum resolved to a broad doublet in the 1D-TOCSY spectrum had a small coupling of ~1.6 Hz indicating that this had α-configuration. The anomeric proton observed at δ_{H} 5.56 showed an HMBC correlation to C-19 which indicated that it corresponded to the anomeric proton of Glc_{I}. Similarly, the anomeric proton observed at δ_{H} 4.65 showed an HMBC correlation to C-13 allowing it to be assigned as the anomeric proton of Glc_{II}.

The ¹H and ¹³C chemical shifts for the C-19 glycoside are provided in Table 9. The ¹H and ¹³C chemical shifts for the C-13 glycoside are provided in Table 10.

**Table 9. ¹H and ¹³C NMR (500 and 125 MHz, CD₃OD), assignments of the C-19 glycoside.**

| | **CC-00336** | |
|---|---|---|
| **Position** | **¹³C** | **¹H** |
| Glc_{I}-1 | 94.1 | 5.56 d (7.8) |
| Glc_{I}-2 | 77.4 | 4.00 m |
| Glc_{I}-3 | 87.7 | 3.88 m |
| Glc_{I}-4 | 69.7 | 3.53 m |
| Glc_{I}-5 | 78.6 | 3.44 m |
| Glc_{I}-6 | 62.4 | 3.70 m, 3.86 m |
| Glc_{V}-1 | 103.3 | 4.96 d (7.8) |
| Glc_{V}-2 | 75.8 | 3.14 m |
| Glc_{V}-3 | 78.0-78.3^{§} | 3.36 m |
| Glc_{V}-4 | 72.7 | 3.18 m |
| Glc_{V}-5 | 78.0-78.3^{§} | 3.32 m |
| Glc_{V}-6 | 63.6 | 3.66 m, 3.89 m |
| Glcv_{VI}-1 | 104.2 | 4.70 d (7.8) |
| Glc_{VI}-2 | 75.2 or 75.4 | 3.28 m |
| Glc_{VI}-3 | 78.0-78.3^{§} | 3.39 m |
| Glc_{VI}-4 | 71.6 | 3.30 m |
| Glc_{VI}-5 | 78.0-78.3^{§} | 3.39 m |
| Glc_{VI}-6 | 62.6 | 3.63 m, 3.90 m |

| | | |
|---|---|---|
| ^{§}Six carbon resonances in the range of 78.0-78.3 (78.04, 78.16, 78.20, and 78.27; two carbons are overlapped each at 78.20 and 78.27), hence chemical shifts could not be unequivocally assigned. | | |

**Table 10. ¹H and ¹³C NMR (500 and 125 MHz, CD₃OD) assignments of the C-13 glycoside.**

| | **CC-00336** | |
|---|---|---|
| **Position** | **¹³C** | **¹H** |
| Glc_{II}-1 | 98.2 | 4.65 d (7.9) |
| Glc_{II}-2 | 77.5 | 3.57 m |
| Glc_{II}-3 | 88.6 | 4.80 m |
| Glc_{II}-4 | 70.2 | 3.44 m |
| Glc_{II}-5 | 77.2 | 3.25 m |
| Glc_{II}-6 | 62.7 | 3.63 m, 3.79 m |
| Rha-1 | 102.1 | 5.42 brs |
| Rha-2 | 72.0 | 3.99 m |
| Rha-3 | 72.1 | 3.68 m |
| Rha-4 | 73.9 | 3.38 m |
| Rha-5 | 70.0 | 4.09 m |
| Rha-6 | 18.2 | 1.21 d (6.1) |
| Glc_{IV}-1 | 104.2 | 4.53 d (7.7) |
| Glc_{IV}-2 | 75.2 or 75.4 | 3.25 m |
| Glc_{IV}-3 | 78.0-78.3^{§} | 3.39 m |
| Glc_{IV}-4 | 71.6 | 3.27 m |
| Glc_{IV}-5 | 78.0-78.3^{§} | 3.39 m |
| Glc_{IV}-6 | 62.6 | 3.62 m, 3.90 m |

| | | |
|---|---|---|
| ^{§}Six carbon resonances in the range of 78.0-78.3 (78.04, 78.16, 78.20, and 78.27; two carbons are overlapped each at 78.20 and 78.27), hence chemical shifts could not be unequivocally assigned. | | |

The structure of **CC-00336** was determined to be (13-[(2-O-α-L-rhamnopyranosyl-3-O-β-D-glucopyranosyl)- β-D-glucopyranosyl)oxy] ent-kaur-16-en-19-oic acid-[(2-O- β-D-glucopyranosyl-3-O- β-D-glucopyranosyl)- β-D-glucopyranosyl) ester]. **CC-00336** has the relatively uncommon 1→2 α-glycoside linkage between Rha and GlcII. **CC-00336** is an isomer of Rebaudioside N, thus named as Rebaudioside N2 (Reb N2).

### EXAMPLE 2: Isolation and Characterization of CC-00338 (Reference)

### The sample used for isolation of CC-00338 was Stevia extract.

HPLC and Isolation. Sample analyses were performed using the analytical HPLC method conditions described in Example 1. Preparative HPLC purification and isolation procedure was also carried out using the method conditions described in Example 1.

MS and MS/MS. MS and MS/MS data were generated with a Waters QTof Micro mass spectrometer equipped with an electrospray ionization source. The sample was analyzed by negative ESI. The sample (0.1 mg) was diluted with 50:50 ACN:H₂O to a concentration of 25 µg/mL for HRMS and MS/MS. Both samples were introduced via direct infusion.

NMR. An attempt was made to dissolve 2.3 mg of the sample in 150 µL CD₃OD, however the sample did not dissolve readily. To this solution, another 50 µL CD₃OD was added and the resulting solution was still cloudy. Addition of another 50 µL CD₃OD turned it to faint cloudy. The sample dissolved completely in ~10 min and was utilized for NMR analysis. All NMR data were acquired on Bruker Avance 500 MHz instruments with either a 2.5 mm inverse probe or a 5 mm broad band probe. The ¹H and ¹³C NMR spectra were referenced to the solvent resonance at δH 3.30 ppm and δC 49.0 ppm, respectively.

### Results and Discussion

Unless otherwise noted, all solvent ratios are listed as percent by volume (v/v).

Primary Purification of **CC-00338.** Approximately 300 g of Stevia extract was processed using the primary preparative HPLC method described above. Collected fractions were analyzed by LC-MS using the analytical method summarized above.

Secondary Purification of **CC-00338.** Material collected from primary purification was processed with conditions summarized above. Fractions were analyzed using the analytical method summarized above.

Tertiary Purification of **CC-00338.** Material collected from secondary purification was processed with conditions summarized above.

Final Batch Preparation of **CC-00338.** Fractions collected in tertiary purification were concentrated by rotary evaporation. The concentrated solution was further dried *via* lyophilization for 48 hours. The final yield of the batch was 4.1 mg. The retention time of **CC-00338** was 26.298 min for CAD and 26.239 for UV according under the conditions described above.

Mass Spectrometry. The ESI-TOF mass spectrum acquired by infusing a sample of **CC-00338** showed a [M-H]⁻ ion at *m*/*z* 1435.6161. The mass of the [M-H]⁻ ion was in good agreement with the molecular formula C₆₂H₁₀₀O₃₇ (calcd for C₆₂H₉₉O₃₇: 1435.5865, error: 2.6 ppm) expected. The MS data confirmed that **CC-00338** has a nominal mass of 1436 Daltons with the molecular formula, C₆₂H₁₀₀O₃₇. The ions observed at *m*/*z* 1533.5935 and 1549.6273 were due to [M-H+H₃PO₄]⁻ and [M-H+TFA]⁻, respectively.

The MS/MS spectrum of **CC-00338,** selecting the [M-H]⁻ ion at *m*/*z* 1435.0 for fragmentation, indicated sequential loss of five glucose units at *m*/*z* 1273.5712, 1111.4523, 949.4687, 787.3939 and 625.3284 and loss of one rhamnose unit at *m*/*z* 479.2623 followed by loss of one glucose unit at *m*/*z* 317.2216 as a major fragmentation ions. Alternative fragmentation patterns for glucose and rhamnose units are also observed in the spectrum. The fragment ions at *m*/*z* 1273.5712 and 1111.4523 are relatively weaker than the other fragment ions. The data thus indicated the presence of six glucose units and one rhamnose unit in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at 300K over a range of mixing times (40-140 msec)) were acquired to allow assignment of **CC-00338.**

The 1D and 2D NMR data indicated that the central core of the glycoside is a diterpene. An HMBC correlation from the methyl protons at δ_{H} 1.24 to the carbonyl at δ_{C} 177.1 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.2, 45.1, and 58.7 allowed assignment of C-3, C-4, and C-5. Analysis of the ¹H-¹³C HSQC-DEPT data indicated that the carbon at δ_{C} 38.2 was a methylene and the carbon at δ_{C} 58.7 was a methine which were assigned as C-3 and C-5, respectively. This left the carbon at δ_{C} 45.1, which did not show a correlation in the HSQC-DEPT spectrum, to be assigned as the quaternary carbon, C-4. The ¹H chemical shifts for C-3 (δ_{H} 1.01 and 2.40) and C-5 (δ_{H} 1.06) were assigned using the HSQC-DEPT data. COSY correlations between the H-3 protons (δ_{H} 1.01 and 2.40) and protons at δ_{H} 1.41 and 1.96 allowed assignment of H-2 protons which in turn showed correlations with the protons at δ_{H} 0.82 and 1.86 which were assigned to H-1. The ¹³C chemical shifts for C-1 and C-2 were then assigned on the basis of HSQC-DEPT correlations.

The other tertiary methyl singlet, observed at δ_{H} 0.95 (δ_{C} 17.3 *via* HSQC-DEPT), showed HMBC correlations to C-1 and C-5 and was assigned as H-20. The methyl protons showed additional HMBC correlations to a quaternary carbon (δ_{C} 40.6) and a methine carbon (δ_{C} 55.1) which were assigned as C-10 and C-9, respectively. H-9 (δ_{H} 0.99) was then determined based on HSQC-DEPT data. COSY correlations between H-5 (δ_{H} 1.06) and the protons at δ_{H} 1.87 and 1.90 then allowed assignment of the H-6 protons which in turn showed correlations to protons at δ_{H} 1.43 and 1.57 which were assigned to H-7. The ¹³C chemical shifts for C-6 (δ_{C} 23.0) and C-7 (δ_{C} 42.7) were then determined from the HSQC-DEPT data. COSY correlations between H-9 (δ_{H} 0.99) and proton at δ_{H} 1.66 allowed assignment of one of the H-11 protons. The ¹H chemical shift for remaining proton at C-11 (δ_{H} 1.78) was assigned using the HSQC-DEPT data. COSY correlations from H-11 protons (δ_{H} 1.66 and 1.78) to the protons at δ_{H} 1.58 and 1.90 allowed assignment of H-12 protons. The HSQC-DEPT data was then used to assign C-11 (δ_{C} 21.4) and C-12 (δ_{C} 39.7). The methine proton H-9 and one of the methylene protons at H-11 (δ_{H} 1.78) showed HMBC correlations to the carbon at δ_{C} 43.0 which was assigned as C-8. In HMBC spectrum, one of the protons at H-11 (δ_{H} 1.78) and H-12 (δ_{H} 1.90) showed correlations to a quaternary carbon at δ_{C} 88.7 and was assigned for C-13. One of the olefinic protons observed at δ_{H} 4.89 also showed HMBC correlations to a carbon at δ_{C} 88.7 (C-13) and was assigned to H-17 (δ_{C} 106.3 *via* HSQC-DEPT). The other olefinic proton at C-17 was assigned to be δ_{H} 5.23 based on HSQC-DEPT data. Although weak, an HMBC correlation from δ_{H} 1.62 to C-13 (δ_{C} 88.7) allowed assignment of one of the methylene protons at C-14. The remaining proton at H-14 was deduced at δ_{H} 2.17 based on HSQC-DEPT data. The HSQC-DEPT data was also used to assign C-14 at δ_{C} 44.3. Although weak, the HMBC correlations from H-17 protons at δ_{H} 4.89 and 5.23 to a carbon at δ_{C} -49.3 (obscured by CD₃OD resonance) allowed assignment ofC-15. The ¹H chemical shifts at C-15 (δ_{H} 2.03 and 2.17) were then assigned using the HSQC-DEPT data. A weak HMBC correlation from H-12 (δ_{H} 1.90) to a quaternary carbon at δ_{C} 153.5 allowed assignment of C-16 to complete the assignment of the central core.

Correlations observed in the ROESY spectrum were used to assign the relative stereochemistry of the central diterpene core. In the ROESY spectrum, NOE correlations were observed between H-14 (δ_{H} 2.17) and H-20 indicating that H-14 and H-20 are on the same face of the rings. Although weak, NOE correlations were also observed between H-18 and H-5 indicating that H-5 and H-18 are on the same face of the rings. The expected NOE correlations between H-5 (δ_{H} 1.06) and H-9 (δ_{H} 0.99) could not be observed due to their very close chemical shifts. However, since the proton and carbon chemical shifts for C-9 consistent with the proton and carbon chemical shifts for C-9 of previously reported compounds from *Stevia,* the relative stereochemistry of C-9 is considered to be the same as for previously reported *Stevia* compounds. NOE correlations were not observed between H-5/H-18 and H-20/H-14 (δ_{H} 2.17) indicating that H-5 and H-18 were on the opposite face of the rings compared to H-14 and H-20.

A summary of the ¹H and ¹³C chemical shifts for the aglycone are found in Table 1 and the structure of the aglycone core is provided in Figure 1.

**Table 1. ¹H and ¹³C NMR (500 and 125 MHz, CD₃OD) assignments of the aglycone.**

| | **CC-00338** | |
|---|---|---|
| **Position** | **¹³C** | **¹H** |
| 1 | 41.8 | 0.82 m |
| | | 1.86 m |
| 2 | 20.5 | 1.41 m |
| | | 1.96 m |
| 3 | 38.2 | 1.01 m |
| | | 2.40 brd (13.0) |
| 4 | 45.1 | --- |
| 5 | 58.7 | 1.06 m |
| 6 | 23.0 | 1.87 m |
| | | 1.90 m |
| 7 | 42.7 | 1.43 m |
| | | 1.57 m |
| 8 | 43.0 | --- |
| 9 | 55.1 | 0.99 m |
| 10 | 40.6 | --- |
| 11 | 21.4 | 1.66 m |
| | | 1.78 m |
| 12 | 39.7 | 1.58 m |
| | | 1.90 m |
| 13 | 88.7 | --- |
| 14 | 44.3 | 1.62 m |
| | | 2.17 m |
| 15 | ~49.3^{†} | 2.03 brd (16.0) |
| | | 2.17 m |
| 16 | 153.5 | --- |
| 17 | 106.3 | 4.89 brs |
| | | 5.23 brs |
| 18 | 29.6 | 1.24 s |
| 19 | 177.1 | --- |
| 20 | 17.3 | 0.95 s |

| | | |
|---|---|---|
| ^{†}Resonance obscured by CD₃OD, assignment based on HSQC-DEPT data. | | |

Analysis of the ¹H-¹³C HSQC-DEPT data for CC-00338 confirmed the presence of seven anomeric protons which were well resolved at δ_{H} 5.74 (δ_{C} 94.0), 5.44 (δ_{C} 101.9), 4.94 (δ_{C} 103.2), 4.79 (δ_{C} 105.5), 4.70 (δ_{C} 104.3), 4.58 (δ_{C} 98.1), and 4.49 (δ_{C} 104.4). Six of the anomeric protons had large couplings (7.4 - 7.9 Hz) indicating that they had β-configurations. One of the anomeric protons at δ_{H} 5.44 observed as a broad singlet in ¹H NMR spectrum resolved to a broad doublet in the 1D-TOCSY spectrum had a small coupling of ~1.6 Hz indicating that this had α-configuration. The anomeric proton observed at δ_{H} 5.74 showed an HMBC correlation to C-19 which indicated that it corresponded to the anomeric proton of Glc_{I}. Similarly, the anomeric proton observed at δ_{H} 4.58 showed an HMBC correlation to C-13 allowing it to be assigned as the anomeric proton of Glc_{II}.

The ¹H and ¹³C chemical shifts for the C-19 glycoside are provided in Table 2. The ¹H and ¹³C chemical shifts for the C-13 glycoside are provided in Table 3.

**Table 2. ¹H and ¹³C NMR (500 and 125 MHz, CD₃OD) assignments of the C-19 glycoside.**

| | **CC-00338** | |
|---|---|---|
| **Position** | **¹³C** | **¹H** |
| Glc_{I}-1 | 94.0 | 5.74 d (7.4) |
| Glc_{I}-2 | 77.7 | 3.97 m |
| Glc_{I}-3 | 87.8 | 3.96 m |
| Glc_{I}-4 | 69.9 | 3.48 m |
| Glc_{I}-5 | 78.1-78.5^{§} | 3.53 m |
| Glc_{I}-6 | 62. 5-62.7* | 3.68 m, 3.88 m |
| Glc_{V}-1 | 103.2 | 4.94 d (7.9) |
| Glc_{V}-2 | 75.7 or 75.8 | 3.12 m |
| Glc_{V}-3 | 78.1-78.5^{§} | 3.35 m |
| Glc_{V}-4 | 72.7 | 3.15 m |
| Glc_{V}-5 | 78.1-78.5^{§} | 3.31 m |
| Glc_{V}-6 | 63.6 | 3.65 m, 3.89 m |
| Glc_{VI}-1 | 104.3 | 4.70 d (7.9) |
| Glc_{VI}-2 | 75.2 or 75.4 | 3.28 m |
| Glc_{VI}-3 | 78.1-78.5^{§} | 3.38 m |
| Glc_{VI}-4 | 71.5 or 71.6 | 3.30 m |
| Glc_{VI}-5 | 78.1-78.5^{§} | 3.40 m |
| Glc_{VI}-6 | 62. 5-62.7^ | 3.64 m, 3.90 m |

| | | |
|---|---|---|
| ^{§}Seven carbon resonances in the range of 78.1-78.5 ppm (78.05, 78.10, 78.18, 78.20, 78.24, 78.32 and 78.52 ppm), hence chemical shifts could not be unequivocally assigned. ^{¶}Five carbon resonances in the range of 62.5-62.7 ppm (62.53, 62.58, 62.62, and 62.67; two carbons are overlapped at 62.58 ppm), hence chemical shifts could not be unequivocally assigned. | | |

**Table 3. ¹H and ¹³C NMR (500 and 125 MHz, CD₃OD) assignments of the C-13 glycoside.**

| | **CC-00338** | |
|---|---|---|
| **Position** | **¹³C** | **¹H** |
| Glc_{II}-1 | 98.1 | 4.58 d (7.7) |
| Glc_{II}-2 | 77.3 | 3.58 m |
| Glc_{II}-3 | 89.0 | 3.74 m |
| Glc_{II}-4 | 70.3 | 3.45 m |
| Glc_{II}-5 | 77.3 | 3.21 m |
| Glc_{II}-6 | 62.5-62.7^{¶} | 3.63 m, 3.78 m |
| Rha-1 | 101.9 | 5.44 brs |
| Rha-2 | 71.8 | 4.19 m |
| Rha-3 | 82.2 | 3.77 m |
| Rha-4 | 72.9 | 3.57 m |
| Rha-5 | 69.7 | 4.14 m |
| Rha-6 | 18.1 | 1.21 d (6.1) |
| Glc_{IV}-1 | 104.4 | 4.49 d (7.8) |
| Glc_{IV}-2 | 75.2 or 75.4 | 3.25 m |
| Glc_{IV}-3 | 78.1-78.5^{§} | 3.38 m |
| Glc_{IV}-4 | 71.5 or 71.6 | 3.27 m |
| Glc_{IV}-5 | 78.1-78.5^{§} | 3.37 m |
| Glc_{IV}-6 | 62.5-62.7^{¶} | 3.62 m, 3.90 m |
| Glc_{III}-1 | 105.5 | 4.79 d (7.9) |
| Glc_{III}-2 | 75.7 or 75.8 | 3.30 m |
| Glc_{III}-3 | 77.3 | 3.66 m |
| Glc_{III}-4 | 71.5 or 71.6 | 3.35 m |
| Glc_{III}-5 | 77.5 | 3.49 m |
| Glc_{III}-6 | 62.5-62.7^{¶} | 3.72m, 3.86 m |

| | | |
|---|---|---|
| ^{§}Seven carbon resonances in the range of 78.1-78.5 ppm (78.05, 78.10, 78.18, 78.20, 78.24, 78.32 and 78.52 ppm), hence chemical shifts could not be unequivocally assigned. ^{¶}Five carbon resonances in the range of 62.5-62.7 ppm (62.53, 62.58, 62.62, and 62.67; two carbons are overlapped at 62.58 ppm), hence chemical shifts could not be unequivocally assigned. | | |

The structure of **CC-00338** was determined to be (13-[(2-O-α-L-rhamnopyranosyl-(3-O-β-D-glucopyranosyl)-3-O- β-D-glucopyranosyl)- β-D-glucopyranosyl)oxy] ent-kaur-16-en-19-oic acid-[((2-O- β-D-glucopyranosyl-3-O- β-D-glucopyranosyl)- β-D-glucopyranosyl) ester].

### EXAMPLE 3: Isolation and Characterization of CC-00339 (Reference)

The sample used for isolation of **CC-00339** wass Stevia extract.

HPLC and Isolation. Sample analyses were performed using the analytical HPLC method conditions described in Example 1. Preparative HPLC purification and isolation procedure was also carried out using the method conditions described in Example 1.

MS and MS/MS. MS and MS/MS data were generated with a Waters QTof Micro mass spectrometer equipped with an electrospray ionization source. The sample was analyzed by negative ESI. The sample (0.1 mg) was diluted with 50:50 ACN:H₂O to a concentration of 25 µg/mL for HRMS and MS/MS. Both samples were introduced via direct infusion.

NMR. An attempt was made to dissolve 1.4 mg of the sample in 150 µL CD3OD, however the sample did not dissolve readily. To this solution, another 50 µL CD3OD was added and the resulting solution was still cloudy. Addition of another 50 µL CD3OD appears did not change the solubility of the sample. Hence CD₃OD soluble portion was utilized for NMR analysis. NMR data were acquired on a Bruker Avance 500 MHz instrument equipped with a 2.5 mm inverse probe. The ¹³C and HMBC data were acquired on a Bruker Avance 600 MHz instrument equipped with a 5 mm cryo-probe. The ¹H NMR spectrum was referenced to the CHD₂OD resonance at δ_{H} 3.30 ppm and ¹³C NMR spectrum was referenced to the CD₃OD resonance at δC 49.0 ppm.

### Results and Discussion

Unless otherwise noted, all solvent ratios are listed as percent by volume (v/v).

Primary Purification of **CC-00339.** Approximately 300 g of *Stevia* extract was processed using the primary preparative HPLC method described above. Collected fractions were analyzed by LC-MS using the analytical method summarized above.

Secondary Purification of **CC-00339.** The material collected from primary purification was processed with conditions summarized above. Fractions were analyzed using the analytical method summarized above.

Tertiary Purification of **CC-00339.** The material collected from secondary purification was processed with conditions summarized above.

Final Batch Preparation of **CC-00339.** The material collected from tertiary purification was concentrated by rotary evaporation. The concentrated solution was further dried *via* lyophilization for 48 hours. The final yield of the batch was 1.4 mg. The retention time of **CC-00339** was 28.216 min for CAD and 28.161 min for UV according under the conditions described above.

Mass Spectrometry. The ESI-TOF mass spectrum acquired by infusing a sample of **CC-00339** showed a [M-H]⁻ ion at *m*/*z* 1273.5712. The mass of the [M-H]⁻ ion was in good agreement with the molecular formula C₅₆H₉₀O₃₂ (calcd for C₅₆H₈₉O₃₂: 1273.5337, error: -1.6 ppm) expected.

The MS data confirmed that **CC-00339** has a nominal mass of 1274 Daltons with the molecular formula, C₅₆H₉₀O₃₂. The ions observed at *m*/*z* 1371.5204 and 1387.5516 were due to [M-H+H₃PO₄]⁻ and [M-H+TFA]⁻, respectively.

The MS/MS spectrum of **CC-00339,** selecting the [M-H]⁻ ion at *m*/*z* 1273.0.0 for fragmentation, indicated loss of two glucose units at *m*/*z* 1111.5007 and 949.4836 followed by loss of one rhamnose unit at *m*/*z* 803.3915 and sequential loss of three glucose units unit at *m*/*z* 641.3223, 479.2729 and 317.2216. The fragment ions at *m*/*z* 949.4836, 803.3915 and 317.2216 are relatively weaker than the other fragment ions. The data thus indicated the presence of five glucose units and one rhamnose unit in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at 300K over a range of mixing times (40-140 msec)) were acquired to allow assignment of **CC-00339.**

The 1D and 2D NMR data indicated that the central core of the glycoside is a diterpene. An HMBC correlation from the methyl protons at δ_{H} 1.27 to the carbonyl at δ_{C} 176.9 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.5, 45.1, and 59.4 allowed assignment of C-3, C-4, and C-5. Analysis of the ¹H-¹³C HSQC-DEPT data indicated that the carbon at δ_{C} 38.5 was a methylene and the carbon at δ_{C} 59.4 was a methine which were assigned as C-3 and C-5, respectively. This left the carbon at δ_{C} 45.1, which did not show a correlation in the HSQC-DEPT spectrum, to be assigned as the quaternary carbon, C-4. The ¹H chemical shifts for C-3 (δ_{H} 1.04 and 2.24) and C-5 (δ_{H} 1.07) were assigned using the HSQC-DEPT data. COSY correlations between the H-3 protons (δ_{H} 1.04 and 2.24) and protons at δ_{H} 1.41 and 1.97 allowed assignment of H-2 protons which in turn showed correlations with the protons at δ_{H} 0.84 and 1.88 which were assigned to H-1. The ¹³C chemical shifts for C-1 and C-2 were then assigned on the basis of HSQC-DEPT correlations.

The other tertiary methyl singlet, observed at δ_{H} 0.95 (δ_{C} 17.4 *via* HSQC-DEPT), showed HMBC correlations to C-1 and C-5 and was assigned as H-20. The methyl protons showed additional HMBC correlations to a quaternary carbon (δ_{C} 40.7) and a methine carbon (δ_{C} 55.1) which were assigned as C-10 and C-9, respectively. H-9 (δ_{H} 0.99) was then determined based on HSQC-DEPT data. COSY correlations between H-5 (δ_{H} 1.07) and the protons at δ_{H} 1.86 and 1.90 then allowed assignment of the H-6 protons which in turn showed correlations to protons at δ_{H} 1.44 and 1.56 which were assigned to H-7. The ¹³C chemical shifts for C-6 (δ_{C} 22.7) and C-7 (δ_{C} 42.7) were then determined from the HSQC-DEPT data. COSY correlations between H-9 (δ_{H} 0.99) and proton at δ_{H} 1.64 allowed assignment of one of the H-11 protons. The ¹H chemical shift for remaining proton at C-11 (δ_{H} 1.82) was assigned using the HSQC-DEPT data. COSY correlations from H-11 protons (δ_{H} 1.64 and 1.82) to the protons at δ_{H} 1.56 and 1.98 allowed assignment of H-12 protons. The HSQC-DEPT data was then used to assign C-11 (δ_{C} 21.4) and C-12 (δ_{C} 38.1). In HMBC spectrum, one of the methylene protons at H-11 (δ_{H} 1.82) and both of the methylene protons at H-12 showed correlations to a quaternary carbon at δ_{C} 87.9 and was assigned for C-13. The olefinic protons observed at δ_{H} ~4.84 (obscured by H₂O resonance) and 5.19 also showed HMBC correlations to a carbon at δ_{C} 87.9 (C-13) and was assigned to H-17 (δ_{C} 105.5 *via* HSQC-DEPT). The HMBC correlations from δ_{H} 1.51 and 2.26 to C-13 (δ_{C} 87.9) allowed assignment of the methylene protons at C-14. The HSQC-DEPT data was used to assign C-14 at δ_{C} 45.7. Similarly, HMBC correlations from H-9 and H-17 to a carbon at δ_{C} ~48.7 (obscured by CD₃OD resonance) allowed assignment of C-15. The ¹H chemical shifts at C-15 (δ_{H} 2.04 and 2.14) were then assigned using the HSQC-DEPT data. The methine proton H-9, the methylene protons H-14 and one of the methylene protons at H-15 (δ_{H} 2.14) showed HMBC correlations to the carbon at δ_{C} 43.3 which was assigned as C-8. The HMBC correlations from H-12 (δ_{H} 1.98), H-14 (δ_{H} 2.26), H-15 and H-17 to a quaternary carbon at δ_{C} 154.3 allowed assignment of C-16 to complete the assignment of the central core.

Correlations observed in the ROESY spectrum were used to assign the relative stereochemistry of the central diterpene core. In the ROESY spectrum, NOE correlations were observed between H-14 (δ_{H} 2.26) and H-20 indicating that H-14 and H-20 are on the same face of the rings. Although weak, NOE correlations were also observed between H-18 and H-5 indicating that H-5 and H-18 are on the same face of the rings. The expected NOE correlations between H-5 (δ_{H} 1.07) and H-9 (δ_{H} 0.99) could not be observed due to their very close chemical shifts. However, since the proton and carbon chemical shifts for C-9 of **CC-00339** are consistent with the proton and carbon chemical shifts for C-9 of previously reported compounds from *Stevia,* the relative stereochemistry of C-9 is considered to be the same as for previously reported *Stevia* compounds. NOE correlations were not observed between H-5/H-18 and H-20/H-14 (δ_{H} 2.26) indicating that H-5 and H-18 were on the opposite face of the rings compared to H-14 and H-20.

A summary of the ¹H and ¹³C chemical shifts for the aglycone are found in Table 1 and the structure of the aglycone core is provided in Figure 1.

**Table 1. ¹H and ¹³C NMR (500 and 150 MHz, CD₃OD) assignments of the aglycone.**

| | **CC-00339** | |
|---|---|---|
| **Position** | **¹³C** | **¹H** |
| 1 | 41.8 | 0.84 m |
| | | 1.88 m |
| 2 | 20.4 | 1.41 m |
| | | 1.97 m |
| 3 | 38.5 | 1.04 m |
| | | 2.24 m |
| 4 | 45.1 | --- |
| 5 | 59.4 | 1.07 m |
| 6 | 22.7 | 1.86 m |
| | | 1.90 m |
| 7 | 42.7 | 1.44 m |
| | | 1.56 m |
| 8 | 43.3 | --- |
| 9 | 55.1 | 0.99 brd (8.0) |
| 10 | 40.7 | --- |
| 11 | 21.4 | 1.64 m |
| | | 1.82 m |
| 12 | 38.1 | 1.56 m |
| | | 1.98 m |
| 13 | 87.9 | --- |
| 14 | 45.7 | 1.51 brd (11.7) |
| | | 2.26 m |
| 15 | ~48.7^{†} | 2.04 brd (17.2) |
| | | 2.14 brd (16.1) |
| 16 | 154.3 | --- |
| 17 | 105.5 | ~4.84^{¥} |
| | | 5.19 brs |
| 18 | 29.8 | 1.27 s |
| 19 | 176.9 | --- |
| 20 | 17.4 | 0.95 s |

| | | |
|---|---|---|
| ^{†}Resonance obscured by CD₃OD, assignment based on HSQC-DEPT data. ^{¥}Resonance obscured by H₂O, assignment based on HSQC-DEPT data. | | |

Analysis of the ¹H-¹³C HSQC-DEPT data for **CC-00339** confirmed the presence of six anomeric protons out of which four were well resolved at δ_{H} 5.65 (δ_{C} 94.4), 5.19 (δ_{C} 101.7), 4.63 (δ_{C} 97.6) and 4.50 (δ_{C} 104.9). The remaining two protons were partially overlapped at 4.60 (δ_{C} 105.1 or 105.2) and 4.58 (δ_{C} 105.1 or 105.2). Five of the anomeric protons had large couplings (7.3 - 9.1 Hz) indicating that they had β-configurations. One of the anomeric protons at δ_{H} 5.19 observed as a broad singlet in ¹H NMR spectrum resolved to a broad doublet in the 1D-TOCSY spectrum had a small coupling of ~2.6 Hz indicating that this had α-configuration. The anomeric proton observed at δ_{H} 5.65 showed an HMBC correlation to C-19 which indicated that it corresponded to the anomeric proton of Glc_{I}. Similarly, the anomeric proton observed at δ_{H} 4.63 showed an HMBC correlation to C-13 allowing it to be assigned as the anomeric proton of Glc_{II}.

The ¹H and ¹³C chemical shifts for the C-19 glycoside are provided in Table 2. The ¹H and ¹³C chemical shifts for the C-13 glycoside are provided in Table 3.

**Table 2. ¹H and ¹³C NMR (500 and 150 MHz, CD₃OD) assignments of the C-19 glycoside.**

| | **CC-00339** | |
|---|---|---|
| **Position** | **¹³C** | **¹H** |
| Glc_{I}-1 | 94.4 | 5.65 d (7.3) |
| Glc_{I}-2 | 79.4 | 3.81 m |
| Glc_{I}-3 | 87.0 | 3.77 m |
| Glc_{I}-4 | 69.5 | 3.58 m |
| Glc_{I}-5 | 77.4-78.1^{§} | 3.45 m |
| Glc_{I}-6 | 62.4-62.7^{¶} | 3.71 m, 3.84 m |
| Rha-1 | 101.7 | 5.19 brs |
| Rha-2 | 70.9 | 4.25 m |
| Rha-3 | 83.0 | 3.72 m |
| Rha-4 | 72.6 | 3.52 m |
| Rha-5 | 70.2 | 3.78 m |
| Rha-6 | 18.4 | 1.22 d (6.1) |
| Glc_{V}-1 | 105.1 or 105.2 | 4.58 d (9.1)^{€} |
| Glc_{V}-2 | 75.4 | 3.28 m |
| Glc_{V}-3 | 77.4-78.1^{§} | 3.41 m |
| Glc_{V}-4 | 71.3 | 3.33 m |
| Glc_{V}-5 | 77.4-78.1^{§} | 3.45 m |
| Glc_{V}-6 | 62.4-62.7^{¶} | 3.69m, 3.87 m |
| Glc_{VI}-1 | 104.9 | 4.50 d (8.0) |
| Glc_{VI}-2 | 75.1 | 3.27 m |
| Glc_{VI}-3 | 77.4-78.1^{§} | 3.41 m |
| Glc_{VI}-4 | 71.5 or 71.6 | 3.28 m |
| Glc_{VI}-5 | 77.4-78.1^{§} | 3.36 m |
| Glc_{VI}-6 | 62.4-62.7^{¶} | 3.64 m, 3.90 m |

| | | |
|---|---|---|
| ^{§}Seven carbon resonances in the range of 77.4-78.1 ppm (77.41, 77.57, 77.65, 77.71, 77.90, 78.00, and 78.14 ppm), hence chemical shifts could not be unequivocally assigned. 'Four carbon resonances in the range of 62.4-62.7 ppm (62.43, 62.61, and 62.71; two carbons are overlapped at 62.61 ppm), hence chemical shifts could not be unequivocally assigned. ^{€}Partially overlapped with Glc_{III} anomeric proton. | | |

**Table 3. ¹H and ¹³C NMR (500 and 150 MHz, CD₃OD) assignments of the C-13 glycoside.**

| | **CC-00339** | |
|---|---|---|
| **Position** | **¹³C** | **¹H** |
| Glc_{II}-1 | 97.6 | 4.63 d (7.7) |
| Glc_{II}-2 | 82.4 | 3.48 m |
| Glc_{II}-3 | 78.3 | 3.55 m |
| Glc_{II}-4 | 71.5 or 71.6 | 3.32 m |
| Glc_{II}-5 | 77.4-78.1^{§} | 3.21 m |
| Glc_{II}-6 | 62.4-62.7^{¶} | 3.64m, 3.80 m |
| Gl_{III}-1 | 105.1 or 105.2 | 4.60 d (8.1)^{€} |
| Glc_{III}-2 | 76.3 | 3.25 m |
| Glc_{III}-3 | 77.4-78.1^{§} | 3.35 m |
| Glc_{III}-4 | 71.8 | 3.27 m |
| Glc_{III}-5 | 78.4 | 3.26 m |
| Glc_{III}-6 | 63.1 | 3.64 m, 3.81 m |

| | | |
|---|---|---|
| ^{§}Seven carbon resonances in the range of 77.4-78.1 ppm (77.41, 77.57, 77.65, 77.71, 77.90, 78.00, and 78.14 ppm), hence chemical shifts could not be unequivocally assigned. ^{¶}Four carbon resonances in the range of 62.4-62.7 ppm (62.43, 62.61, and 62.71; two carbons are overlapped at 62.61 ppm), hence chemical shifts could not be unequivocally assigned. ^{€}Partially overlapped with Glcv anomeric proton. | | |

The structure of **CC-00339** was determined to be (13-[(2-O-β-D-glucopyranosyl)- β-D-glucopyranosyl)oxy] ent-kaur-16-en-19-oic acid-[((2-O-α-L-rhamnopyranosyl-(3-O- β-D-glucopyranosyl)-3-O- β-D-glucopyranosyl)- β-D-glucopyranosyl) ester].

### EXAMPLE 4: Isolation and Characterization of CC-00343 (Reference)

The material used for the isolation of **CC-00343** was Stevia extract.

HPLC and Isolation. Sample analyses were performed using the analytical HPLC method conditions described in Example 1. Preparative HPLC purification and isolation procedure was also carried out using the method conditions described in Example 1.

MS and MS/MS. MS and MS/MS data were generated with a Waters QTof Micro mass spectrometer equipped with an electrospray ionization source. The sample was analyzed by negative ESI. The sample (0.1 mg) was diluted with 50:50 ACN:H₂O to a concentration of 0.2 mg/mL for HRMS and MS/MS. Both samples were introduced via direct infusion.

NMR. An attempt was made to dissolve 2.4 mg of the sample in 150 µL CD₃OD, however the sample did not dissolve readily. To this solution, another 50 µL CD₃OD was added and the resulting solution was still cloudy. Addition of another 50 µL CD₃OD turned it to faint cloudy. The soluble portion of the sample was utilized for NMR analysis. NMR data were acquired on a Bruker Avance 500 MHz instrument equipped with a 2.5 mm inverse probe. The ¹³C and HMBC data were acquired using a Bruker Avance 600 MHz instrument equipped with a 5 mm cryo-probe. The ¹H NMR spectrum was referenced to the CHD₂OD resonance at δH 3.30 ppm and ¹³C NMR spectrum was referenced to the CD₃OD resonance at δC 49.0 ppm.

### Results and Discussion

Unless otherwise noted, all solvent ratios are listed as percent by volume (v/v).

Primary Purification of **CC-00343.** Approximately 300 g of *Stevia* extract was processed using the primary preparative HPLC method described above. Collected fractions were analyzed by LC-MS using the analytical method summarized above.

Secondary Purification of **CC-00343.** Material collected from primary purification was processed with conditions summarized above. Fractions were analyzed using the analytical method summarized above.

Tertiary Purification of **CC-00343.** Material collected from secondary purification was processed with conditions summarized above. Fractions were analyzed using the analytical method summarized above.

Final Batch Preparation of **CC-00343.** Material collected from tertiary purification was concentrated by rotary evaporation for final isolation. The concentrated solution was further dried *via* lyophilization for 48 hours. The final yield of the batch was 4.5 mg. The retention time of **CC-00343** was 24.944 min for CAD and 24.833 for UV according under the conditions described above.

Mass Spectrometry. The ESI-TOF mass spectrum acquired by infusing a sample of **CC-00343** showed a [M-H]⁻ ion at *m*/*z* 1435.5356 . The mass of the [M-H]⁻ ion was in good agreement with the molecular formula C₆₂H₁₀₀O₃₇ (calcd for C₆₂H₉₉O₃₇: 1435.5865, error: 2.6 ppm) expected for **CC-00343.** The MS data confirmed that **CC-00343** has a nominal mass of 1436 Daltons with the molecular formula, C₆₂H₁₀₀O₃₇.

The MS/MS spectrum of **CC-00343,** selecting the [M-H]⁻ ion at *m*/*z* 1435.0 for fragmentation, indicated sequential loss of three glucose units at *m*/*z* 1273.5117, 1111.4932 and 949.4018 and loss of one rhamnose unit at *m*/*z* 803.3438 followed by sequential loss of three glucose units at *m*/*z* 641.2920, 479.2464 and 317.2158 as a major fragmentation ions. The data thus indicated the presence of six glucose units and one rhamnose unit in the structure.

NMR Spectroscopy. A series of NMR experiments including ¹H NMR (500 MHz, CD₃OD at 300K and 292K), ¹³C NMR (125 MHz, CD₃OD at 292K), ¹H-¹H COSY (500 MHz, CD₃OD at 292K), HSQC-DEPT (500 MHz, CD₃OD at 292K), HMBC (500 MHz, CD₃OD at 292K), ROESY (500 MHz, CD₃OD at 292K), and 1D TOCSY (500 MHz, CD₃OD at 292K over a range of mixing times (40-140 msec)) were acquired to allow assignment of **CC-00343.**

In the ¹H NMR spectrum acquired at 300K, one of the anomeric proton was partially obscured by water resonance at 4.83 ppm . Therefore, VT experiments were performed to acquire ¹H NMR spectrum. In the ¹H NMR spectrum acquired at 292K, the anomeric proton was sufficiently resolved and thus additional NMR data were acquired at 292K. The 1D and 2D NMR data indicated that the central core of the glycoside is a diterpene. An HMBC correlation from the methyl protons at δ_{H} 1.26 to the carbonyl at δ_{C} 177.1 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.5, 45.1, and 59.3 allowed assignment of C-3, C-4, and C-5. Analysis of the ¹H-¹³C HSQC-DEPT data indicated that the carbon at δ_{C} 38.5 was a methylene and the carbon at δ_{C} 59.3 was a methine which were assigned as C-3 and C-5, respectively. This left the carbon at δ_{C} 45.1, which did not show a correlation in the HSQC-DEPT spectrum, to be assigned as the quaternary carbon, C-4. The ¹H chemical shifts for C-3 (δ_{H} 1.04 and 2.23) and C-5 (δ_{H} 1.08) were assigned using the HSQC-DEPT data. COSY correlations between the H-3 protons (δ_{H} 1.04 and 2.23) and protons at δ_{H} 1.41 and 1.96 allowed assignment of H-2 protons which in turn showed correlations with the protons at δ_{H} 0.84 and 1.87 which were assigned to H-1. The ¹³C chemical shifts for C-1 and C-2 were then assigned on the basis of HSQC-DEPT correlations.

The other tertiary methyl singlet, observed at δ_{H} 0.94 (δ_{C} 17.7 *via* HSQC-DEPT), showed HMBC correlations to C-1 and C-5 and was assigned as H-20. The methyl protons showed additional HMBC correlations to a quaternary carbon (δ_{C} 40.7) and a methine carbon (δ_{C} 55.0) which were assigned as C-10 and C-9, respectively. H-9 (δ_{H} 1.00) was then determined based on HSQC-DEPT data. COSY correlations between H-5 (δ_{H} 1.08) and the protons at δ_{H} 1.88 and 1.92 then allowed assignment of the H-6 protons which in turn showed correlations to protons at δ_{H} 1.44 and 1.56 which were assigned to H-7. The ¹³C chemical shifts for C-6 (δ_{C} 22.9) and C-7 (δ_{C} 42.8) were then determined from the HSQC-DEPT data. COSY correlations between H-9 (δ_{H} 1.00) and proton at δ_{H} 1.64 allowed assignment of one of the H-11 protons. The ¹H chemical shift for remaining proton at C-11 (δ_{H} 1.80) was assigned using the HSQC-DEPT data. COSY correlations from H-11 protons (δ_{H} 1.64 and 1.80) to a proton at δ_{H} 1.94 allowed assignment of one of the H-12 protons. The ¹H chemical shift for remaining proton at C-12 (δ_{H} 1.52) was assigned using the HSQC-DEPT data. The HSQC-DEPT data was then used to assign C-11 (δ_{C} 21.2) and C-12 (δ_{C} 39.8). The methine proton H-9 and methylene protons at H-11 showed HMBC correlations to the carbon at δ_{C} 42.9 which was assigned as C-8. In HMBC spectrum, methylene protons at H-12 showed correlations to a quaternary carbon at δ_{C} 88.7 and was assigned for C-13. The olefinic protons observed at δ_{H} 4.87 and 5.26 also showed HMBC correlations to a carbon at δ_{C} 88.7 (C-13) and was assigned to H-17 (δ_{C} 105.8 *via* HSQC-DEPT). Also, HMBC correlations from methylene protons δ_{H} 1.54 and 2.23 to C-13 (δ_{C} 88.7) allowed assignment of methylene protons at C-14. The HSQC-DEPT data was used to assign C-14 at δ_{C} 45.5. Similarly, HMBC correlations from H-8, H-14 and H-17 protons to a carbon at δ_{C} 48.6 allowed assignment of C-15. The ¹H chemical shifts at C-15 (δ_{H} 2.05 and 2.14) were then assigned using the HSQC-DEPT data. The HMBC correlations from H-12 (δ_{H} 1.94), H-15 and H-17 to a quaternary carbon at δ_{C} 153.4 allowed assignment of C-16 to complete the assignment of the central core.

Correlations observed in the ROESY spectrum were used to assign the relative stereochemistry of the central diterpene core. In the ROESY spectrum, NOE correlations were observed between H-14 (δ_{H} 2.23) and H-20 indicating that H-14 and H-20 are on the same face of the rings. Although weak, NOE correlations were also observed between H-18 and H-5 indicating that H-5 and H-18 are on the same face of the rings. The expected NOE correlations between H-5 (δ_{H} 1.08) and H-9 (δ_{H} 1.00) could not be observed due to their very close chemical shifts. However, since the proton and carbon chemical shifts for C-9 of **CC-00343** are consistent with the proton and carbon chemical shifts for C-9 of previously reported compounds from *Stevia,* the relative stereochemistry of C-9 is considered to be the same as for previously reported *Stevia* compounds. NOE correlations were not observed between H-5/H-18 and H-20/H-14 (δ_{H} 2.23) indicating that H-5 and H-18 were on the opposite face of the rings compared to H-14.

A summary of the ¹H and ¹³C chemical shifts for the aglycone are found in Table 1 and the structure of the aglycone core is provided in Figure 1.

**Table 1. ¹H and ¹³C NMR (500 and 150 MHz, CD₃OD) assignments of the aglycone.**

| | CC-00343 | |
|---|---|---|
| Position | ¹³C | ¹H |
| 1 | 41.8 | 0.84 m |
| | | 1.87 m |
| 2 | 20.4 | 1.41 m |
| | | 1.96 m |
| 3 | 38.5 | 1.04 m |
| | | 2.23 brd (11.8) |
| 4 | 45.1 | --- |
| 5 | 59.3 | 1.08 m |
| 6 | 22.9 | 1.88 m |
| | | 1.92 m |
| 7 | 42.8 | 1.44 m |
| | | 1.56 m |
| 8 | 42.9 | --- |
| 9 | 55.0 | 1.00 m |
| 10 | 40.7 | --- |
| 11 | 21.2 | 1.64 m |
| | | 1.80 m |
| 12 | 38.8 | 1.52 m |
| | | 1.94 m |
| 13 | 88.7 | --- |
| 14 | 45.5 | 1.54 m |
| | | 2.23 brd (11.8) |
| 15 | 48.6 | 2.05 brd (17.2) |
| | | 2.14 brd (17.1) |
| 16 | 153.4 | --- |
| 17 | 105.8 | 4.87^{†} |
| | | 5.26 brs |
| 18 | 29.7 | 1.26 s |
| 19 | 177.1 | --- |
| 20 | 17.7 | 0.94 s |

| | | |
|---|---|---|
| ^{†}Resonance overlapped with Glc_{III} H-1, assignment based on HSQC-DEPT data. | | |

Analysis of the ¹H-¹³C HSQC-DEPT data for **CC-00343** confirmed the presence of seven anomeric protons four of which were well resolved at δ_{H} 5.69 (δ_{C} 94.1), 5.29 (δ_{C} 101.6), 4.67 (δ_{C} 104.3) and 4.63 (δ_{C} 97.3). One of the anomeric protons was partially overlapped with H-17 (δ_{H} 4.87) was observed at δ_{H} 4.86 (δ_{C} 103.7). The remaining two anomeric protons were partially overlapped and observed at δ_{H} 4.55 (δ_{C} 105.6) and 4.53 (δ_{C} 104.4). Six of the anomeric protons had large couplings (7.2 - 8.1 Hz) indicating that they had β-configurations. One of the anomeric protons at δ_{H} 5.29 observed as a broad singlet in ¹H NMR spectrum resolved to a broad doublet in the 1D-TOCSY spectrum had a small coupling of ~1.4 Hz indicating that this had α-configuration. The anomeric proton observed at δ_{H} 5.69 showed an HMBC correlation to C-19 which indicated that it corresponded to the anomeric proton of Glc_{I}. Similarly, the anomeric proton observed at δ_{H} 4.63 showed an HMBC correlation to C-13 allowing it to be assigned as the anomeric proton of Glc_{II}.

The ¹H and ¹³C chemical shifts for the C-19 glycoside are provided in Table 2. The ¹H and ¹³C chemical shifts for the C-13 glycoside are provided in Table 3.

**Table 2. ¹H and ¹³C NMR (500 and 150 MHz, CD₃OD) assignments of the C-19 glycoside.**

| | **CC-00343** | |
|---|---|---|
| **Position** | **¹³C** | **¹H** |
| Glc_{I}-1 | 94.1 | 5.69 d (7.3) |
| Glc_{I}-2 | 77.9 | 3.81 m |
| Glc_{I}-3 | 87.5 | 3.81 m |
| Glc_{I}-4 | 69.6 | 3.61 m |
| Glc_{I}-5 | 77.6 or 77.7 | 3.48 m |
| Glc_{I}-6 | 62.5 or 62.6 or 62.7^{¶} | 3.71 m, 3.83 m |
| Rha-1 | 101.6 | 5.29 brs |
| Rha-2 | 71.9 | 3.99 m |
| Rha-3 | 72.1 | 3.83 m |
| Rha-4 | 83.2 | 3.59 m |
| Rha-5 | 69.1 | 3.81 m |
| Rha-6 | 18.5 | 1.31 d (6.2) |
| Glc_{V}-1 | 105.6 | 4.55 d (7.2) |
| Glc_{V}-2 | 76.0 | 3.16 m |
| Glc_{V}-3 | 78.0-78.3^{§} | 3.35 m |
| Glc_{V}-4 | 71.5 or 71.6^{¥} | ~3.28 m |
| Glc_{V}-5 | 78.0-78.3^{§} | 3.24 m |
| Glc_{V}-6 | 62.5 or 62.6 or 62.7^{¶} | 3.67 m, 3.84 m |
| Glc_{VI}-1 | 104.4 | 4.53 d (7.4) |
| Glc_{VI}-2 | 75.1 | 3.27 m |
| Glc_{VI}-3 | 78.0-78.3^{§} | 3.38 m |
| Glc_{VI}-4 | 71.5 or 71.6^{¥} | ~3.28 m |
| Glc_{VI}-5 | 78.0-78.3^{§} | 3.34 m |
| Glc_{VI}-6 | 62.5 or 62.6 or 62.7^{¶} | 3.62 m, 3.89 m |

| | | |
|---|---|---|
| ^{¶}Five carbon resonances in the range of 62.5-62.7 ppm (62.51, 62.62, 62.64, and 62.74; two carbons are overlapped at 62.74 ppm), hence chemical shifts could not be unequivocally assigned. ^{§}Seven carbon resonances in the range of 78.0-78.3 ppm (78.00, 78.06, 78.18, 78.25, and 78.31 ppm; two carbons overlapped each at 78.25 and 78.31), hence chemical shifts could not be unequivocally assigned. ^{¥}Three carbon resonances at 71.5 and 71.6 ppm (71.45, 71.52 and 71.57 ppm), hence chemical shifts could not be unequivocally assigned. | | |

**Table 3. ¹H and ¹³C NMR (500 and 150 MHz, CD₃OD) assignments of the C-13 glycoside.**

| | **CC-00343** | |
|---|---|---|
| **Position** | **¹³C** | **¹H** |
| Glc_{II}-1 | 97.3 | 4.63 d (7.8) |
| Glc_{II}-2 | 79.9 | 3.63 m |
| Glc_{II}-3 | 87.7 | 3.75 m |
| Glc_{II}-4 | 70.2 | 3.38 m |
| Glc_{II}-5 | 77.6 or 77.7 | 3.24 m |
| Glc_{II}-6 | 62.5 or 62.6 or 62.7^{¶} | 3.65 m, 3.82 m |
| Glc_{III}-1 | 103.7 | 4.86 d (8.1) |
| Glc_{III}-2 | 76.2 | 3.18 m |
| Glc_{III}-3 | 78.0-78.3^{§} | 3.33 m |
| Glc_{III}-4 | 72.5 | 3.11 m |
| Glc_{III}-5 | 78.5 | 3.28 m |
| Glc_{III}-6 | 63.6 | 3.56m, 3.82 m |
| Glc_{IV}-1 | 104.3 | 4.67 d (7.8) |
| Glc_{IV}-2 | 75.4 | 3.26 m |
| Glc_{IV}-3 | 78.0-78.3^{§} | 3.36 m |
| Glc_{IV}-4 | 71.5 or 71.6^{¥} | 3.28 m |
| Glc_{IV}-5 | 78.0-78.3^{§} | 3.36 m |
| Glc_{IV}-6 | 62.5 or 62.6 or 62.7^{¶} | 3.63 m, 3.89 m |

| | | |
|---|---|---|
| ^{¶}Five carbon resonances in the range of 62.5-62.7 ppm (62.51, 62.62, 62.64, and 62.74; two carbons are overlapped at 62.74 ppm), hence chemical shifts could not be unequivocally assigned. ^{§}Seven carbon resonances in the range of 78.0-78.3 ppm (78.00, 78.06, 78.18, 78.25, and 78.31 ppm; two carbons overlapped each at 78.25 and 78.31), hence chemical shifts could not be unequivocally assigned. ^{¥}Three carbon resonances at 71.5 and 71.6 ppm (71.45, 71.52 and 71.57 ppm), hence chemical shifts could not be unequivocally assigned. | | |

The structure of **CC-00343** was determined to be 13-[(2-*O*-β-D-glucopyranosyl-3-*O*- β-D-glucopyranosyl)- β-D-glucopyranosyl)oxy] *ent*-kaur-16-en-19-oic acid-[((2-*O*-α-L-rhamnopyranosyl-(4-*O*- β-D-glucopyranosyl)-3-*O*- β-D-glucopyranosyl)- β-D-glucopyranosyl) ester]. **CC-00343** has a relatively uncommon 1→4 α-glycoside linkage between Rha and GlcV. **CC-00343** is an isomer of Rebaudioside O and is thus named Rebaudioside O3 (Reb O3).

### EXAMPLE 5: Isolation and Characterization of CC-00350

The material used for the isolation of **CC-00350** was *Stevia* extract.

HPLC Analysis. HPLC analyses were performed on a Waters 2695 Alliance System coupled to a Waters 996 Photo Diode Array (PDA) detector. In addition, sample purities were assessed using an ESA Corona Charged Aerosol Detector (CAD). Sample analyses were performed using the method conditions described in Tables 1-4.

**Table 1: Analytical HPLC Conditions for Fraction Analysis in Primary Process**

| | | |
|---|---|---|
| **Column** | Phenomenex Synergi Hydro RP (4.6 × 250 mm, 4 µm) | |
| **Column Temperature (°C)** | 55 | |
| **Sample Temperature (°C)** | Ambient | |
| **Mobile Phases** | (A) 0.0284% ammonium acetate (NH₄OA_{c}) and 0.0116% acetic acid (HOAc) in water | |
| | (B) MeCN | |
| **Flow Rate (mL/min)** | 1.0 | |

| **Detection** | CAD and UV at 210 nm | |
|---|---|---|
| **Gradient** | | |
| Time (min) | %A | %B |
| 0.0 - 8.5 | 75 | 25 |
| 10.0 | 71 | 29 |
| 16.5 | 70 | 30 |
| 18. 5 - 24. 5 | 66 | 34 |
| 26.5 - 29.0 | 48 | 52 |
| 31.0 - 37.0 | 30 | 70 |
| 38.0 | 75 | 25 |

**Table 2: Analytical HPLC Conditions for Fraction Analysis in Secondary Process**

| | | |
|---|---|---|
| **Column (Dimensions, mm)** | XBridge Phenyl (4.6 × 150 mm, 3.5 µm) PN186003352, SN012634135129 | |
| **Column Temperature (°C)** | Ambient | |
| **Sample Temperature (°C)** | Ambient | |
| **Mobile Phases** | (A) Water | |
| | (B) MeCN | |
| **Detection** | CAD and UV at 210 nm | |
| **Flow Rate (mL/min)** | 1 mL/min | |

| **Gradient** | | |
|---|---|---|
| **Time (min)** | **% A** | **% B** |
| 0.0 - 20.0 | 80 | 20 |
| 20.1 - 25.0 | 50 | 50 |
| 25.1 | 80 | 20 |

**Table 3: Analytical HPLC Conditions for Fraction Analysis in Tertiary and Quaternary Process**

| | | |
|---|---|---|
| **Column (Dimensions, mm)** | Waters XBridge Amide (4.6 × 150 mm, 3.5 µm) PN186004869, SN 01253516613401 | |
| **Column Temperature (°C)** | Ambient | |
| **Sample Temperature (°C)** | Ambient | |
| **Mobile Phases** | (A) Water | |
| | (B) MeCN | |
| **Detection** | CAD and PDA at 210 nm | |
| **Flow Rate (mL/min)** | 1 mL/min | |

| **Gradient** | | |
|---|---|---|
| **Time (min)** | **% A** | **% B** |
| 0.0 | 22 | 78 |
| 30.0 | 22 | 78 |
| 30.01 - 37.0 | 90 | 10 |
| 37.01 | 22 | 78 |

**Table 4: Analytical HPLC Conditions for Fraction Analysis in Quinary Process**

| | | |
|---|---|---|
| **Column (Dimensions, mm)** | XBridge Phenyl (4.6 × 150 mm, 3.5 µm) PN186003352, SN012634135129 | |
| **Column Temperature (°C)** | Ambient | |
| **Sample Temperature (°C)** | Ambient | |
| **Mobile Phases** | (A) Water | |
| | (B) MeCN | |
| **Detection** | CAD and UV at 210 nm | |
| **Flow Rate (mL/min)** | 1 mL/min | |

| **Gradient** | | |
|---|---|---|
| **Time (min)** | **% A** | **% B** |
| 0.0 - 40.0 | 81 | 19 |
| 40.01 - 47.0 | 10 | 90 |
| 47.01 | 81 | 19 |

Primary Preparative HPLC Method. Primary processing was performed using either a pre-packed Waters XBridge RP18 column (50 × 250 mm, 7 µm) or Phenomenex Luna C18 (2) column (50 × 250 mm). The purification process was performed with a Waters Delta Prep LC Model 2000/4000 system coupled to a UV-Vis detector. Details of the preparative method are summarized in Table 5.

**Table 5: Conditions for Primary Preparative HPLC Method.**

| | | | |
|---|---|---|---|
| **Column** | Waters XBridge RP18 (50 × 250 mm, 7 µm) or Phenomenex Luna C18 (2) (50 × 250 mm) @ ambient | | |
| **Flow Rate (mL/min)** | 100 | | |
| **Detection** | UV at 220 nm | | |
| **Mobile Phases** | (A) 65:35 Water/Methanol (MeOH) with 0.05% HOAc | | |
| | (B) 40:60 Water/Methanol (MeOH) with 0.05% HOAc | | |
| | (C) MeOH | | |
| **Sample preparation** | 4-10 g dissolved in 80 mL of MP-A | | |

| **Gradient** | | | |
|---|---|---|---|
| **Time (min)** | **% A** | **% B** | **% C** |
| 0 - 35 | 100 | 0 | 0 |
| 50 - 63 | 0 | 100 | 0 |
| 63.1 - 68 | 0 | 0 | 100 |
| 68.1 | 100 | 0 | 0 |

Secondary Preparative HPLC Method. The secondary processing was performed using a Waters XBridge Phenyl (19 × 250 mm, 5 µm, PN 186004024, SN 125I130651II02) column. The purification process was performed with a Waters Delta Prep LC Model 2000/4000 system coupled to a UV-Vis detector. Details of the preparative method are summarized in Table 6.

**Table 6: Conditions for Secondary Preparative HPLC Method.**

| | |
|---|---|
| **Column** | Waters XBridge Phenyl (19 × 250 mm, 5 µm) |
| **Flow Rate (mL/min)** | 20 |
| **Detection** | UV at 210 nm |
| **Mobile Phases** | (A) 20% MeCN in Water |
| | (B) MeCN |
| **Sample preparation** | 250 mg, 50 mg/mL mostly water |
| **Gradient:** | Isocratic 100% MP-A for 40 min |

Tertiary and Quaternary Processing Method. The tertiary and quaternary processing were performed using a Waters XBridge Amide (19 × 250 mm, 5 µm, PN 186006606, SN 0107341600112 02)) column. The purification process was performed with a Waters Delta Prep LC Model 2000/4000 system coupled to a UV-Vis detector. Details of the preparative method are summarized in Table 7.

**Table 7: Conditions for Tertiary and Quaternary HPLC Process.**

| | |
|---|---|
| **Column** | Waters XBridge Amide (19 × 250 mm, 5 µm) |
| **Flow Rate (mL/min)** | 20 |
| **Detection** | UV at 210 nm |
| **Mobile Phases** | (A) 20% Water in MeCN |
| | (B) Water |
| **Sample preparation** | brought up in mostly organic solvent |
| **Gradient:** | 30-min linear ramp from 100% MP-A to 100% MP-B |

Quinary Preparative HPLC Method. The quinary processing was performed using a Waters XBridge Phenyl (19 × 250 mm, 5 µm, PN 186004024, SN 125I130651II02) column. The purification process was performed with a Waters Delta Prep LC Model 2000/4000 system coupled to a UV-Vis detector. Details of the preparative method are summarized in Table 8.

**Table 8: Conditions for Quinary Preparative HPLC Method.**

| | |
|---|---|
| **Column** | Waters XBridge Phenyl (19 × 250 mm, 5 µm) |
| **Flow Rate (mL/min)** | 20 |
| **Detection** | UV at 210 nm |
| **Mobile Phases** | (C) 20% MeCN in Water |
| | (D) MeCN |
| **Sample preparation** | brought up in mostly water |
| **Gradient:** | Isocratic 100% MP-A for 40 min |

Isolation Procedure. Fractions collected during the final pre-concentration step were filtered through a stainless steel sieve and concentrated *in vacuo* using a Buchi^{®} Rotary Evaporator, Model R-114. The concentrated solution was dried for 48 - 72 h using the Kinetics Flexi-Dry Personal Freeze Dryer.

MS and MS/MS. MS and MS/MS data were generated with a Waters QTof Micro mass spectrometer equipped with an electrospray ionization source. The sample was analyzed by negative ESI. The sample (0.1 mg) was diluted with H₂O to a concentration of 0.2 mg/mL for HRMS and MS/MS and introduced *via* direct infusion.

NMR. The sample was prepared by dissolving 2.9 mg in 150 µL of CD₃OD and NMR data were acquired on a Bruker Avance 500 MHz instrument equipped with a 2.5 mm inverse probe. The ¹H NMR spectrum was referenced to the CHD₂OD resonance at δ_{H} 3.30 ppm and ¹³C NMR spectrum was referenced to the CD₃OD resonance at δ_{C} 49.0 ppm.

### Results and Discussion

Unless otherwise noted, all solvent ratios are listed as percent by volume (v/v).

Primary Purification of **CC-00350.** Approximately 300 g of Stevia extract was processed using the primary preparative HPLC method described above. Collected fractions were analyzed by LC-MS using the analytical method summarized in Table 1.

Secondary Purification of **CC-00350.** The material collected from primary purification was processed with conditions summarized above. Collected fractions were analyzed using the analytical method summarized in Table 2.

Tertiary Purification of **CC-00350.** The material collected from secondary purification was processed with conditions summarized above. Collected fractions were analyzed using the analytical method summarized in Table 3.

Quaternary Purification of **CC-00350.** The material collected from teriatry purification was processed with conditions summarized above. Collected fractions were analyzed using the analytical method summarized in Table 3.

Quinary Purification of **CC-00350.** The material collected from quaternary purification was processed with conditions summarized above.

Final Batch Preparation of **CC-00350.** The fraction collected in quinary purification was concentrated by rotary evaporation for final isolation. The concentrated solution was further dried *via* lyophilization for 48 h. The final yield of the batch was 6.0mg. The retention time of **CC-00350** was 27.171 min for CAD and 27.115 min for UV according under the conditions described in Example 1.

Mass Spectrometry. The ESI-TOF mass spectrum acquired by infusing a sample of **CC-00350** showed a [M-H]⁻ ion at *m*/*z* 1127.4894. The mass of the [M-H]⁻ ion was in good agreement with the molecular formula C₅₀H₈₀O₂₈ (calcd for C₅₀H₇₉O₂₈: 1127.4758, error: -2.0 ppm) expected for CC-00350. The MS data confirmed that **CC-00350** has a nominal mass of 1128 Daltons with the molecular formula, C₅₀H₈₀O₂₈. The ion observed at *m*/*z* 1225.4415 was due to [M-H+H₃PO₄₁⁻.

The MS/MS spectrum of **CC-00350**, selecting the [M-H]⁻ ion at *m*/*z* 1127.0 for fragmentation, indicated sequential loss of five glucose units at *m*/*z* 965.4377, 803.3664, 641.3323, 479.2650 and 317.2084. The data thus indicated the presence of five glucose units in the structure. The ions at *m*/*z* 803.3664 and 317.2084 are weaker than other ions.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K) and 1D TOCSY (500 MHz, CD₃OD over a range of mixing times (40-140 msec) at 300K) were acquired to allow assignment of **CC-00350**.

The 1D and 2D NMR data indicated that the central core of the glycoside is a diterpene. HMBC correlation from the methyl protons at δ_{H} 1.24 to the carbonyl at δ_{C} 177.8 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.6, 45.2, and 58.5 allowed assignment of C-3, C-4, and C-5. Analysis of the ¹H-¹³C HSQC-DEPT data indicated that the carbon at δ_{C} 38.6 was a methylene and the carbon at δ_{C} 58.5 was a methine which were assigned as C-3 and C-5, respectively. This left the carbon at δ_{C} 45.2, which did not show a correlation in the HSQC-DEPT spectrum, to be assigned as the quaternary carbon, C-4. The ¹H chemical shifts for C-3 (δ_{H} 1.02 and 2.30) and C-5 (δ_{H} 1.07) were assigned using the HSQC-DEPT data. A COSY correlation between one of the H-3 protons (δ_{H} 1.02) and the protons at δ_{H} 1.42 and 2.02 allowed assignment of the H-2 protons which in turn showed COSY correlations to the protons at δ_{H} 0.85 and 1.87 which were assigned to H-1. The ¹³C chemical shifts for C-1 and C-2 were then assigned on the basis of HSQC-DEPT correlations.

The other tertiary methyl singlet, observed at δ_{H} 0.95 (δ_{C} 17.3), showed HMBC correlations to C-1 and C-5 and was assigned as H-20. The methyl protons showed additional HMBC correlations to a quaternary carbon (δ_{C} 40.6) and a methine carbon (δ_{C} 55.2) which were assigned as C-10 and C-9, respectively. The ¹H chemical shift for C-9 (δ_{H} 0.97) was assigned using the HSQC-DEPT data. COSY correlations between H-5 (δ_{H} 1.07) and protons at d_{H} 1.86 and 1.91 then allowed assignment of the H-6 protons which in turn showed correlations to protons at δ_{H} 1.42 and 1.55 which were assigned to H-7. The ¹³C chemical shifts for C-6 (δ_{C} 23.4) and C-7 (δ_{C} 42.9) were then determined from the HSQC-DEPT data. COSY correlations between H-9 (δ_{H} 0.97) and the proton at δ_{H} 1.63 allowed assignment of one of the H-11 protons. The ¹H chemical shift for remaining proton at C-11 (δ_{H} 1.79) was assigned using the HSQC-DEPT data. COSY correlations from H-11 protons (δ_{H} 1.63 and 1.79) to a proton at δ_{H} 2.02 allowed assignment of one of the H-12 protons. The ¹H chemical shift for remaining proton at C-12 (δ_{H} 1.52) was assigned using the HSQC-DEPT data. The HSQC-DEPT data was also used to assign C-11 (δ_{C} 21.1) and C-12 (δ_{C} 38.6). The olefinic proton observed at δ_{H} 4.83 (obscured by water resonance) and 5.21 showed HMBC correlations to a quaternary carbon at δ_{C} 88.4 (C-13) and were assigned to H-17 (δ_{C} 105.5 *via* HSQC-DEPT). The methine proton H-9 showed HMBC correlations to a quaternary carbon at δ_{C} 42.7 and a methylene carbon at δ_{C} 45.0 which were assigned as C-8 and C-14, respectively. The ¹H chemical shifts at C-14 (δ_{H} 1.51 and 2.25) were assigned using the HSQC-DEPT data. HMBC correlation from H-14 (δ_{H} 2.25) to a carbon at δ_{C} 48.1 allowed assignment of C-15. The ¹H chemical shifts at C-15 (δ_{H} 2.05 and 2.12) were assigned using the HSQC-DEPT data. HMBC correlations from H-14 (δ_{H} 2.25), H-15 (δ_{H} 2.12) and H-17 (δ_{H} 5.21) to a quaternary carbon at δ_{C} 153.6 allowed assignment of C-16 to complete the assignment of the central core.

Correlations observed in the ROESY spectrum were used to assign the partial relative stereochemistry of the central diterpene core. In the ROESY spectrum, NOE correlations were observed between H-20 (δ_{H} 0.95) and H-14 (δ_{H} 2.25) indicating that H-14 and H-20 are on the same face of the rings. Similarly, NOE correlations were observed between H-18 and H-5 but NOE correlations were not observed between H-5/H-18 and H-20/H-14 (δ_{H} 2.25) indicating that H-5 and H-18 were on the opposite face of the rings compared to H-14 and H-20. Based on available ROESY data, the relative stereochemistry at H-9 could not be determined, however based on comparison of ¹H and ¹³C chemical data with the reported data for related *Stevia* glycosides, the relative stereochemistry at H-9 is proposed as presented herein.

A summary of the ¹H and ¹³C chemical shifts for the aglycone are found in Table 9 and the structure of the aglycone core is provided in Figure 1.

**Table 9. ¹H and ¹³C NMR (500 and 125 MHz, CD₃OD) assignments of aglycone.**

| **Position** | **¹³C** | **¹H** |
|---|---|---|
| 1 | 41.5 | 0.85 m |
| | | 1.87 m |
| 2 | 20.4 | 1.42 m |
| | | 2.02 m |
| 3 | 38.6 | 1.02 m |
| | | 2.30 brd (13.2) |
| 4 | 45.2 | --- |
| 5 | 58.5 | 1.07 m |
| 6 | 23.4 | 1.86 m |
| | | 1.91 m |
| 7 | 42.9 | 1.42 m |
| | | 1.55 m |
| 8 | 42.7 | --- |
| 9 | 55.2 | 0.97 brd (8.2) |
| 10 | 40.6 | --- |
| 11 | 21.1 | 1.63 m |
| | | 1.79 m |
| 12 | 38.6 | 1.52 m |
| | | 2.02 m |
| 13 | 88.4 | --- |
| 14 | 45.0 | 1.51 m |
| | | 2.25 brd (11.5) |
| 15 | 48.1 | 2.05 m |
| | | 2.12 m |
| 16 | 153.6 | --- |
| 17 | 105.5 | 4.83^{¥} |
| | | 5.21 brs |
| 18 | 29.1 | 1.24 s |
| 19 | 177.8 | --- |
| 20 | 17.3 | 0.95 s |

| | | |
|---|---|---|
| *Resonance obscured by H₂O, assignment based on HSQC-DEPT data. | | |

Analysis of the ¹H-¹³C HSQC-DEPT data confirmed the presence of five anomeric protons which were well resolved at δ_{H} 5.56 (δ_{C} 94.3), 5.02 (δ_{C} 103.3), 4.75 (δ_{C} 104.0), 4.66 (δ_{C} 97.0), and 4.58 (δ_{C} 105.4) in the ¹H NMR spectrum acquired at 300K. All anomeric protons had large couplings (7.5 - 8.0 Hz) indicating that they had β-configurations. The anomeric proton observed at δ_{H} 5.56 showed an HMBC correlation to C-19 which indicated that it corresponded to the anomeric proton of Glci. Similarly, the anomeric proton observed at δ_{H} 4.66 showed a HMBC correlation to C-13 allowing it to be assigned as the anomeric proton of Glen.

A summary of the ¹H and ¹³C chemical shifts for the glycoside at C-19 are found in Table 10. A summary of the ¹H and ¹³C chemical shifts for the glycoside at C-13 are found in Table 11.

**Table 10. ¹H and ¹³C NMR (500 and 125 MHz, CD₃OD) assignments of the C-19 glycoside.**

| | **CC-00350** | |
|---|---|---|
| **Position** | **¹³C** | **¹H** |
| Glc_{I}-1 | 94.3 | 5.56 d (7.9) |
| Glc_{I}-2 | 77.3 | 3.99 t (8.5) |
| Glc_{I}-3 | 87.8 | 4.05 t (8.5) |
| Glc_{I}-4 | 69.9 | 3.50 m |
| Glc_{I}-5 | 77.9-78.5^{§} | 3.47 m |
| Glc_{I}-6 | 62.4 | 3.70 m, 3.84 m |
| Glc_{V}-1 | 103.3 | 5.02 d (8.0) |
| Glc_{V}-2 | 76.0^{¥} | 3.18 m |
| Glc_{V}-3 | 77.9-78.5^{§} | 3.36 m |
| Glc_{V}-4 | 72.8 | 3.19 m |
| Glc_{V}-5 | 77.9-78.5^{§} | 3.32 m |
| Glc_{V}-6 | 63.7 | 3.65 m, 3.90 m |
| Glc_{VI}-1 | 104.0 | 4.75 d (7.9) |
| Glc_{VI}-2 | 75.4 | 3.29 m |
| Glc_{VI}-3 | 77.9-78.5^{§} | 3.38 m |
| Glc_{VI}-4 | 71.6 or 71.7 | 3.28 m |
| Glc_{VI}-5 | 77.9-78.5^{§} | 3.43 m |
| Glc_{VI}-6 | 62.6 | 3.63 m, 3.91 m |

| | | |
|---|---|---|
| ^{§}Eight carbon resonances in the range of 77.9-78.5 ppm (77.85, 77.88, 77.94, 78.01, 78.14, 78.34, 78.39 and 78.49), hence chemical shifts could not be unequivocally assigned. ^{¥}Two overlapping carbon resonances (Glcv C-2 and Glcm C-2) observed at 76.0 ppm. | | |

**Table 11. ¹H and ¹³C NMR (500 and 125 MHz, CD₃OD), assignments of C-13 glycoside.**

| | **CC-00350** | |
|---|---|---|
| **Position** | **¹³C** | **¹H** |
| GIc_{II}-1 | 97.0 | 4.66 d (7.8) |
| GIc_{II}-2 | 82.6 | 3.43 m |
| GIc_{II}-3 | 77.9-78.5^{§} | 3.66 m |
| GIc_{II}-4 | 71.6 or 71.7 | 3.32 m |
| GIc_{II}-5 | 77.9-78.5^{§} | 3.21 m |
| GIc_{II}-6 | 62.8 | 3.64 m, 3.79 m |
| Glc_{III}-1 | 105.4 | 4.58 d (7.5) |
| Glc_{III}-2 | 76.0^{¥} | 3.31 m |
| Glc_{III}-3 | 77.9-78.5^{§} | 3.36 m |
| GIc_{III}-4 | 72.2 | 3.17 m |
| GIc_{III}-5 | 77.9-78.5^{§} | 3.25 m |
| GIc_{III}-6 | 63.3 | 3.61 m, 3.82 m |

| | | |
|---|---|---|
| ^{§}Eight carbon resonances in the range of 77.9-78.5 ppm (77.85, 77.88, 77.94, 78.01, 78.14, 78.34, 78.39 and 78.49), hence chemical shifts could not be unequivocally assigned. ^{¥}Two overlapping carbon resonances (Glc_{V} C-2 and Glc_{III} C-2) observed at 76.0 ppm. | | |

The structure of **CC-00350** was determined to be (13-[(2-*O*-β-D-glucopyranosyl)- β-D-glucopyranosyl)oxy] *ent*-kaur-16-en-19-oic acid (2-*O*- β-D-glucopyranosyl-3-*O*- β-D-glucopyranosyl)- β-D-glucopyranosyl) ester].

### EXAMPLE 6: Isolation and Characterization of CC-00327 (Reference)

The sample used for isolation of **CC-00327** was Stevia extract.

HPLC and Isolation. HPLC analyses were performed on a Waters 2695 Alliance System coupled to a Waters 996 Photo Diode Array (PDA) detector. In addition, sample purities were assessed using an ESA Corona Charged Aerosol Detector (CAD). Sample analyses were performed using the method conditions described in Tables 1-3.

**Table 1: Analytical HPLC Conditions for Fraction Analysis in Primary Process**

| | | |
|---|---|---|
| **Column** | Phenomenex Synergi Hydro RP (4.6 × 250 mm, 4 µm) | |
| **Column Temperature** | 55 | |
| **(°C)** | | |
| **Sample Temperature (°C)** | Ambient | |
| **Mobile Phases** | (C) 0.0284% ammonium acetate (NH₄OAc) and 0.0116% acetic acid (HOAc) in water | |
| | (D) MeCN | |
| **Flow Rate (mL/min)** | 1.0 | |
| **Detection** | CAD and UV at 210 nm | |

| **Gradient** | | |
|---|---|---|
| **Time (min)** | **% A** | **%B** |
| 0.0 - 8.5 | 75 | 25 |
| 10.0 | 71 | 29 |
| 16.5 | 70 | 30 |
| 18.5 - 24.5 | 66 | 34 |
| 26.5 - 29.0 | 48 | 52 |
| 31.0 - 37.0 | 30 | 70 |
| 38.0 | 75 | 25 |

**Table 2: Analytical HPLC Conditions for Fraction Analysis in Secondary Process**

| | | |
|---|---|---|
| **Column (Dimensions, mm)** | XBridge Phenyl (4.6 × 150 mm, 3.5 µm) | |
| **Column Temperature (°C)** | Ambient | |
| **Sample Temperature (°C)** | Ambient | |
| **Mobile Phases** | (C) Water | |
| | (D) MeCN | |
| **Detection** | CAD and UV at 210 nm | |
| **Flow Rate (mL/min)** | 1 mL/min | |

| **Gradient** | | |
|---|---|---|
| **Time (min)** | **% A** | **% B** |
| 0.0 - 30.0 | 79 | 21 |
| 30.0 - 35.0 | 95 | 5 |
| 35.0 - 40.0 | 79 | 21 |

**Table 3: Analytical HPLC Conditions for Fraction Analysis in Tertiary Process**

| | | | |
|---|---|---|---|
| **Column (Dimensions, mm)** | | Waters XBridge Amide (4.6 × 150 mm, 3.5 µm) | |
| **Column Temperature (°C)** | | Ambient | |
| **Sample Temperature (°C)** | | Ambient | |
| **Mobile Phases** | | (C) Water | |
| | | (D) MeCN | |
| **Detection** | | CAD and PDA at 210 nm | |
| **Flow Rate (mL/min)** | | 1 mL/min | |

| **Gradient** | | | |
|---|---|---|---|
| **Time (min)** | **% A** | | **% B** |
| 0.0 - 30.0 | 20 | | 80 |
| 30.0 - 35.0 | 95 | | 5 |
| 35.0 - 40.0 | 20 | | 80 |

Primary Preparative HPLC Method. Primary processing was performed using a pre-packed Waters Symmetry C18 column (50 × 250 mm, 7 µm). The purification process was performed with a Waters Delta Prep LC Model 2000/4000 system coupled to a UV-Vis detector. Details of the primary preparative method are summarized in Table 4.

**Table 4: Conditions for primary preparative HPLC method**

| | | |
|---|---|---|
| **Column** | Waters Symmetry C18 (50 × 250 mm) | |
| **Flow Rate (mL/min)** | 140 | |
| **Detection** | UV at 220 nm | |
| **Mobile Phases** | (A) 70:30 Water/MeCN | |
| | (B) MeCN | |

| | | |
|---|---|---|
| **Load (mL)** | ~160 | |
| **Sample preparation** | ~19.5 g of steviol glycosides dissolved 160 mL of water | |

| **Time (min)** | **%A** | **%B** |
|---|---|---|
| 0 - 70 | 100 | 0 |
| 70 - 80 | 10 | 90 |

Secondary Preparative HPLC Method. Secondary processing was performed using a pre-packed Waters XBridge Phenyl OBD column (19 × 250 mm, 5 µm). The purification process was performed with a Waters Delta Prep LC Model 2000/4000 system coupled to a UV-Vis detector. Details of the preparative method are summarized in Table 5.

**Table 5: Conditions for Secondary Preparative HPLC Method**

| | | |
|---|---|---|
| **Column** | Waters XBridge Phenyl OBD (19 × 250 mm) | |
| **Flow Rate (mL/min)** | 17 | |
| **Detection** | UV at 210 nm | |
| **Mobile Phases** | (A) 79:21 Water/MeCN | |
| | (B) MeCN | |
| **Load (mL)** | ~5 | |
| **Sample preparation** | dissolve in 5 mL of mobile phase A (MP-A) | |

| **Time (min)** | **%A** | **%B** |
|---|---|---|
| 0 - 50 | 100 | 0 |
| 50 - 60 | 10 | 90 |

Tertiary Preparative HPLC Method. Tertiary processing was performed using a pre-packed Waters XBridge Amide column (19 × 250 mm, 5 µm). The purification process was performed with a Waters Delta Prep LC Model 2000/4000 system coupled to a UV-Vis detector. Details of the preparative method are summarized in Table 6.

**Table 6: Conditions for Tertiary Preparative HPLC Method**

| | | |
|---|---|---|
| **Column** | Waters XBridge Amide (19 × 250 mm) | |
| **Flow Rate (mL/min)** | 17 | |
| **Detection** | UV at 210 nm | |
| **Mobile Phases** | (A) 20:80 Water/MeCN | |
| | (B) 80:20 Water/MeCN | |
| **Load (mL)** | ~5-10 | |
| **Sample preparation** | dissolve in 5 mL of MP-A | |

| **Time (min)** | **%A** | **%B** |
|---|---|---|
| 0.0 - 50 | 100 | 0 |
| 50 - 60 | 10 | 90 |

Quaternary Preparative HPLC Method. The quaternary processing of lot MLSG-0907001(A1A4) was performed using a pre-packed Waters XBridge Amide column (19 × 250 mm, 5 µm). The purification process was performed with a Waters Delta Prep LC Model 2000/4000 system coupled to a UV-Vis detector. Details of the preparative method are summarized in Table 7.

**Table 7: Conditions for quaternary preparative HPLC method**

| | | |
|---|---|---|
| **Column** | Waters XBridge Amide (19 × 250 mm) | |
| **Flow Rate (mL/min)** | 17 | |
| **Detection** | UV at 210 nm | |
| **Mobile Phases** | (A) 20:80 Water/MeCN | |
| | (B) 80:20 Water/MeCN | |
| **Load (mL)** | ~8 | |
| **Sample preparation** | ~8 mL in MP-A | |

| **Time (min)** | **%A** | **%B** |
|---|---|---|
| 0 - 50 | 100 | 0 |
| 50 - 60 | 0 | 100 |

Quinary Preparative HPLC Method. The quinary processing was performed using a pre-packed Waters XBridge Amide column (19 × 250 mm, 5 µm). The purification process was performed with a Waters Delta Prep LC Model 2000/4000 system coupled to a UV-Vis detector. Details of the preparative method are summarized in Table 8.

**Table 8: Conditions for Quinary Preparative HPLC Method**

| | | |
|---|---|---|
| **Column** | Waters XBridge Amide (19 × 250 mm) | |
| **Flow Rate (mL/min)** | 17 | |
| **Detection** | UV at 210 nm | |
| **Mobile Phases** | (C) 20:80 Water/MeCN | |
| | (D) 80:20 Water/MeCN | |
| **Load (mL)** | ~10 | |
| **Sample preparation** | Combined fractions from quaternary processing in MP-A | |

| **Time (min)** | **%A** | **%B** |
|---|---|---|
| 0 - 50 | 100 | 0 |
| 50 - 60 | 0 | 100 |

Isolation Procedure. Purified fractions were concentrated *in vacuo* using a Buchi^{®} Rotary Evaporator, Model R-114. The concentrated solution was dried for 48 - 72 h using the Vertis Freezemobile Freeze Dryer.

MS and MS/MS. MS and MS/MS data were generated with a Waters QTof Micro mass spectrometer equipped with an electrospray ionization source. The sample was analyzed by negative ESI. The sample (∼0.08 mg) was diluted with 50:50 H₂O:ACN to a concentration of 20 µg/mL for HRMS and MS/MS and were introduced *via* direct infusion.

NMR. The sample was prepared by dissolving 1.4 mg in 150 µL of CD₃OD and NMR data were acquired on Bruker Avance 500 MHz instrument equipped with a 2.5 mm inverse probe and a 5 mm broad band probe. The ¹H NMR spectrum was referenced to the CHD₂OD resonance at δ_{H} 3.30 ppm and ¹³C NMR spectrum was referenced to the CD₃OD resonance at δ_{C} 49.0 ppm.

### Results and Discussion

Unless otherwise noted, all solvent ratios are listed as percent by volume (v/v).

Primary Purification of **CC-00327.** Approximately 120 g of *Stevia* extract was processed using the primary preparative HPLC method described above. Collected fractions were analyzed by HPLC using the analytical methods summarized above.

Secondary Purification of **CC-00327.** Material collected from primary purifiection was pooled, concentrated, and then processed with conditions summarized above. Fractions were analyzed using the analytical method summarized above. Four peaks were observed: 11.609 min, 12.307 min, 13.592 min and 14.392 min.

Tertiary Purification of **CC-00327.** Material collected from secondary purification was processed with conditions summarized above. Fractions were analyzed using the analytical method summarized above. Four peaks were observed: 4.720 min, 7.300 min, 7.526 min and 9.063 min.

Quaternary Purification of **CC-00327.** Material collected from tertiary purification was processed with conditions summarize above. Fractions were analyzed using the analytical method summarized above. Four peaks were observed: 4.721 min, 7.184 min, 7.514 min and 9.055 min.

Quinary Purification of **CC-00327.** Material collected from quaternary purification was processed with conditions summarized above. Fractions were analyzed using the analytical method summarized above. The material had a single peak at 9.246 min.

Final Batch Preparation. Material collected from quinary purification was concentrated by rotary evaporation and lyophilized for about 72 h. The HPLC analysis was performed using the method summarized above. The final batch had a purity of 95.8% (AUC, CAD) according to the conditions in Example 1 with a retention time of 33.007 min for CAD.

Mass Spectrometry. The ESI-TOF mass spectrum acquired by infusing a sample of **CC-00327** showed a [M-H]⁻ ion at *m*/*z* 967.4461. The mass of the [M-H]⁻ ion was in good agreement with the molecular formula C₄₄H₇₂O₂₃ (calcd for C₄₄H₇₁O₂₃: 967.4386, error: 2.4 ppm) expected . The MS data confirmed that **CC-00327** has a nominal mass of 968 Daltons with the molecular formula, C₄₄H₇₂O₂₃. The ions observed at *m*/*z* 1003.3870 and 1081.4044 were due to [M+Cl]⁻and [M-H+TFA]⁻, respectively.

The MS/MS spectrum of **CC-00327**, selecting the [M-H]⁻ ion at *m*/*z* 967.0 for fragmentation, indicated loss of one glucose unit at *m*/*z* 805.3484; however, it did not show any additional fragmentation with collision energy of 25 eV. When higher collision energy was applied (40 eV), the parent ion was not observed but the ion at *m*/*z* 805.3622 was again observed corresponding to loss of one sugar. The ion at *m*/*z* 643.3157 corresponds to loss of another glucose unit followed by loss of rhamnose and third glucose at *m*/*z* 497.2592 and 335.2191, respectively. The mass fragmentation pattern thus indicated the presence of three glucose and one rhamnose units in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at300K over a range of mixing times (40-140 msec)) were acquired to allow assignment of **CC-00327.**

The 1D and 2D NMR data indicated that the central core of the glycoside is a diterpene. An HMBC correlation from the methyl protons at δ_{H} 1.20 to the carbonyl at δ_{C} 178.5 allowed assignment of one of the tertiary methyl groups (C-18) as well as C-19 and provided a starting point for the assignment of the rest of the aglycone. Additional HMBC correlations from the methyl protons (H-18) to carbons at δ_{C} 39.1, 45.0, and 58.4 allowed assignment of C-3, C-4, and C-5. Analysis of the ¹H-¹³C HSQC-DEPT data indicated that the carbon at δ_{C} 39.1 was a methylene and the carbon at δ_{C} 58.4 was a methine which were assigned as C-3 and C-5, respectively. This left the carbon at δ_{C} 45.0, which did not show a correlation in the HSQC-DEPT spectrum, to be assigned as the quaternary carbon, C-4. The ¹H chemical shifts for C-3 (δ_{H} 1.05 and 2.13) and C-5 (δ_{H} 1.11) were assigned using the HSQC-DEPT data. COSY correlations between the H-3 protons (δ_{H} 1.05 and 2.13) and protons at δ_{H} 1.42 and 1.95 allowed assignment of the H-2 protons which in turn showed correlations with the protons at δ_{H} 0.85 and 1.83 which were assigned to H-1. The remaining ¹³C chemical shifts for C-1 and C-2 were then assigned on the basis of HSQC-DEPT correlations.

The other tertiary methyl singlet, observed at δ_{H} 1.00 (δ_{C} 16.1), showed HMBC correlations to C-1 and C-5 and was assigned as H-20. The methyl protons showed additional HMBC correlations to a quaternary carbon (δ_{C} 40.8) and a methine carbon (δ_{C} 56.1) which were assigned as C-10 and C-9, respectively. COSY correlations between H-5 (δ_{H} 1.11) and protons at δ_{H} 1.80 and 2.05 then allowed assignment of the H-6 protons which in turn showed correlations to protons at δ_{H} 1.37 and 1.60 which were assigned to H-7. The ¹³C chemical shifts for C-6 (δ_{C} 23.0) and C-7 (δ_{C} 43.4) were then determined from the HSQC-DEPT data. COSY correlations between H-9 (δ_{H} 0.89) and a proton at δ_{H} 1.65 allowed assignment of one of the H-11 protons. The ¹H chemical shift for the remaining proton at C-11 (δ_{H} 1.76) was assigned using the HSQC-DEPT data. The ¹³C chemical shift for C-11 (δ_{C} 20.8) was also determined from the HSQC-DEPT data. Position 12 could not be assigned unambiguously using correlations from H-9 or H-11. Therefore as discussed below the third tertiary methyl resonance observed at δ_{H} 1.23 was used as a starting point to assign the remaining unassigned positions of central core.

The third tertiary methyl singlet, observed at δ_{H} 1.23 (δ_{C} 22.1 *via* HSQC-DEPT), showed HMBC correlations to δ_{C} 55.6, 78.2 or 78.3 or 78.4 and 89.4. Analysis of the ¹H-¹³C HSQC-DEPT data indicated that the carbon at δ_{C} 55.6 was a methylene (δ_{H} 1.44 and 1.59 *via* HSQC-DEPT) and assigned to C-15, whereas the carbon at δ_{C} 89.4 which did not show correlations in the HSQC-DEPT spectrum was assigned as the quaternary carbon C-13. Based on comparison with other related *Stevia* glycosides and the quaternary carbon at 78.2 or 78.3 or 78.4 was then assigned to C-16. The upfield carbon chemical shift of C-16 was indicative of a hydroxyl group at this position. The tertiary methyl group at δ_{H} 1.23 was then assigned to position 17. One of the H-15 protons (δ_{H} 1.44) showed HMBC correlations to the methylene carbon at δ_{C} 40.2 which was assigned to C-14. The ¹H chemical shifts at C-14 (δ_{H} 2.00 and 2.07) were assigned using the HSQC-DEPT data. Although weak, HMBC correlations from H-14 to a carbon at δ_{C} 32.8 allowed assignment of C-12 and its protons at δ_{H} 1.74 and 1.77 were then assigned *via* HSQC-DEPT. These assignments were further confirmed by HMBC correlations from H-14 (δ_{H} 2.00) and H-15 (δ_{H} 1.59) to C-16 and H-14 to C-13 and C-15. HMBC correlations observed from H-11 (δ_{H} 1.76)/H-14 and H-15 to δ_{C} 43.4 allowed the assignment of remaining unassigned carbon C-8 to complete the assignment of the central core.

Correlations observed in the ROESY spectrum were used to assign the relative stereochemistry of the central diterpene core. In the ROESY spectrum, NOE correlations were observed between H-14 and H-20 indicating that H-14 and H-20 are on the same face of the rings. Similarly, NOE correlations were observed between H-9 and H-5 as well as H-5 and H-18 but NOE correlations were not observed between H-5/H-9/H-18 and H-14/H-20 indicating that H-5, H-9 and H-18 were on the opposite face of the rings compared to H-14 and H-20. The relative stereochemistry of hydroxyl group at C-16 could not be assigned unambiguously due to presence of other methyl protons close to H-17 protons at ~δ_{H} 1.23.

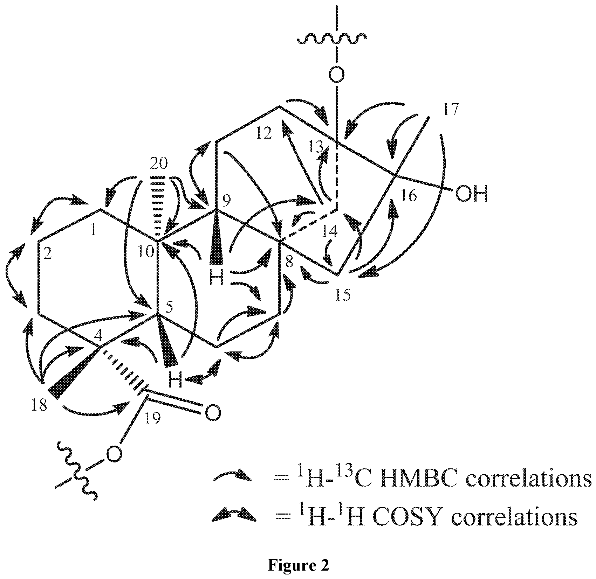

A summary of the ¹H and ¹³C chemical shifts for the aglycone are found in Table 9 and a summary of the key HMBC and COSY correlations used to assign the aglycone region are provided in Figure 2.

**Table 9. ¹H and ¹³C NMR (500 and 150 MHz, CD₃OD) assignments of the aglycone.**

| | **CC-00327** | |
|---|---|---|
| **Position** | **¹³C** | **¹H** |
| 1 | 41.8 | 0.85 m |
| | | 1.83 m |
| 2 | 19.9 | 1.42 m |
| | | 1.95 m |
| 3 | 39.1 | 1.05 m |
| | | 2.13 brd (13.0) |
| 4 | 45.0 | --- |
| 5 | 58.4 | 1.11 brd (11.8) |
| 6 | 23.0 | 1.80 m |
| | | 2.05 m |
| 7 | 43.4 | 1.37 m |
| | | 1.60 m |
| 8 | 43.4 | --- |
| 9 | 56.1 | 0.89 m |
| 10 | 40.8 | --- |
| 11 | 20.8 | 1.65 m |
| | | 1.76 m |
| 12 | 32.8 | 1.74 m |
| | | 1.77 m |
| 13 | 89.4 | --- |
| 14 | 40.2 | 2.00 m |
| | | 2.07 m |
| 15 | 55.6 | 1.44 d (14.1) |
| | | 1.59 d* (14.5) |
| 16 | 78.2 or 78.3 or 78.4 | --- |
| 17 | 22.1 | 1.23 s |
| 18 | 28.6 | 1.20 s |
| 19 | 178.5 | --- |
| 20 | 16.1 | 1.00 s |

| | | |
|---|---|---|
| *Resonance partially overlapped with one of the H-7 protons. | | |

Analysis of the ¹H-¹³C HSQC-DEPT data for **CC-00327** confirmed the presence of four anomeric protons out of which two were well resolved at δ_{H} 5.48 (δ_{C} 101.6) and 5.34 (δ_{C} 95.8) and two were partially overlapped and observed at δ_{H} 4.50 (δ_{C} 98.5) and 4.49 (δ_{C} 104.2) in the ¹H NMR spectrum acquired at 300 K. Three of the anomeric protons at δ_{H} 5.34, 4.50 and 4.49 had large couplings (7.6-8.2 Hz) indicating that they had β-configurations. One of the anomeric protons at δ_{H} 5.48 observed as a broad singlet in ¹H NMR spectrum resolved to a doublet in the 1D-TOCSY spectrum had a small coupling of 1.9 Hz indicating that this had α-configuration. The anomeric proton observed at δ_{H} 5.34 showed an HMBC correlation to C-19 which indicated that it corresponded to the anomeric proton of Glci. Similarly, the anomeric proton observed at δ_{H} 4.50 showed an HMBC correlation to C-13 allowing it to be assigned as the anomeric proton of Glc_{II}.

The ¹H and ¹³C chemical shifts for the C-19 glycoside are provided in Table 10. The ¹H and ¹³C chemical shifts for the C-13 glycoside are provided in Table 5.

**Table 10. ¹H and ¹³C NMR (500 and 150 MHz, CD₃OD) assignments of the C-19 glycoside.**

| | **CC-00327** | |
|---|---|---|
| **Position** | **¹³C** | **¹H** |
| Glc_{I}-1 | 95.8 | 5.34 d (8.2) |
| Glc_{I}-2 | 73.9 or 74.0 | 3.35 m |
| Glc_{I}-3 | 78.8 or 78.9 | 3.41 m |
| Glc_{I}-4 | 70.7 or 71.0 | 3.36 m |
| Glc_{I}-5 | 78.2-78.9^{§} | 3.37 m |
| Glc_{I}-6 | 62.4 | 3.68 m |
| | | 3.83 brd (12.0) |

| | | |
|---|---|---|
| ^{§}Five carbon resonances in the range of 78.2-78.9 (78.24, 78.33, 78.36, 78.80 and 78.85), hence chemical shifts could not be unequivocally assigned. | | |

**Table 11. ¹H and ¹³C NMR (500 and 150 MHz, CD₃OD) assignments of the C-13 glycoside.**

| | **CC-00327** | |
|---|---|---|
| **Position** | **¹³C** | **¹H** |
| Glc_{II}-1 | 98.5 | 4.50 d (7.6) |
| Glc_{II}-2 | 76.6 | 3.59 m |
| Glc_{II}-3 | 89.6 | 3.72 m |
| Glc_{II}-4 | 70.7 or 71.0 | 3.37 m |
| Glc_{II}-5 | 77.2 | 3.34 m |
| Glc_{II}-6 | 63.0 | 3.59m, 3.89 m |
| Rha-1 | 101.6 | 5.48 brs* |
| Rha-2 | 72.2 | 3.93 m |
| Rha-3 | 71.9 | 3.67 m |
| Rha-4 | 73.9 or 74.0 | 3.39 m |
| Rha-5 | 70.0 | 4.03 m |
| Rha-6 | 18.3 | 1.22 d (6.3)^{¥} |
| Glc_{IV}-1 | 104.2 | 4.49 d (7.6) |
| Glc_{IV}-2 | 75.2 | 3.25 m |
| Glc_{IV}-3 | 78.2-78.9^{§} | 3.36 m |
| Glc_{IV}-4 | 71.5 | 3.28 m |
| Glc_{IV}-5 | 78.2-78.9^{§} | 3.35 m |
| Glc_{IV}-6 | 62.6 | 3.62 m, 3.89 m |

| | | |
|---|---|---|
| *Resonance not resolved. *Resonance partially overlapped with H-17 protons. ^{§}Five carbon resonances in the range of 78.2-78.9 (78.24, 78.33, 78.36, 78.80 and 78.85), hence chemical shifts could not be unequivocally assigned. | | |

The structure of **CC-00327** was determined to be (13-[(2-O-α-L-rhamnopyranosyl-3-O-β-D-glucopyranosyl)- β-D-glucopyranosyl)oxy]-ent-kaur-16-hydroxy-17-methyl-19-oic acid-( β-D-glucopyranosyl) ester].

### EXAMPLE 7: Isolation and Characterization of CC-00340 (Reference)

The sample used for isolation of **CC-00340** was *Stevia* extract.

HPLC and Isolation. Sample analyses were performed using the analytical HPLC method conditions described in Example 1. Preparative HPLC purification and isolation procedure was also carried out using the method conditions described in Example 1- with the exception of the secondary preparative conditions, where mobile phase (A) was 18% MeCN in water as opposed to 20% MeCN in water.

Isolation Procedure. Fractions collected during the final pre-concentration step were filtered through a stainless steel sieve and concentrated *in vacuo* using a Buchi^{®} Rotary Evaporator, Model R-114. The concentrated solution was dried for 48 - 72 h using the Kinetics Flexi-Dry Personal Freeze Dryer.

MS and MS/MS. MS and MS/MS data were generated with a Waters QTof Micro mass spectrometer equipped with an electrospray ionization source. The sample was analyzed by negative ESI. The sample (~0.2 mg) was diluted with 50:50 H₂O:ACN to a concentration of 200 µg/mL for HRMS and MS/MS and were introduced *via* direct infusion.

NMR. The sample was prepared by dissolving 2.0 mg in 130 µL of CD₃OD and NMR data were acquired on a Bruker Avance 500 MHz instrument equipped with a 2.5 mm inverse probe and a 5 mm broad band probe. The ¹H NMR spectrum was referenced to the CHD₂OD resonance at δ_{H} 3.30 ppm and ¹³C NMR spectrum was referenced to the CD₃OD resonance at δ_{C} 49.0 ppm.

### Results and Discussion

Unless otherwise noted, all solvent ratios are listed as percent by volume (v/v).

Primary Purification of **CC-00340.** Approximately 300 g of *Stevia* extract was processed using the primary preparative HPLC method described above. Collected fractions were analyzed by LC-MS using the analytical method summarized above.

Secondary Purification of **CC-00340.** Material collected from primary purification was processed with conditions summarized above. Fractions were analyzed using the analytical method summarized above.

Tertiary Purification of **CC-00340.** Material collected from secondary purification was processed with conditions summarized above. Fractions were analyzed using the analytical method summarized above.

Final Batch Preparation of **CC-00340.** Material collected from tertiary purification was concentrated by rotary evaporation for final isolation. The concentrated solution was further dried *via* lyophilization for 48 h. The final yield of the batch was 5.2 mg. The retention time of **CC-00340** was 22.846 min for CAD and 22.829 min for UV according under the conditions described in Example 1.

Mass Spectrometry. The ESI-TOF mass spectrum acquired by infusing a sample of **CC-00340** showed a [M-H]⁻ ion at *m*/*z* 981.3842. The mass of the [M-H]⁻ ion was in good agreement with the molecular formula C₄₄H₇₀O₂₄ (calcd for C₄₄H₆₉O₂₄: 981.4179, error: 0.0 ppm) expected. The MS data confirmed that **CC-00340** has a nominal mass of 982 Daltons with the molecular formula, C₄₄H₇₀O₂₄.

The MS/MS spectrum of **CC-00340**, selecting the [M-H]⁻ ion at *m*/*z* 981.0 for fragmentation, indicated sequential loss of four glucose units at *m*/*z* 819.3353, 657.3010, 495.2528 and 333.1919 indicating the presence of four glucose units in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at300K over a range of mixing times (40-140 msec))were acquired to allow assignment of **CC-00340.**

The 1D and 2D NMR data indicated that the central core of the glycoside is a diterpene. An HMBC correlation from the methyl protons at δ_{H} 1.23 to the carbonyl at δ_{C} 178.4 allowed assignment of one of the tertiary methyl groups (C-18) as well as C-19 and provided a starting point for the assignment of the rest of the aglycone. Additional HMBC correlations from the methyl protons (H-18) to carbons at δ_{C} 39.1, 45.0, and 58.5 allowed assignment of C-3, C-4, and C-5. Analysis of the ¹H-¹³C HSQC-DEPT data indicated that the carbon at δ_{C} 39.1 was a methylene and the carbon at δ_{C} 58.5 was a methine which were assigned as C-3 and C-5, respectively. This left the carbon at δ_{C} 45.0, which did not show a correlation in the HSQC-DEPT spectrum, to be assigned as the quaternary carbon, C-4. The ¹H chemical shifts for C-3 (δ_{H} 1.05 and 2.15) and C-5 (δ_{H} 1.11) were assigned using the HSQC-DEPT data. A COSY correlation between H-3 protons (δ_{H} 1.05 and 2.15) and a proton at δ_{H} 1.41 allowed assignment of one of the H-2 proton which in turn showed correlation with a proton at δ_{H} 0.84 which was assigned to H-1. The remaining ¹H and ¹³C chemical shifts for C-1 and C-2 were then assigned on the basis of additional COSY and HSQC-DEPT correlations.

The other tertiary methyl singlet, observed at δ_{H} 0.97, showed HMBC correlations to C-1 and C-5 and was assigned as H-20. The methyl protons showed additional HMBC correlations to a quaternary carbon (δ_{C} 40.9) and a methine carbon (δ_{C} 53.8) which were assigned as C-10 and C-9, respectively. The HSQC-DEPT data was then used to assign H-9 (δ_{H} 0.93). COSY correlations between H-5 (δ_{H} 1.11) and protons at δ_{H} 1.90 and 1.93 then allowed assignment of the H-6 protons which in turn showed correlations to protons at δ_{H} 1.33 and 1.75 which were assigned to H-7. The ¹³C chemical shifts for C-6 (δ_{C} 22.2) and C-7 (δ_{C} 36.6) were then determined from the HSQC-DEPT data. The H-9 proton (δ_{H} 0.93) showed HMBC correlation to a quaternary carbon at δ_{C} 47.1 which was assigned to C-8. Similarly, H-9 proton showed additional HMBC correlation to a methylene carbon at δ_{C} 20.9 assigned to C-11. HSQC-DEPT data was then used to assign C-11 protons at δ_{H} 1.44 and 1.78. One of the C-11 protons (δ_{H} 1.78) showed COSY correlation to a proton at δ_{H} 1.91 which was assigned as the H-12 proton. The HSQC-DEPT data was then used to assign C-12 (δ_{C} 39.1) and remaining proton at H-12 (δ_{H} 1.50). HMBC correlations from H-11 (δ_{H} 1.78) and H-12 (δ_{H} 1.91) to a quaternary carbon at δ_{C} 87.9 allowed assignment ofC-13. The olefinic protons observed at δ_{H} 5.31 and 5.51 showed HMBC correlations to the same carbon at δ_{C} 87.9 (C-13) and were assigned to H-17 (δ_{C} 111.2 *via* HSQC-DEPT). A proton at δ_{H} 2.07 showed HMBC correlations to C-12 (δ_{C} 39.1) and was assigned to H-14. HSQC-DEPT data was used to assign remaining H-14 (δ_{H} 1.76) as well as the C-14 (δ_{C} 41.4). HMBC correlations from H-14 (δ_{H} 2.07) and H-17 to a carbon at δ_{C} 157.3 allowed assignment of C-16. Additional HMBC correlations from H-9, H-14 and H-17 to a carbon at δ_{C} 82.4 indicated a change at C-15, which was the only unassigned carbon in the central core. The downfield chemical shift compared to other *Stevia* glycosides indicated the presence of a hydroxyl group at C-15. This was further supported by downfield chemical shift of H-15 (δ_{H} 3.69) which was assigned using the HSQC-DEPT data. HMBC correlations from H-15 to C-9, C-13 and C-16 further confirmed the assignments made above to complete the assignment of the central core.

Correlations observed in the NOESY spectrum were used to assign the partial relative stereochemistry of the central diterpene core. In the NOESY spectrum, NOE correlations were observed between H-14 (δ_{H} 2.07) and H-20 indicating that H-14 and H-20 are on the same face of the rings. Similarly, although weak, NOE correlations were observed between H-18 and H-5 as well as H-5 and H-9 but NOE correlations were not observed between H-9 and H-20/H-14 (δ_{H} 2.16) indicating that H-5, H-9 and H-18 were on the opposite face of the rings compared to H-14 and H-20. The relative stereochemistry of hydroxyl group at C-15 could not be assigned unambiguously due to several overlapping protons at ~δ_{H} 3.69.

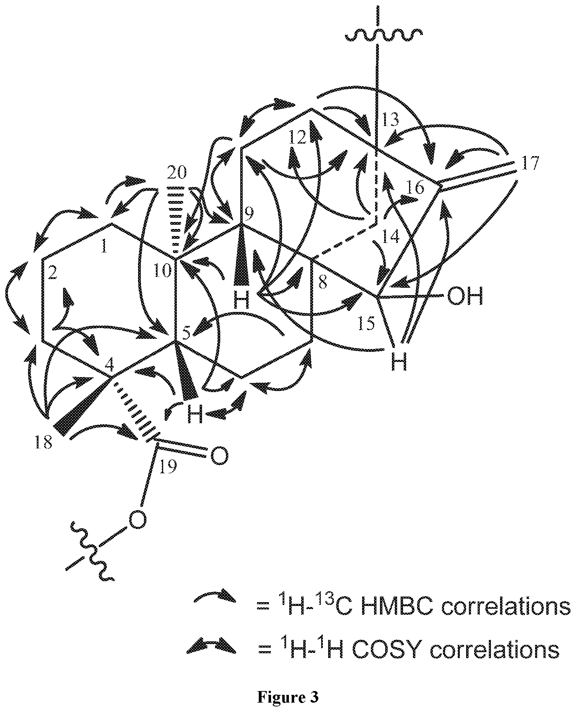

A summary of the ¹H and ¹³C chemical shifts for the aglycone are found in Table 1 and a summary of the key HMBC and COSY correlations used to assign the aglycone region are provided in Figure 3.

**Table 1. ¹H and ¹³C NMR (500 and 125 MHz, CD₃OD) assignments of the aglycone.**

| | **CC-00340** | |
|---|---|---|
| **Position** | **¹³C** | **¹H** |
| 1 | 41.7 | 0.84 m |
| | | 1.88 m |
| 2 | 20.1 | 1.41 m |
| | | 1.95 m |
| 3 | 39.1 | 1.05 m |
| | | 2.15 brd (12.9) |
| 4 | 45.0 | --- |
| 5 | 58.5 | 1.11 m |
| 6 | 22.2 | 1.90 m |
| | | 1.93 m |
| 7 | 36.6 | 1.33 m |
| | | 1.75 m |
| 8 | 47.1 | --- |
| 9 | 53.8 | 0.93 brd (8.3) |
| 10 | 40.9 | --- |
| 11 | 20.9 | 1.44 m |
| | | 1.78 m |
| 12 | 39.1 | 1.50 m |
| | | 1.91 m |
| 13 | 87.9 | --- |
| 14 | 41.4 | 1.76 m |
| | | 2.07 brd (11.8) |
| 15 | 82.4 | 3.69^ |
| 16 | 157.3 | --- |
| 17 | 111.2 | 5.31 brs |
| | | 5.51 brs |
| 18 | 28.9 | 1.23 s |
| 19 | 178.4 | --- |
| 20 | 16.5 | 0.97 s |

| | | |
|---|---|---|
| ^{¶}Obscured by sugar protons, multiplicity could not be determined. | | |

Analysis of the ¹H-¹³C HSQC-DEPT data for **CC-00340** confirmed the presence of four anomeric protons, of which one was well resolved at δ_{H} 5.40 (δ_{C} 95.8) and two were partially overlapped at δ_{H} 4.67 (δ_{C} 97.3) and 4.66 (δ_{C} 104.4) in the ¹H NMR spectrum acquired at 300 K.

One anomeric proton that was partially obscured by water resonance at δ_{H} 4.83 at 300K was resolved in the ¹H NMR spectrum acquired at 298K at δ_{H} 4.81 (δ_{C} 103.9). All four anomeric protons had large couplings (8.2-7.8 Hz) indicating that they had β-configurations. The anomeric proton observed at δ_{H} 5.40 showed an HMBC correlation to C-19 which indicated that it corresponded to the anomeric proton of Glci. Similarly, the anomeric proton observed at δ_{H} 4.67 showed an HMBC correlation to C-13 allowing it to be assigned as the anomeric proton of Glc_{II}.

The ¹H and ¹³C chemical shifts for the C-19 glycoside are provided in Table 2. The ¹H and ¹³C chemical shifts for the C-13 glycoside are provided in Table 3.

**Table 2. ¹H and ¹³C NMR (500 and 125 MHz, CD₃OD), assignments of the C-19 glycoside.**

| | **CC-00340** | |
|---|---|---|
| **Position** | **¹³C** | **¹H** |
| Glc_{I}-1 | 95.8 | 5.40 d (8.2) |
| Glc_{I}-2 | 74.1 | 3.34 m |
| Glc_{I}-3 | 78.2-78.7^{§} | 3.45 m |
| Glc_{I}-4 | 71.0 | ~3.36 m |
| Glc_{I}-5 | 78.2-78.7^{§} | ~3.36 m |
| Glc_{I}-6 | 62.4 or 62.5 | 3.68 m, 3.83 m |

| | | |
|---|---|---|
| ^{§}Six carbon resonances in the range of 78.2-78.7 ppm (78.23, 78.32, 78.35, 78.54 and 78.71; 78.35 for two carbons), hence chemical shifts could not be unequivocally assigned. | | |

**Table 3. ¹H and ¹³C NMR (500 and 125 MHz, CD₃OD), assignments of the C-13 glycoside.**

| | **CC-00340** | |
|---|---|---|
| **Position** | **¹³C** | **¹H** |
| Glc_{II}-1 | 97.3 | 4.67 d (7.8) |
| Glc_{II}-2 | 80.4 | 3.61 m |
| Glc_{II}-3 | 87.6 | 3.69 m |
| Glc_{II}-4 | 70.6 | 3.34 m |
| Glc_{II}-5 | 77.6 | 3.26 m |
| Glc_{II}-6 | 63.0 | 3.60 m, 3.85 m |
| Glc_{III}-1 | 103.9 | 4.81 d (7.9) |
| Glc_{III}-2 | 75.7 | 3.24 m |
| Glc_{III}-3 | 78.2-78.7^{§} | 3.34 m |
| Glc_{III}-4 | 72.2 | 3.18 m |
| Glc_{III}-5 | 78.2-78.7^{§} | 3.26 m |
| Glc_{III}-6 | 63.6 | 3.58m, 3.83 m |
| Glc_{IV}-1 | 104.4 | 4.66 d (7.8) |
| Glc_{IV}-2 | 75.4 | 3.26 m |
| Glc_{IV}-3 | 78.2-78.7^{§} | 3.36 m |
| Glc_{IV}-4 | 71.6 | 3.29 m |
| Glc_{IV}-5 | 78.2-78.7^{§} | 3.34 m |
| Glc_{IV}-6 | 62.4 or 62.5 | 3.63 m, 3.88 m |

| | | |
|---|---|---|
| ^{§}Six carbon resonances in the range of 78.2-78.7 ppm (78.23, 78.32, 78.35, 78.54 and 78.71; 78.35 for two carbons), hence chemical shifts could not be unequivocally assigned. | | |

The structure of **CC-00340** was determined to be (13-[(2-*O*-β-D-glucopyranosyl-3-*O*- β-D-glucopyranosyl)- β-D-glucopyranosyl)oxy] ent-kaur-15-hydroxy-16-en-19-oic acid- β-D-glucopyranosyl ester.

### EXAMPLE 8: Isolation and Characterization of CC-00344 (Reference)

The sample used for isolation of **CC-00344** was Stevia extract.

HPLC and Isolation. Sample analyses were performed using the analytical HPLC method conditions described in Example 1. Preparative HPLC purification and isolation procedure was also carried out using the method conditions described in Example 1.

MS and MS/MS. MS and MS/MS data were generated with a Waters QTof Micro mass spectrometer equipped with an electrospray ionization source. The sample was analyzed by negative ESI. The sample (0.1 mg) was diluted with 50:50 ACN:H₂O to a concentration of 0.2 mg/mL for HRMS and MS/MS. Both samples were introduced *via* direct infusion.

NMR. The sample was prepared by dissolving 2.4 mg in 130 µL of CD₃OD and NMR data were acquired on a Bruker Avance 500 MHz instrument equipped with a 2.5 mm inverse probe. The

¹H NMR spectrum was referenced to the CHD₂OD resonance at δ_{H} 3.30 ppm and ¹³C NMR spectrum was referenced to the CD₃OD resonance at δ_{C} 49.0 ppm.

### Results and Discussion

Unless otherwise noted, all solvent ratios are listed as percent by volume (v/v).

Primary Purification of **CC-00344.** Approximately 300 g of Stevia extract was processed using the primary preparative HPLC method described above. Collected fractions were analyzed by LC-MS using the analytical method summarized above.

Secondary Purification of **CC-00344.** Material collected from primary purification was processed with conditions summarized above. Fractions were analyzed using the analytical method summarized above.

Tertiary Purification of **CC-00344.** Material collected from secondary purification was processed with conditions summarized above. Fractions were analyzed using the analytical method summarized above.

Final Batch Preparation of **CC-00344.** Material collected from tertiary purification was concentrated by rotary evaporation for final isolation. The concentrated solution was further dried *via* lyophilization for 48 hours. The final yield of the batch was 15.5 mg. The purity was >99% (AUC, CAD). The retention time of **CC-00344** was 17.445 min for CAD and 17.386 min for UV according under the conditions described in Example 1.

Mass Spectrometry. The ESI-TOF mass spectrum acquired by infusing a sample of **CC-00344** showed a [M-H]⁻ ion at *m*/*z* 821.3843. The mass of the [M-H]⁻ ion was in good agreement with the molecular formula C₃₈H₆₂O₁₉ (calcd for C₃₆H₆₁O₁₉: 821.3807, error: -3.2 ppm) expected for **CC-00344.** The MS data confirmed that **CC-00344** has a nominal mass of 822 Daltons with the molecular formula, C₃₈H₆₂O₁₉. The ion observed at *m*/*z* 935.3779 is most likely due to [M+CF₃COOH-H]⁻ adduct. The ion observed at *m*/*z* 1643.8234 is due to [2M-H]⁻.

The MS/MS spectrum of **CC-00344,** selecting the [M-H]⁻ ion at *m*/*z* 821.0 for fragmentation, indicated sequential loss of two glucose units at *m*/*z* 659.3062 and 497.2821 followed by loss of one rhamnose unit at *m*/*z* 351.2183 as a major fragmentation ions. The data thus indicated the presence of two glucose units and one rhamnose unit in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K), and 1D TOCSY (500 MHz, CD₃OD at 300K over a range of mixing times (40-140 msec)) were acquired to allow assignment of **CC-00344**.

The 1D and 2D NMR data indicated that the central core of the glycoside is a diterpene. An HMBC correlation from the methyl protons at δ_{H} 1.25 to the carbonyl at δ_{C} 176.9 allowed assignment of one of the tertiary methyl groups (C-18) as well as C-19 and provided a starting point for the assignment of the rest of the aglycone. Additional HMBC correlations from the methyl protons (H-18) to carbons at δ_{C} 38.5, 45.1, and 59.1 allowed assignment of C-3, C-4, and C-5. Analysis of the ¹H-¹³C HSQC-DEPT data indicated that the carbon at δ_{C} 38.5 was a methylene and the carbon at δ_{C} 59.1 was a methine which were assigned as C-3 and C-5, respectively. This left the carbon at δ_{C} 45.1, which did not show a correlation in the HSQC-DEPT spectrum, to be assigned as the quaternary carbon, C-4. The ¹H chemical shifts for C-3 (δ_{H} 1.05 and 2.25) and C-5 (δ_{H} 1.07) were assigned using the HSQC-DEPT data. COSY correlations between the H-3 protons (δ_{H} 1.05 and 2.25) and the protons at δ_{H} 1.40 and 1.96 allowed assignment of H-2 protons which in turn showed correlations with the protons at δ_{H} 0.84 and 1.85 which were assigned to H-1. The ¹³C chemical shifts for C-1 and C-2 were then assigned on the basis of HSQC-DEPT correlations.

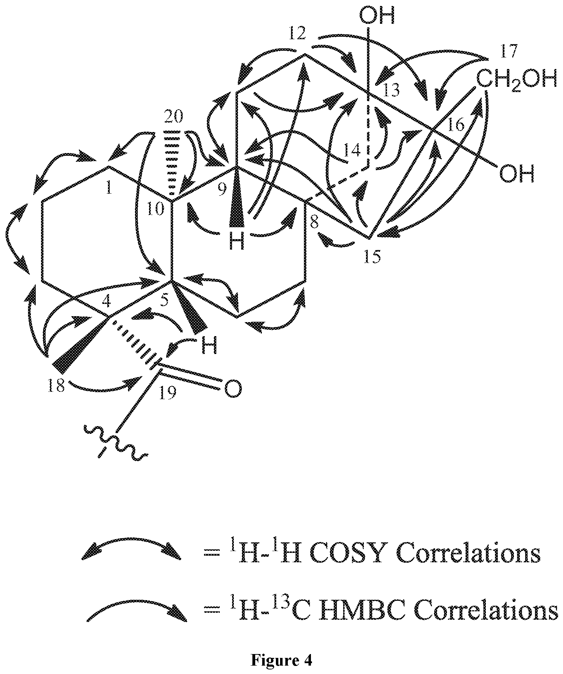

The other tertiary methyl singlet, observed at δ_{H} 0.94 (δ_{C} 17.2 *via* HSQC-DEPT), showed HMBC correlations to C-1 and C-5 and was assigned as H-20. The methyl protons showed additional HMBC correlations to a quaternary carbon (δ_{C} 40.7) and a methine carbon (δ_{C} 56.0) which were assigned as C-10 and C-9, respectively. H-9 (δ_{H} 0.94) was then determined based on HSQC-DEPT data. COSY correlations between H-5 (δ_{H} 1.07) and the protons at δ_{H} 1.82 and 1.87 then allowed assignment of the H-6 protons which in turn showed correlations to protons at δ_{H} 1.39 and 1.60 which were assigned to H-7. The ¹³C chemical shifts for C-6 (δ_{C} 22.9) and C-7 (δ_{C} 43.5) were then determined from the HSQC-DEPT data. COSY correlations between H-9 (δ_{H} 0.94) and the protons at δ_{H} 1.67 and 1.79 allowed assignment of H-11 protons. The HSQC-DEPT data was then used to assign C-11 (δ_{C} 20.9). The protons observed at δ_{H} 1.65 and 1.69 showed HMBC correlations to the carbon at δ_{C} 20.9 (C-11) and were assigned for H-12. The HSQC-DEPT data was then used to assign C-12 (δ_{C} 34.4). The methine proton H-9 showed HMBC correlations to the carbon at δ_{C} 42.5 which was assigned as C-8. In HMBC spectrum, methylene protons at H-11 and H-12 showed correlations to a quaternary carbon at δ_{C} 81.1 and was assigned for C-13. The methylene protons observed at δ_{H} 3.56 and 3.63 also showed HMBC correlations to a carbon at δ_{C} 81.1 (C-13) and were assigned to H-17 (δ_{C} 66.0 *via* HSQC-DEPT). Also, HMBC correlations from methylene protons δ_{H} 1.71 and 1.85 to C-13 (δ_{C} 81.1) allowed assignment of methylene protons at C-14. The HSQC-DEPT data was used to assign C-14 at δ_{C} 44.5. Similarly, methylene protons observed at δ_{H} 1.48 and 1.53 showed HMBC correlations to C-8, C-9, C-14 and C-17 and were assigned for H-15. The ¹³C chemical shift for C-15 (δ_{C} 52.2) was then assigned using the HSQC-DEPT data. The HMBC correlations from H-12, H-14, H-15 and H-17 to a quaternary carbon at δ_{C} 79.5 allowed assignment of C-16. The up field shift of C-16 resonance compared to related *Stevia* glycosides indicated the presence of a hydroxyl group at C-16. Also, the absence of a sugar unit at C-13 and its up field shift compared to related *Stevia* glycosides indicated the presence of a hydroxyl group at C-13. Similarly, the up field shift of C-17 resonance compared to related *Stevia* glycosides indicated that the C-17 is a hydroxymethylene group. Based on available NMR data, there is no indication of dimerization in the structure and hence C-17 unit is further confirmed to be a hydroxymethylene unit, thus allowed to complete the assignment of the central core. Other key HMBC correlations to support these assignments are presented in Figure 4.

ROESY data was acquired to assign the relative stereochemistry of the central diterpene core. NOE correlations were observed between H-18 and H-5 indicating that they are on the same side of the ring. Unfortunately, due to overlap of the protons at C-9 and C-20 (δ_{H} 0.94), the relative stereochemistry of H-9, H-20 and H-14 could not be assigned unambiguously. However, since the proton and carbon chemical shifts for C-9, C-14 and C-20 of CC-00344 are consistent with the proton and carbon chemical shifts for C-9, C-14 and C-20 of previously reported compounds from *Stevia,* the relative stereochemistry of C-9, C-14 and C-20 is considered to be the same as for previously reported *Stevia* compounds. Based on available NMR data, the relative stereochemistry of hydroxyl group at C-16 could not be assigned unambiguously due to overlapping of the key protons which are needed to assign the relative stereochemistry.

A summary of the ¹H and ¹³C chemical shifts for the aglycone are found in Table 1 and a summary of the key HMBC and COSY correlations used to assign the aglycone region are provided in Figure 4.

**Table 1. ¹H and ¹³C NMR (500 and 125 MHz, CD₃OD) assignments of the aglycone.**

| | **CC-00344** | |
|---|---|---|
| **Position** | **¹³C** | **¹H** |
| 1 | 41.7 | 0.84 ddd (17.0, 13.3, 3.8) |
| | | 1.85 m |
| 2 | 20.4 | 1.40 m |
| | | 1.96 m |
| 3 | 38.5 | 1.05 m |
| | | 2.25 brd (13.5) |
| 4 | 45.1 | --- |
| 5 | 59.1 | 1.07 m |
| 6 | 22.9 | 1.82 m |
| | | 1.87 m |
| 7 | 43.5 | 1.39 m |
| | | 1.60 m |
| 8 | 42.5 | --- |
| 9 | 56.0 | 0.94^{¶} |
| 10 | 40.7 | --- |
| 11 | 20.9 | 1.67 m |
| | | 1.79 m |
| 12 | 34.4 | 1.65 m |
| | | 1.69 m |
| 13 | 81.1 | --- |
| 14 | 44.5 | 1.71 brd (11.6) |
| | | 1.85 m |
| 15 | 52.2 | 1.48 brd (14.8) |
| | | 1.53 brd (14.9) |
| 16 | 79.5 | --- |
| 17 | 66.0 | 3.56 brd (11.4) |
| | | 3.63^{†} |
| 18 | 29.7 | 1.25 s |
| 19 | 176.9 | --- |
| 20 | 17.2 | 0.94 s |

| | | |
|---|---|---|
| ^{¶} Resonance overlapped with H-20, assignment based on HSQC-DEPT data ^{†}resonance overlapped with sugar protons, assignment based on HSQC-DEPT data. | | |

Analysis of the ¹H-¹³C HSQC-DEPT data for **CC-00344** confirmed the presence of three anomeric protons which were well resolved at δ_{H} 5.63 (δ_{C} 94.3), 5.22 (δ_{C} 102.0) and 4.54 (δ_{C} 104.4). Two of the anomeric protons had large couplings (7.1 and 7.8 Hz) indicating that they had β-configurations. One of the anomeric protons at δ_{H} 5.22 observed as a broad doublet in ¹H NMR spectrum had a small coupling of 1.5 Hz indicating that this had α-configuration. The anomeric proton observed at δ_{H} 5.63 showed an HMBC correlation to C-19 which indicated that it corresponded to the anomeric proton of Glc_{I}.

The ¹H and ¹³C chemical shifts for the C-19 glycoside are provided in Table 2.

**Table 2. ¹H and ¹³C NMR (500 and 125 MHz, CD₃OD) assignments of the C-19 glycoside.**

| | **CC-00344** | |
|---|---|---|
| **Position** | **¹³C** | **¹H** |
| Glc_{I}-1 | 94.3 | 5.63 d (7.1) |
| Glc_{I}-2 | 78.6 | 3.81 m |
| Glc_{I}-3 | 87.0 | 3.78 m |
| Glc_{I}-4 | 69.4 | 3.61 m |
| Glc_{I}-5 | 77.8 | 3.42 m |
| Glc_{I}-6 | 62.5 | 3.71 m, 3.79 m |
| Rha-1 | 102.0 | 5.22 brd (1.5) |
| Rha-2 | 72.0 or 72.1 | 3.98 m |
| Rha-3 | 72.0 or 72.1 | 3.62 m |
| Rha-4 | 73.8 | 3.36 m |
| Rha-5 | 70.6 | 3.72 m |
| Rha-6 | 18.4 | 1.22 d (6.2) |
| Glc_{VI}-1 | 104.4 | 4.54 d (7.8) |
| Glc_{VI}-2 | 75.1 | 3.27 m |
| Glc_{VI}-3 | 78.1 | 3.39 m |
| Glc_{VI}-4 | 71.6 | 3.29 m |
| Glc_{VI}-5 | 78.3 | 3.35 m |
| Glc_{VI}-6 | 62.6 | 3.64 m, 3.90 dd (11.8, 2.1) |

The structure of **CC-00344** was determined to be 13,16-Dihydroxy-17-hydroxymethylene-ent-kaur-19-oic acid-[(2-O-α-L-rhamnopyranosyl-3-O-β-D-glucopyranosyl)-β-D-glucopyranosyl) ester]. The central diterpene core contains hydroxyl groups at positions 13 and 16 as well as a hydroxymethylene group at position 17.

### EXAMPLE 9: Isolation and Characterization of CC-00349 (Reference)

The sample used for isolation of **CC-00349** was Stevia extract.

HPLC and Isolation. Analytical HPLC conditions for fraction analysis in primary, secondary and quaternary processing and were as described in Example 1. Analytical HPLC analysis conditions for fraction analysis in tertiary processing were as described in Example 5. Analytical HPLC conditions for final purity evaluation were carried out using the primary HPLC analytical method described in Example 1.

Primary, secondary and tertiary preparative HPLC purification was carried out as described in Example 5. Quaternary HPLC purification was carried out as described in Table 4.

**Table 4: Conditions for Quaternary Preparative HPLC Method.**

| | |
|---|---|
| Column | Waters XBridge Phenyl (19 × 250 mm, 5 µm) |
| Flow Rate (mL/min) | 20 |
| Detection | UV at 210 nm |
| Mobile Phases | (A) 20% MeCN in Water |
| | (B) MeCN |
| Sample preparation | Concentrated sample brought up in high aqueous |
| Gradient: | Isocratic 100% MP-A for 40 min |

Isolation Procedure. Fractions collected during the final pre-concentration step were filtered through a stainless steel sieve and concentrated *in vacuo* using a Buchi^{®} Rotary Evaporator, Model R-114. The concentrated solution was dried for 48 - 72 h using the Kinetics Flexi-Dry Personal Freeze Dryer.

MS and MS/MS. MS and MS/MS data were generated with a Waters QTof Micro mass spectrometer equipped with an electrospray ionization source. The sample was analyzed by negative ESI. The sample (0.1 mg) was diluted with H₂O to a concentration of 0.2 mg/mL for HRMS and MS/MS and introduced *via* direct infusion.

NMR. An attempt was made to dissolve 2.0 mg of the sample in 130 µL CD₃OD, however the sample did not dissolve readily. To this sample, another 50 µL CD₃OD was added and the resulting solution was still cloudy. Addition of another 50 µL CD₃OD turned it to faint cloudy. The soluble portion of the sample was utilized for NMR analysis. All NMR data were acquired on a Bruker Avance 500 MHz instrument equipped with a 2.5 mm inverse probe and a 5 mm broad band probe. The ¹H NMR spectrum was referenced to the CHD₂OD resonance at δ_{H} 3.30 ppm and ¹³C NMR spectrum was referenced to the CD₃OD resonance at δ_{C} 49.0 ppm.

### Results and Discussion

Unless otherwise noted, all solvent ratios are listed as percent by volume (v/v).

Primary Purification of **CC-00349.** Approximately 300 g of Stevia extract was processed using the primary preparative HPLC method described above. Collected fractions were analyzed by LC-MS using the analytical method summarized above.

Secondary Purification of **CC-00349.** Material collected from primary purification was processed with conditions summarized above. Collected fractions were analyzed using the analytical method summarized above.

Tertiary Purification of **CC-00349.** Material collected from secondary purification was processed with conditions summarized above. Collected fractions were analyzed using the analytical method summarized above.

Quaternary Purification of **CC-00349.** Materical collected from tertiary purification was processed with conditions summarized above.

Final Batch Preparation of **CC-00349.** Material collected from quaternary purification was concentrated by rotary evaporation for final isolation. The concentrated solution was further dried *via* lyophilization for 48 h. The final yield of the batch was 2.2 mg. The final purity was determined to be greater than 99.0% (AUC). The retention time of **CC-00349** was 6.472 minutes under the HPLC conditions described above for final purity evaluation.

Mass Spectrometry. The ESI-TOF mass spectrum acquired by infusing a sample of **CC-00349** showed a [M-H]⁻ ion at *m*/*z* 999.4203. The mass of the [M-H]⁻ ion was in good agreement with the molecular formula C₄₄H₇₂O₂₅ (calcd for C₄₄H₇₁O₂₅: 999.4284, error: -1.5 ppm) expected for **CC-00349.** The MS data confirmed that **CC-00349** has a nominal mass of 1000 Daltons with the molecular formula, C₄₄H₇₂O₂₅. The ions observed at *m*/*z* 1045.4567 and 1113.4458 were due to [M-H+2Na]⁻ and [M-H+TFA]⁻, respectively.

The MS/MS spectrum of **CC-00349**, selecting the [M-H]⁻ ion at *m*/*z* 999.0 for fragmentation, indicated loss of one glucose unit at *m*/*z* 837.3749; however, it did not show any additional fragmentation with collision energy of 20 eV. When higher collision energy was applied (50 eV), the parent ion was not observed but the ion at *m*/*z* 837.3749 was again observed corresponding to loss of one glucose unit. The ion at *m*/*z* 675.3264 corresponds to loss of second glucose unit followed by loss of two additional glucose units at *m*/*z* 513.2776 and 351.2150. The mass fragmentation pattern thus indicated the presence of four glucose units in the structure.

NMR Spectroscopy. A series of NMR experiments including ¹H NMR (500 MHz, CD₃OD at 300K), ¹³C NMR (125 MHz, CD₃OD at 300K), ¹H-¹H COSY (500 MHz, CD₃OD at 300K), HSQC-DEPT (500 MHz, CD₃OD at 300K), HMBC (500 MHz, CD₃OD at 300K), ROESY (500 MHz, CD₃OD at 300K) and 1D TOCSY (500 MHz, CD₃OD at 300K over a range of mixing times (40-140 msec) were acquired to allow assignment of **CC-00349.**

The 1D and 2D NMR data indicated that the central core of the glycoside is a diterpene. An HMBC correlation from the methyl protons at δ_{H} 1.20 to the carbonyl at δ_{C} 178.4 allowed assignment of one of the tertiary methyl groups (C-18) as well as C-19 and provided a starting point for the assignment of the rest of the aglycone. Additional HMBC correlations from the methyl protons (H-18) to carbons at δ_{C} 39.0, 45.0, and 58.3 allowed assignment of C-3, C-4, and C-5. Analysis of the ¹H-¹³C HSQC-DEPT data indicated that the carbon at δ_{C} 39.0 was a methylene and the carbon at δ_{C} 58.3 was a methine which were assigned as C-3 and C-5, respectively. This left the carbon at δ_{C} 45.0, which did not show a correlation in the HSQC-DEPT spectrum, to be assigned as the quaternary carbon, C-4. The ¹H chemical shifts for C-3 (δ_{H} 1.05 and 2.15) and C-5 (δ_{H} 1.11) were assigned using the HSQC-DEPT data. COSY correlations between the H-3 protons (δ_{H} 1.05 and 2.15) and the protons at δ_{H} 1.42 and 1.94 allowed assignment of the H-2 protons which in turn showed COSY correlations to the protons at δ_{H} 0.85 and 1.83 which were assigned to H-1. The ¹³C chemical shifts for C-1 and C-2 were then assigned on the basis of HSQC-DEPT correlations.

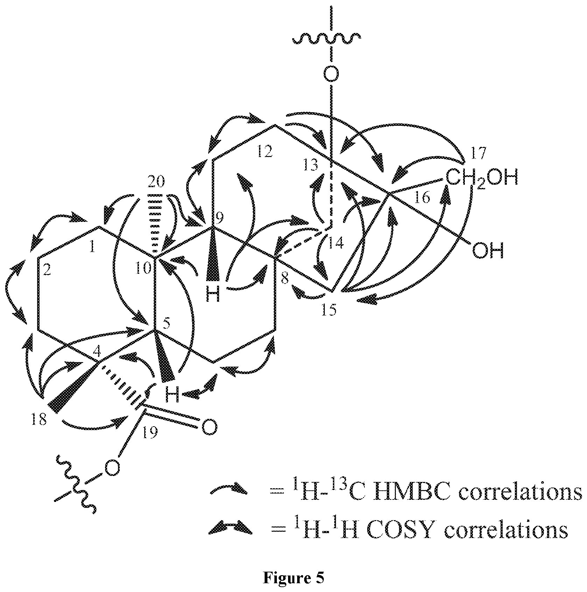

The other tertiary methyl singlet, observed at δ_{H} 0.99 (δ_{C} 16.2 *via* HSQC-DEPT), showed HMBC correlations to C-1 and C-5 and was assigned as H-20. The methyl protons showed additional HMBC correlations to a quaternary carbon (δ_{C} 40.7) and a methine carbon (δ_{C} 56.1) which were assigned as C-10 and C-9, respectively. The ¹H chemical shift for C-9 (δ_{H} 0.93) was assigned using the HSQC-DEPT data. COSY correlations between H-5 (δ_{H} 1.11) and protons at δ_{H} 1.82 and 2.03 then allowed assignment of the H-6 protons which in turn showed correlations to protons at δ_{H} 1.39 and 1.60 which were assigned to H-7. The ¹³C chemical shifts for C-6 (δ_{C} 23.1) and C-7 (δ_{C} 43.3) were then determined from the HSQC-DEPT data. COSY correlations between H-9 (δ_{H} 0.93) and the proton at δ_{H} 1.70 allowed assignment of one of the H-11 protons. The ¹H chemical shift for remaining proton at C-11 (δ_{H} 1.83) was assigned using the HSQC-DEPT data. COSY correlations from one of the H-11 protons (δ_{H} 1.83) to a proton at δ_{H} 2.01 allowed assignment of one of the H-12 protons. The ¹H chemical shift for remaining proton at C-12 (δ_{H} 1.73) was assigned using the HSQC-DEPT data. The HSQC-DEPT data was also used to assign C-11 (δ_{C} 20.9) and C-12 (δ_{C} 30.9). The methylene protons observed at δ_{H} 3.55 and 3.76 showed HMBC correlations to a quaternary carbon at δ_{C} 88.4 (C-13) and were assigned to H-17 (δ_{C} 66.3 *via* HSQC-DEPT). The proton and carbon chemical shift for position 17 indicated that the methylene unit is attached to a hydroxyl group. The methine proton H-9 showed HMBC correlations to a quaternary carbon at δ_{C} 43.1 and a methylene carbon at δ_{C} 42.7 which were assigned as C-8 and C-14, respectively. The ¹H chemical shifts at C-14 (δ_{H} 1.94 and 2.07) were assigned using the HSQC-DEPT data. HMBC correlation from H-14 (δ_{H} 2.07) to a carbon at δ_{C} 51.3 allowed assignment ofC-15. The ¹H chemical shift at C-15 (δ_{H} 1.46, both protons overlapped) was assigned using the HSQC-DEPT data. HMBC correlations from H-14 (δ_{H} 2.07), H-15 and H-17 (δ_{H} 3.55) to a quaternary carbon at δ_{C} 80.7 allowed assignment of C-16 to complete the assignment of the central core. The up field shift of C-16 in comparison to other *Stevia* glycosides having the exocyclic double bond at C-16 indicated absence of an exocyclic double bond and presence of a hydroxyl group at C-16. Other key HMBC correlations used to confirm the central core assignments are presented in Figure 5.

Correlations observed in the ROESY spectrum were used to assign the partial relative stereochemistry of the central diterpene core. In the ROESY spectrum, NOE correlations were observed between H-20 (δ_{H} 0.99) and H-14 (δ_{H} 2.07) indicating that H-14 and H-20 are on the same face of the rings. Similarly, NOE correlations were observed between H-5 and H-9 but NOE correlations were not observed between H-5/H-9 and H-20/H-14 (δδ_{H} 2.07) indicating that H-5 and H-9 were on the opposite face of the rings compared to H-14 and H-20 as shown in Figure 25. Based on available ROESY data, the relative stereochemistry at H-18 could not be determined, however based on comparison of ¹H and ¹³C chemical data with the reported data for related *Stevia* glycosides, the relative stereochemistry at H-9 is proposed as presented in Figure 5. Also, based on available ROESY data, the relative stereochemistry of hydroxyl group at C-16 could not be assigned unambiguously.

A summary of the ¹H and ¹³C chemical shifts for the aglycone are found in Table 5.

**Table 5. ¹H and ¹³C NMR (500 and 125 MHz, CD₃OD) assignments of the aglycone.**

| | **CC-00349** | |
|---|---|---|
| **Position** | **¹³C** | **¹H** |
| 1 | 41.8 | 0.85 m |
| | | 1.83 m |
| 2 | 20.0 | 1.42 m |
| | | 1.94 m |
| 3 | 39.0 | 1.05 m |
| | | 2.15 brd (13.5) |
| 4 | 45.0 | --- |
| 5 | 58.3 | 1.11 brd (12.1) |
| 6 | 23.1 | 1.82 m |
| | | 2.03 m |
| 7 | 43.3 | 1.39 m |
| | | 1.60 brd (12.8) |
| 8 | 43.1 | --- |
| 9 | 56.1 | 0.93 brd (7.5) |
| 10 | 40.7 | --- |
| 11 | 20.9 | 1.70 m |
| | | 1.83 m |
| 12 | 30.9 | 1.73 m |
| | | 2.01 m |
| 13 | 88.4 | --- |
| 14 | 42.7 | 1.94 m |
| | | 2.07 brd (11.4) |
| 15 | 51.3 | 1.46 brs^{€} |
| 16 | 80.7 | --- |
| 17 | 66.3 | 3.55 brd (11.6) |
| | | 3.76 brd (11.6) |
| 18 | 28.7 | 1.20 s |
| 19 | 178.4 | --- |
| 20 | 16.2 | 0.99 s |

| | | |
|---|---|---|
| ^{€}Methylene resonances overlapped and observed as broad singlet. | | |

Analysis of the ¹H-¹³C HSQC-DEPT data confirmed the presence of four anomeric protons which were well resolved at δ_{H} 5.36 (δ_{C} 95.8), 4.86 (δ_{C} 104.0), 4.68 (δ_{C} 97.1), and 4.65 (δ_{C} 104.3) in the ¹H NMR spectrum acquired at 300K. All anomeric protons had large couplings (7.8 - 8.1 Hz) indicating that they had β-configurations. The anomeric proton observed at δ_{H} 5.36 showed an HMBC correlation to C-19 which indicated that it corresponded to the anomeric proton of Glci. Similarly, the anomeric proton observed at δ_{H} 4.68 showed an HMBC correlation to C-13 allowing it to be assigned as the anomeric proton of Glen.

The ¹H and ¹³C chemical shifts for the C-19 glycoside are provided in Table 6. The ¹H and ¹³C chemical shifts for the C-13 glycoside are provided in Table 7.

**Table 9. ¹H and ¹³C NMR (500 and 125 MHz, CD₃OD) assignments of the C-19 glycoside.**

| | **CC-00349** | |
|---|---|---|
| Position | 13C | ¹H |
| Glci-1 | 95.8 | 5.36 d (8.1) |
| Glc_{I}-2 | 74.0 | 3.36 m |
| Glc_{I}-3 | 78.1-78.8^{§} | 3.41 m |
| Glc_{I}-4 | 71.1 or 71.2 | ~3.37 m |
| Glc_{I}-5 | 78.1-78.8^{§} | 3.34 m |
| Glc_{I}-6 | 62.4^{¥} or 62.5 or 62.8 | 3.68 m, 3.82 m |

| | | |
|---|---|---|
| ^{§}Six carbon resonances in the range of 78.1-78.8 ppm (78.14, 78.26, 78.30, 78.32, and 78.78; two carbons overlapped at 78.78 ppm), hence chemical shifts could not be unequivocally assigned. ^{¥}Two overlapping carbon resonances observed at 62.4 ppm. | | |

**Table 10. ¹H and ¹³C NMR (500 and 125 MHz, CD₃OD) assignments of the C-13 glycoside.**

| | **CC-00349** | |
|---|---|---|
| Position | ¹³C | ¹H |
| Glc_{II}-1 | 97.1 | 4.68 d (7.8) |
| Glc_{II}-2 | 80.1 | 3.61 m |
| Glc_{II}-3 | 88.2 | 3.72 m |
| Glc_{II}-4 | 70.5 | 3.34 m |
| Glc_{II}-5 | 77.4 | 3.31 m |
| Glc_{II}-6 | 62.4^{¥} or 62.5 or 62.8 | 3.63 m, 3.88 m |
| Glc_{III}-1 | 104.0 | 4.86 d (8.1) |
| Glc_{III}-2 | 75.8 | 3.13 m |
| Glc_{III}-3 | 78.1-78.8^{§} | 3.33 m |
| Glc_{III}-4 | 71.1 or 71.2 | ~3.34 m |
| Glc_{III}-5 | 78.1-78.8^{§} | 3.25 m |
| Glc_{III}-6 | 62.4^{¥} or 62.5 or 62.8 | 3.70 m, 3.84 m |
| Glc_{IV}-1 | 104.3 | 4.65 d (7.9) |
| Glc_{IV}-2 | 75.4 | 3.25 m |
| Glc_{IV}-3 | 78.1-78.8^{§} | 3.35 m |
| Glc_{IV}-4 | 71.6 | 3.28 m |
| Glc_{IV}-5 | 78.1-78.8^{§} | ~3.35 m |
| Glc_{IV}-6 | 62.4^{¥} or 62.5 or 62.8 | 3.62 m, 3.88 m |

| | | |
|---|---|---|
| ^{§}Six carbon resonances in the range of 78.1-78.8 ppm (78.14, 78.26, 78.30, 78.32, and 78.78; two carbons overlapped at 78.78 ppm), hence chemical shifts could not be unequivocally assigned. ^{¥} Two overlapping carbon resonances observed at 62.4 ppm. | | |

The structure of **CC-00349** was determined to be (13-[(2-O-β-D-glucopyranosyl-3-O- β-D-glucopyranosyl)- β-D-glucopyranosyl)oxy] ent-kaur-16,17-dihydroxy-19-oic acid (β-D-glucopyranosyl) ester]. **CC-00349** is a 16,17-dihydroxy derivative of Rebaudioside A.

## Claims

1. A beverage or beverage product comprising an isolated and purified diterpene glycoside of the following formula: 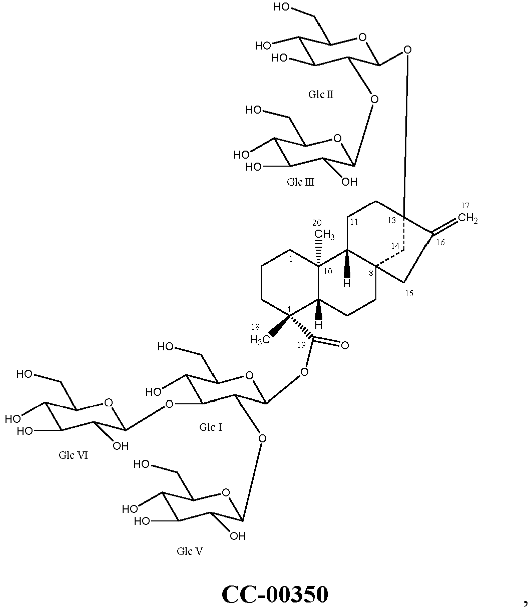 wherein the diterpene glycoside is present in the beverage in a concentration from about 25 ppm to about 600 ppm.

2. The beverage or beverage product of claim 1, wherein the diterpene glycoside is present in the beverage in a concentration from about 100 ppm to about 600 ppm.

3. The beverage or beverage product of claim 1, wherein the beverage is selected from the group consisting of an enhanced sparkling beverage, cola, lemon-lime flavored sparkling beverage, orange flavored sparkling beverage, grape flavored sparking beverage, strawberry flavored sparkline beverage, pineapple flavored sparkling beverage, ginger ale, root beer, fruit juice, fruit-flavored juice, nectar, vegetable juice, vegetable-flavored juice, sports drink, energy drink, enhanced water drink, near water drink, coconut water, tea, coffee, cocoa drink, beverage containing milk components, beverage containing cereal extract and smoothie.

4. The beverage or beverage product of claim 1, wherein the beverage further comprises one or more sweeteners.

5. The beverage or beverage product of claim 4, wherein the one or more sweeteners comprises a carbohydrate sweetener.

6. The beverage or beverage product of claim 5, wherein the sweetener is selected from the group consisting of sucrose, fructose, glucose, high fructose corn/starch syrup, beet sugar and cane sugar.

7. The beverage or beverage product of claim 4, wherein the one or more sweeteners are selected from the group consisting of rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside M, rebaudioside N, rebaudioside O, dulcoside A, ducosie B, rubusoside, stevia, Stevioside, mogroside IV, mogroside V, mogroside VI, Luo Han Guo and siamenoside.

8. The beverage or beverage product of claim 7, wherein the sweetener is rebaudioside M.

## Patentansprüche

1. Getränk oder Getränkeprodukt, umfassend ein isoliertes oder aufgereinigtes Diterpenglycosid der folgenden Formel: 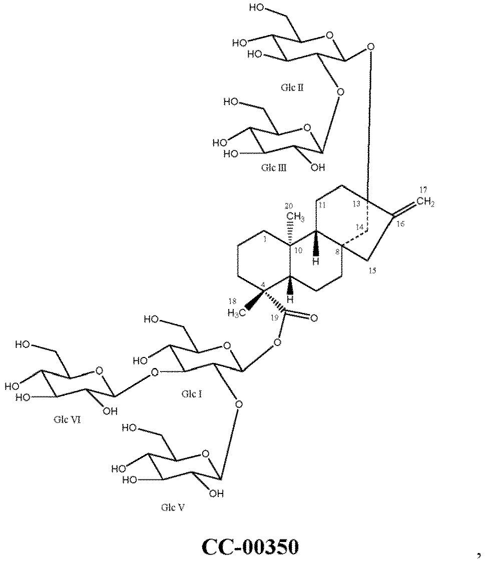 wobei das Diterpenglycosid in dem Getränk in einer Konzentration von etwa 25 ppm bis etwa 600 ppm vorliegt.

2. Getränk oder Getränkeprodukt nach Anspruch 1, wobei das Diterpenglycosid in dem Getränk in einer Konzentration von etwa 100 ppm bis etwa 600 ppm vorliegt.

3. Getränk oder Getränkeprodukt nach Anspruch 1, wobei das Getränk aus der Gruppe bestehend aus einem verstärkt sprudelnden Getränk, Cola, einem sprudelnden Getränk mit Zitronen-Limonen-Geschmack, einem sprudelnden Getränk mit Orangengeschmack, einem sprudelnden Getränk mit Weintraubengeschmack, einem sprudelnden Getränk mit Erdbeergeschmack, einem sprudelnden Getränk mit Ananasgeschmack, Ginger-Ale, Wurzelbier, Fruchtsaft, Saft mit Fruchtgeschmack, Nektar, Gemüsesaft, Saft mit Gemüsegeschmack, Sportgetränken, Energiegetränken, einem angereicherten Wassergetränk, einem Near-Water-Drink, Kokosnusswasser, Tee, Kaffee, einem Kakaogetränk, einem Getränk mit Milchkomponenten, einem Getränk mit Getreideextrakt und einem Smoothie ausgewählt ist.

4. Getränk oder Getränkeprodukt nach Anspruch 1, wobei das Getränk ferner ein oder mehrere Süßungsmittel umfasst.

5. Getränk oder Getränkeprodukt nach Anspruch 4, wobei das eine oder die mehreren Süßungsmittel ein Kohlenhydrat-Süßungsmittel umfassen.

6. Getränk oder Getränkeprodukt nach Anspruch 5, wobei das Süßungsmittel aus der Gruppe bestehend aus Saccharose, Fructose, Glucose, fructosereichem Mais/Stärke-Sirup, Rübenzucker und Rohrzucker ausgewählt ist.

7. Getränk oder Getränkeprodukt nach Anspruch 4, wobei das eine oder die mehreren Süßungsmittel aus der Gruppe bestehend aus Rebaudiosid A, Rebaudiosid B, Rebaudiosid C, Rebaudiosid D, Rebaudiosid E, Rebaudiosid F, Rebaudiosid I, Rebaudiosid H, Rebaudiosid L, Rebaudiosid K, Rebaudiosid J, Rebaudiosid M, Rebaudiosid N, Rebaudiosid O, Dulcosid A, Dulcosid B, Rubusosid, Stevia, Steviosid, Mogrosid IV, Mogrosid V, Mogrosid VI, Luo Han Guo und Siamenosid ausgewählt sind.

8. Getränk oder Getränkeprodukt nach Anspruch 7, wobei sich bei dem Süßungsmittel um Rebaudiosid M handelt.

## Revendications

1. Boisson ou produit de boisson comprenant un glycoside de diterpène isolé et purifié de la formule suivante : 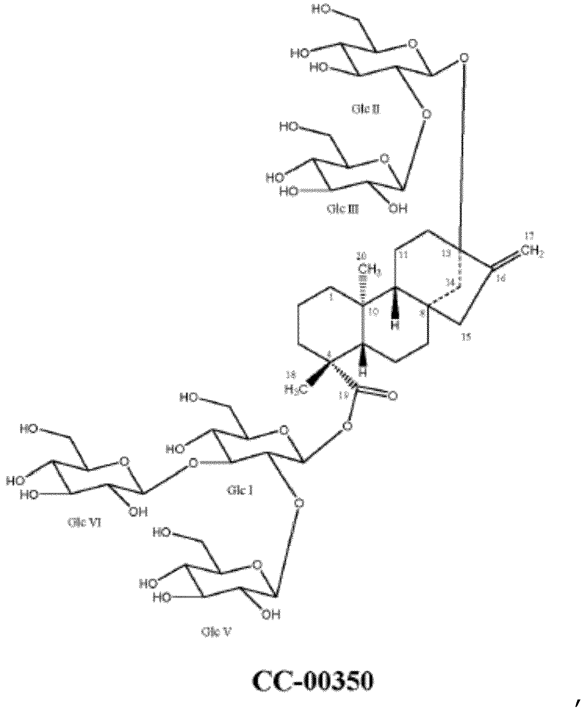 le glycoside de diterpène étant présent dans la boisson en une concentration d'environ 25 ppm à environ 600 ppm.

2. Boisson ou produit de boisson selon la revendication 1, le glycoside de diterpène étant présent dans la boisson en une concentration d'environ 100 ppm à environ 600 ppm.

3. Boisson ou produit de boisson selon la revendication 1, la boisson étant choisie dans le groupe constitué par une boisson à pétillance améliorée, un cola, une boisson pétillante aromatisée au citron-citron vert, une boisson pétillante aromatisée à l'orange, une boisson pétillante aromatisée au raisin, une boisson pétillante aromatisée à la fraise, une boisson pétillante aromatisée à l'ananas, un soda au gingembre, une racinette, du jus de fruit, du jus aromatisé aux fruits, un nectar, du jus de légumes, du jus aromatisé aux légumes, une boisson pour le sport, une boisson énergisante, une boisson à base d'eau enrichie, une boisson proche de l'eau, de l'eau de coco, du thé, du café, une boisson au cacao, une boisson contenant des composants du lait, une boisson contenant de l'extrait de céréales et un smoothie.

4. Boisson ou produit de boisson selon la revendication 1, la boisson comprenant en outre un ou plusieurs édulcorants.

5. Boisson ou produit de boisson selon la revendication 4, l'édulcorant ou les édulcorants comprenant un édulcorant de type glucide.

6. Boisson ou produit de boisson selon la revendication 5, l'édulcorant étant choisi dans le groupe constitué par le saccharose, le fructose, le glucose, un sirop de maïs/amidon à haute teneur en fructose, le sucre de betterave et le sucre de canne.

7. Boisson ou produit de boisson selon la revendication 4, l'édulcorant ou les édulcorants étant choisis dans le groupe constitué par le rébaudioside A, le rébaudioside B, le rébaudioside C, le rébaudioside D, le rébaudioside E, le rébaudioside F, le rébaudioside I, le rébaudioside H, le rébaudioside L, le rébaudioside K, le rébaudioside J, le rébaudioside M, le rébaudioside N, le rébaudioside O, le dulcoside A, le dulcoside B, le rubusoside, le stévia, un stévioside, le mogroside IV, le mogroside V, le mogroside VI, Luo Han Guo et le siaménoside.

8. Boisson ou produit de boisson selon la revendication 7, l'édulcorant étant le rébaudioside M.
